(19) Europäisches Patentamt
European Patent Office
Office européen des brevets

(11) EP 3 762 383 B1

(12) EUROPEAN PATENT SPECIFICATION

(45) Date of publication and mention of the grant of the patent:
**06.12.2023 Bulletin 2023/49**

(21) Application number: **19716018.7**

(22) Date of filing: **06.03.2019**

(51) International Patent Classification (IPC):
***C07D 409/12*** (2006.01) ***C07D 471/04*** (2006.01)
***A61P 25/16*** (2006.01) ***A61P 25/28*** (2006.01)
***A61P 25/18*** (2006.01) ***A61K 31/381*** (2006.01)
***A61K 31/4436*** (2006.01) ***A61K 31/519*** (2006.01)

(52) Cooperative Patent Classification (CPC):
**C07D 409/12; A61P 25/16; A61P 25/18; A61P 25/28; C07D 471/04**

(86) International application number:
**PCT/US2019/020896**

(87) International publication number:
**WO 2019/173437 (12.09.2019 Gazette 2019/37)**

(54) **POSITIVE ALLOSTERIC MODULATORS OF DOPAMINE 1 RECEPTOR AND METHOD OF USE THEREOF**

POSITIVE ALLOSTERISCHE MODULATOREN DES DOPAMIN-1-REZEPTORS UND VERFAHREN ZUR VERWENDUNG DAVON

MODULATEURS ALLOSTÉRIQUES POSITIFS DU RÉCEPTEUR DE LA DOPAMINE 1 ET PROCÉDÉ D'UTILISATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.03.2018 US 201862639271 P**

(43) Date of publication of application:
**13.01.2021 Bulletin 2021/02**

(73) Proprietors:
- **The United States of America, as represented by the Secretary, Department of Health and Human Services**
  **Bethesda, Maryland 20892-7788 (US)**
- **University of Kansas**
  **Lawrence, Kansas 66045 (US)**
- **The University of North Carolina at Chapel Hill**
  **Chapel Hill, North Carolina 27516 (US)**

(72) Inventors:
- **SIBLEY, David R.**
  **Gaithersburg, Maryland 20879 (US)**
- **LUDERMAN, Kathryn D.**
  **Silver Spring, Maryland 20906 (US)**
- **CONROY, Jennie L.**
  **Gaithersburg, Maryland 20878 (US)**
- **FREE, R. Benjamin**
  **Rockville, Maryland 20853 (US)**
- **JAIN, Prashi**
  **Houston, Texas 77096 (US)**
- **SOUTHALL, Noel T.**
  **Potomac, Maryland 20854 (US)**
- **FERRER, Marc**
  **Potomac, Maryland 20854 (US)**
- **AUBÉ, Jeffrey**
  **Chapel Hill, North Carolina 27517 (US)**
- **FRANKOWSKI, Kevin**
  **Pittsboro, North Carolina 27312 (US)**

(74) Representative: **Pfenning, Meinig & Partner mbB**
**Patent- und Rechtsanwälte**
**Theresienhöhe 11a**
**80339 München (DE)**

(56) References cited:
**WO-A1-2014/091446**
**WO-A2-2005/033102**
**WO-A2-2006/044826**
**WO-A2-2012/080727**
**CN-A- 108 558 848**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KRANZ M ET AL: "Identification of PDE4B Over 4D subtype-selective inhibitors revealing an unprecedented binding mode", BIOORGANIC & MEDICINAL CHEMISTRY, PERGAMON, GB, vol. 17, no. 14, 15 July 2009 (2009-07-15) , pages 5336-5341, XP026303003, ISSN: 0968-0896, DOI: 10.1016/J.BMC.2009.03.061 [retrieved on 2009-04-05]
- A. SORIANO ET AL: "A Hybrid Indoloquinolizidine Peptide as Allosteric Modulator of Dopamine D1 Receptors", JOURNAL OF PHARMACOLOGY AND EXPERIMENTAL THERAPEUTICS, vol. 332, no. 3, 1 March 2010 (2010-03-01) , pages 876-885, XP055398019, US ISSN: 0022-3565, DOI: 10.1124/jpet.109.158824
- M. A. LEWIS ET AL: "Discovery of D1 Dopamine Receptor Positive Allosteric Modulators: Characterization of Pharmacology and Identification of Residues that Regulate Species Selectivity", PHARMACOLOGICAL REVIEWS, vol. 354, no. 3, 24 July 2015 (2015-07-24) , pages 340-349, XP055398017, US ISSN: 0031-6997, DOI: 10.1124/jpet.115.224071 cited in the application
- ADRIAN HALL ET AL: "Novel Strategies To Activate the Dopamine D 1 Receptor: Recent Advances in Orthosteric Agonism and Positive Allosteric Modulation", JOURNAL OF MEDICINAL CHEMISTRY, vol. 62, no. 1, 11 December 2018 (2018-12-11), pages 128-140, XP055586616, ISSN: 0022-2623, DOI: 10.1021/acs.jmedchem.8b01767

## Description

CROSS REFERENCE TO A RELATED APPLICATION

This patent application claims the benefit of U.S. Provisional Patent Application No. 62/639,271, filed March 6, 2018.

STATEMENT REGARDING FEDERALLY SPONSORED RESEARCH AND DEVELOPMENT

This invention was made with Government support under NIH Molecular Libraries Initiative awards (MH094203 and U54HG005031) awarded by the National Institutes of Health. The Government has certain rights in this invention.

BACKGROUND OF THE INVENTION

There is great interest in identifying small molecule ligands for G-protein coupled receptors (GPCRs), as nearly 50% of all FDA-approved drugs target these important receptor proteins (Eglen 2007). Unfortunately, many of the ligands used as drugs or pharmacological tools are not very selective and exhibit problematic or limiting side effects due to inappropriate off target signaling. A very important therapeutic subclass of GPCRs is that activated by dopamine (DA), a crucial neurotransmitter in both the central nervous system (CNS) and the periphery (Sibley and Monsma 1992). In mammals, five distinct DA receptor (DAR) subtypes have been identified and are divided into two subfamilies based on their structure, pharmacology, and signaling properties (Sibley and Monsma 1992). The D1-like DARs (D1R and D5R) are coupled to $G\alpha_{s/olf}$ proteins and activate adenylyl cyclase, resulting in increased intracellular cAMP levels. In contrast, the D2-like DARs (D2R, D3R and D4R) are coupled to $G\alpha_{i/o}$ proteins and function to inhibit adenylyl cyclase activity and also modulate $K^+$ and $Ca^{2+}$ channel activities. Dysfunction of the CNS dopaminergic system is involved in the etiology of a number of neuropsychiatric disorders, which are classically treated with drugs that either stimulate or block various DAR subtypes; making the dopamine receptors a key target for therapeutics.

The most highly expressed DAR subtype is the D1R, which is found in high abundance in various regions of the mammalian forebrain including the striatum, cerebral cortex, hippocampus, and the olfactory bulb. Physiologically, it is believed to play a crucial role in regulating movement, cognition, learning and memory, as well as reward and reinforcement. As such, the D1R provides an attractive drug target for the treatment of a number of neuropsychiatric disorders, among them the decline of cognition and memory, both hallmarks of Alzheimer's disease, schizophrenia, and Parkinson's disease. Goldman-Rakic and colleagues have shown that an optimum level of D1R activity in the prefrontal cortex (PFC) is required for ideal performance in learning and memory tasks (Goldman-Rakic, Castner et al. 2004). Either too little or too much D1R stimulation impairs PFC function (Arnsten and Dudley 2005; Arnsten and Li 2005; Vijayraghavan, Wang et al. 2007). Thus, low doses of D1 agonists improve working memory and attention, whereas high levels of DA release, such as during stress, impair PFC function. These observations have led to the "inverted U" hypothesis of the relationship between D1 receptor stimulation and normal physiological functioning of the PFC (Arnsten, Vijayraghavan et al. 2009). Further, suboptimal levels of D1R stimulation have been suggested to underlie age-associated learning deficits and contribute to the decreased cognition that is observed in various pathophysiological states, especially schizophrenia. Currently, all antipsychotic agents block D2R activity, which effectively treats the positive symptoms of schizophrenia (hallucinations, etc.); however, D2R blockade does not treat the severe cognitive impairment that is observed with this disorder. To date, there are no drugs that effectively treat cognition in people with schizophrenia. A subcommittee of the Measurement and Treatment Research to Improve Cognition in Schizophrenia (MATRICS) program evaluated a range of molecular targets for treating cognition. A pharmacological target suggested is the D1R in the PFC (Tamminga 2006). Animal tests have confirmed the positive effects of D1R stimulation on working memory and cognitive function (Goldman-Rakic, Castner et al. 2004). These findings highlight the potential therapeutic importance of D1R targeting compounds.

One approach to target the complex interplay of D1R physiology and pathology is through the development of compounds that uniquely influence the D1R by enhancing the endogenous signaling, without directly activating the receptor itself. These compounds are termed positive allosteric modulators (PAMs), and they bind to a site on a receptor separate from the orthosteric binding pocket of the endogenous ligand. PAMs enhance endogenous ligand activity through augmenting the potency, efficacy, or both, of the agonist. Developing PAMs for a given target may have advantages over traditional orthosteric agonists; for instance, fewer off-target side effects (as they likely to bind to a less conserved region of a receptor) as well as decreased or no receptor desensitization (as they don't activate the receptor themselves which leads to desensitization) (May, Leach et al. 2007; Gjoni and Urwyler 2008). Allosteric compounds can also function as "stabilizers" of signaling pathways, as they exert their effects by modulating the activity of endogenous neurotransmitters which constantly fluctuate in response to neuronal tone. Taken together development of D1R PAMs presents an attractive target for potential lead therapeutic compounds.

Thus, there remains an unmet need in the art for positive allosteric modulators of the D1R and for a method of treatment of disorders responsive to such modulators.

A. SOR!ANO ET AL, "A Hybrid Indoloquinolizidine Peptide as Allosteric Modulator of Dopamine D1 Receptors", JOURNAL OF PHARMACOLOGY AND EXPERIMENTAL THERAPEUTICS, US, (20100301), vol. 332, no. 3, doi:10.1124/jpet.109.158824, ISSN 0022-3565, pages 876 - 885, XP055398019 is a review of indoloquinolizidine compounds as allosteric modulator of dopamine D1 receptors.

M. A. LEWIS ET AL, "Discovery of D1 Dopamine Receptor Positive Allosteric Modulators: Characterization of Pharmacology and Identification of Residues that Regulate Species Selectivity", PHARMACOLOGICAL REVIEWS, US, (20150724), vol. 354, no. 3, doi:10.1124/jpet.115.224071, ISSN 0031-6997, pages 340 - 349, XP055398017 is a review of indoloquinolizidine compounds as allosteric modulator of dopamine D1 receptors.

BRIEF SUMMARY OF THE INVENTION

The invention provides a compound of formula (I):

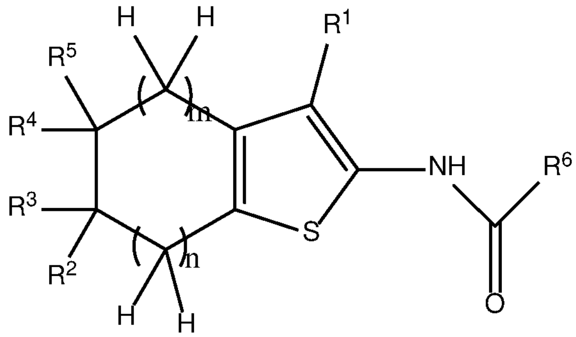

(I)

wherein $R^1$ is $-CONR^7R^8$,
$R^2$ is $C_1$-$C_6$ alkyl or $C_6$-$C_{10}$ aryl and $R^3$-$R^5$ are all H
m and n are 1,
$R^6$ is 2-pyridyl, 3-pyridyl, or 2-quinolinyl, and
$R^7$ and $R^8$ are independently H or $C_1$-$C_6$ alkyl,
wherein $R^6$ is optionally substituted with one or more groups selected from the group consisting of halo, $C_1$-$C_6$ alkyl, and $C_1$-$C_6$ alkoxy,
or a pharmaceutically acceptable salt thereof.

The invention also provides a compound of formula (III):

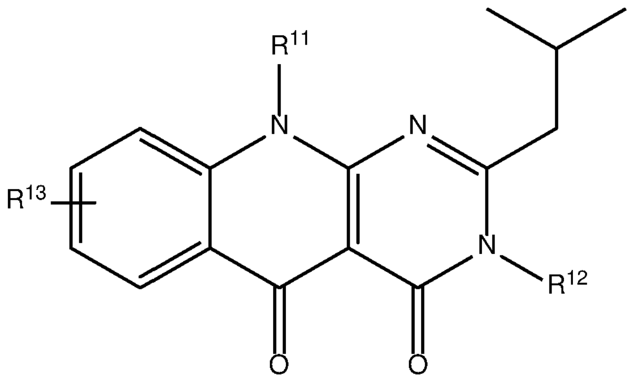

(III)

wherein $R^{11}$ is $C_1$-$C_6$ alkyl,

$R^{12}$ is phenyl, optionally substituted with one or more substituents selected from the group consisting of halo, $C_1$-$C_6$

alkyl, or $C_1$-$C_6$ alkoxy, and
$R^{13}$ is H, halo, or $C_1$-$C_6$ alkoxy,
or a pharmaceutically acceptable salt thereof.

The invention further provides a compound of formula (I):

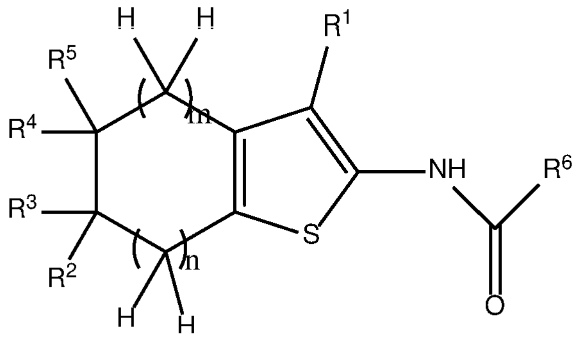

(I)

wherein $R^1$ is -CN or -CONR$^7$R$^8$,
$R^2$ is $C_1$-$C_6$ alkyl or $C_6$-$C_{10}$ aryl and $R^3$-$R^5$ are all H or wherein $R^4$ is $C_1$-$C_6$ alkyl or $C_6$-$C_{10}$ aryl and $R^2$, $R^3$, and $R^5$ are all H,
m and n are independently 1 or 2,
$R^6$ is 2-pyridyl, 3-pyridyl, 4-pyridyl, or 2-quinolinyl, and
$R^7$ and $R^8$ are independently H or $C_1$-$C_6$ alkyl,
wherein $R^6$ is optionally substituted with one or more groups selected from the group consisting of halo, $C_1$-$C_6$ alkyl, and $C_1$-$C_6$ alkoxy,
or a pharmaceutically acceptable salt thereof, and/or
a compound of formula (II):

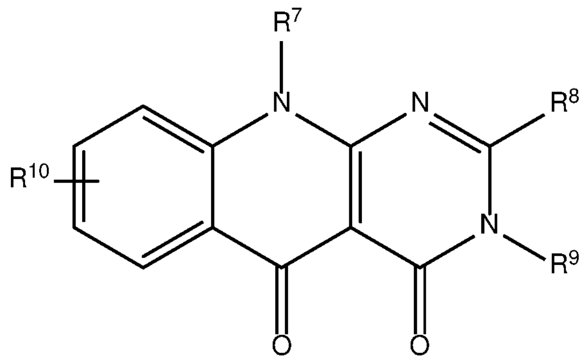

(II)

wherein $R^7$ is $C_1$-$C_6$ alkyl,
$R^8$ is $C_1$-$C_6$ alkyl, $C_3$-$C_8$-cycloalkyl, or $C_3$-$C_8$-cycloalkyl-$C_1$-$C_6$-alkylene,
$R^9$ is phenyl, optionally substituted with one or more substituents selected from the group consisting of halo, $C_1$-$C_6$ alkyl, or $C_1$-$C_6$ alkoxy, and
$R^{10}$ is H, halo, or $C_1$-$C_6$ alkoxy,
or a pharmaceutically acceptable salt thereof for use in treating or preventing a disease or disorder responsive to activation of a D1 dopamine receptor agonist in a mammal in need thereof.

BRIEF DESCRIPTION OF THE SEVERAL VIEWS OF THE DRAWING(S)

FIG. 1A shows β -arrestin recruitment following stimulation by the indicated concentrations of dopamine either alone (DA) or in the presence of 50 μM of compound **1.**

FIG. 1B shows β -arrestin recruitment following stimulation by the indicated concentrations of dopamine either alone (DA) or in the presence of 50 μM of compound **28.**

FIG. 2A shows dopamine concentration response curves in a β-arrestin recruitment assay for increasing concentrations of compound **1.**

FIG. 2B shows dopamine concentration response curves in a β-arrestin recruitment assay for increasing concentrations of compound **28.**

FIG. 3A shows the effect of compound **1** on the dopamine potency for cAMP accumulation.

FIG. 3B shows the effect of compound **1** on forskolin stimulated cAMP accumulation.

FIG. 3C shows the effect of compound **28** on the dopamine potency for cAMP accumulation.

FIG. 3D shows the effect of compound **28** on forskolin stimulated cAMP accumulation.

FIG. 4A shows the results of a binding competition assay to determine dopamine's affinity for the receptor in the presence of increasing concentration of compound **1**.

FIG. 4B shows the results of a binding competition assay to determine dopamine's affinity for the receptor in the presence of increasing concentration of compound **28.**

FIG. 4C shows the results of a radioligand competition displacement binding for [3H]-SCH23390 binding in combination with compound **1** and compound **28.**

FIG. 5A shows the results of β-arrestin recruitment assays using dose responses of the known agonist of the D1R fenlodopam, both in the presence and absence of compounds **1** and **28.**

FIG. 5B shows the results of β-arrestin recruitment assays using dose responses of the known agonist of the D1R apomorphine, both in the presence and absence of compounds **1** and **28.**

FIG. 5C shows the results of β-arrestin recruitment assays using dose responses of the known partial agonist of the D1R SKF38393, both in the presence and absence of compounds **1** and **28.**

FIG. 5D shows the results of β-arrestin recruitment assays using dose responses of the known partial agonist of the D1R SKF77434, both in the presence and absence of compounds **1** and **28.**

FIG. 6A shows the concentration-response curves for β-arrestin recruitment with dopamine plus either 50 μM compound **1** or 50 μM compound **28.**

FIG. 6B shows the concentration-response curves for cAMP accumulation with dopamine plus either 50 μM compound **1** or 50 μM compound **28.**

FIG. 7A shows the results of competition binding of [$^3$H]-SCH23390 with compound **5** at the dopamine D1 receptor.

FIG. 7B shows the results of competition binding of [$^3$H]-SCH23390 with compound **6** at the dopamine D1 receptor.

FIG. 7C shows the results of competition binding of [$^3$H]-SCH23390 with compound **7** at the dopamine D1 receptor.

FIG. 8A shows β-arrestin recruitment to the D1R in the presence of 50 μM compound **5.**

FIG. 8B shows β-arrestin recruitment to the D1R in the presence of 50 μM compound **6.**

FIG. 8C shows β-arrestin recruitment to the D1R in the presence of 50 μM compound **7**.

FIG. 9A shows β-arrestin recruitment to the D5R in the presence of 50 μM compound **5.**

FIG. 9B shows β-arrestin recruitment to the D5R in the presence of 50 μM compound **6.**

FIG. 9C shows β-arrestin recruitment to the D5R in the presence of 50 μM compound **7**.

FIG. 10 shows agonist-mediated D1R internalization as a function of dopamine concentration in the presence of 50 μM compound **1** or 50 μM compound **28.**

FIG. 11 shows β-arrestin recruitment as measured following stimulation by the indicated concentrations of known agonist dihydrexidine in the absence or presence of 50 μM of either compound **1** or compound **28.**

FIG. 12A shows the structure of compound B.

FIG. 12B shows β-arrestin recruitment as measured following stimulation with dopamine in the absence (DA) or in the presence of 50 μM compound **1**, 100 μM Compound B, or a combination of the two compounds.

FIG. 12C shows β-arrestin recruitment as measured following stimulation with dopamine in the absence (DA) or in the presence of 50 μM compound **28,** 100 μM Compound B, or a combination of the two compounds.

FIG. 13A shows $D_1$R-arrestin BRET ratios vs. dopamine concentration for the wild-type $D_1$R alone or in the presence of 50 μM compound **1** or 50 μM compound **28.**

FIG. 13B shows $D_1$R-arrestin BRET ratios vs. dopamine concentration for the mutant R130Q $D_1$R alone or in the presence of 50 μM compound **1** or 50 μM compound **28.**

FIG. 13C shows $D_1$R-G protein BRET ratios vs. dopamine concentration for the mutant R130Q $D_1$R alone or in the presence of 50 μM compound **1** or 50 μM compound **28.**

FIG. 13D shows $D_1$R-G protein BRET ratios vs. dopamine concentration for the wild-type $D_1$R alone or in the presence of 50 μM compound **1** or 50 μM compound **28.**

FIG. 14 shows β-arrestin recruitment as measured following stimulation by the indicated concentrations of dopamine either alone (DA) or in the presence of 50 μM of either compound **1** or compound **28** in the DiscoverX assay, using

cells stably expressing human D5R.

FIGS. 15A-15C show the $D_2$R-arrestin BRET ratios, $D_3$R-arrestin BRET ratios, and $D_4$R-arrestin BRET ratios versus dopamine concentration, respectively, following stimulation by the indicated concentrations of dopamine (DA) in the presence of 50 μM of either compound **1** or compound **28** in the β-arrestin BRET assay.

FIG. 15D shows the β2AR-arrestin BRET ratios versus epinephrine concentration, following stimulation by the indicated concentrations of epinephrine (EPI) in the presence of 50 μM of either compound **1** or compound **28** in the β-arrestin BRET assay.

DETAILED DESCRIPTION OF THE INVENTION

The invention provides a compound of formula (I):

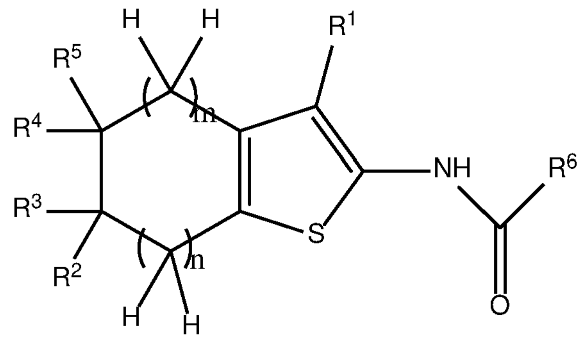

(I)

wherein $R^1$ is or $-CONR^7R^8$,
$R^2$ is $C_1$-$C_6$ alkyl or $C_6$-$C_{10}$ aryl and $R^3$-$R^5$ are all H,
m and n are 1,
$R^6$ is 2-pyridyl, 3-pyridyl, or 2-quinolinyl, and
$R^7$ and $R^8$ are independently H or $C_1$-$C_6$ alkyl,
wherein $R^6$ is optionally substituted with one or more groups selected from the group consisting of halo, $C_1$-$C_6$ alkyl, and $C_1$-$C_6$ alkoxy,
or a pharmaceutically acceptable salt thereof.

In an embodiment, $R^6$ is 2-pyridyl, or 3-pyridyl.

In certain preferred embodiments, the compound of formula (I) is selected from the group consisting of:

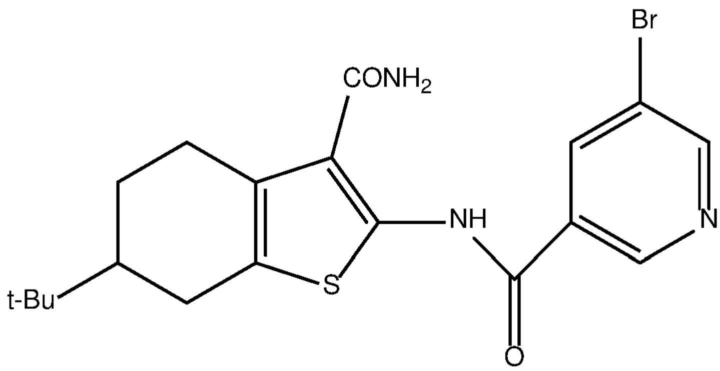

,

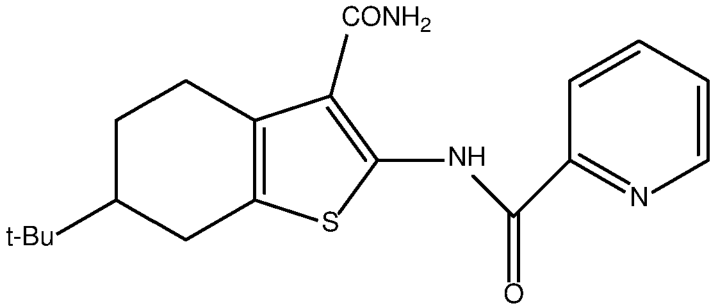

,

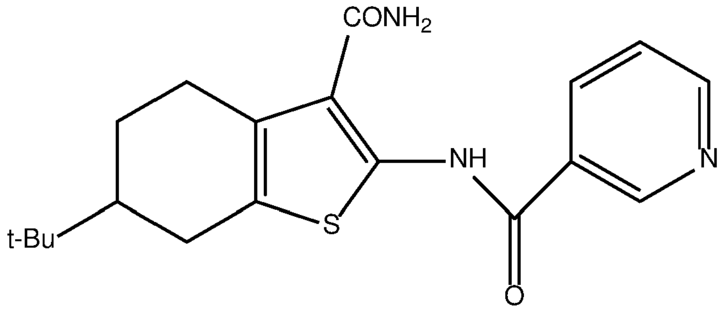

,

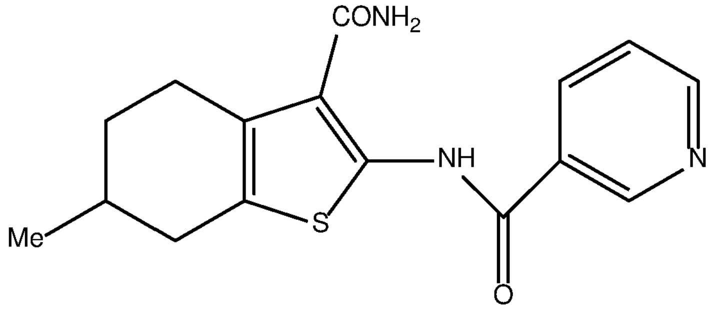

,

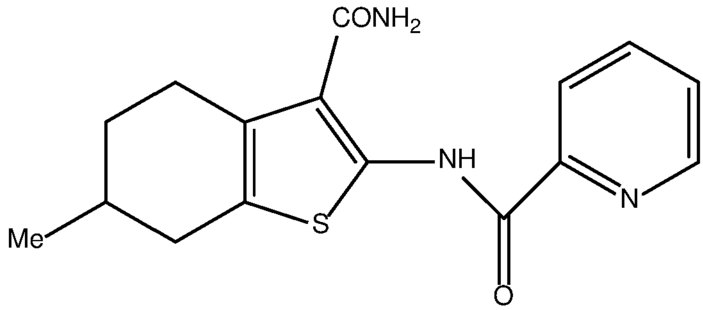

.

In an embodiment of the compound of formula (I), $R^6$ is 2-quinolinyl.

In certain particular embodiments, the compound is:

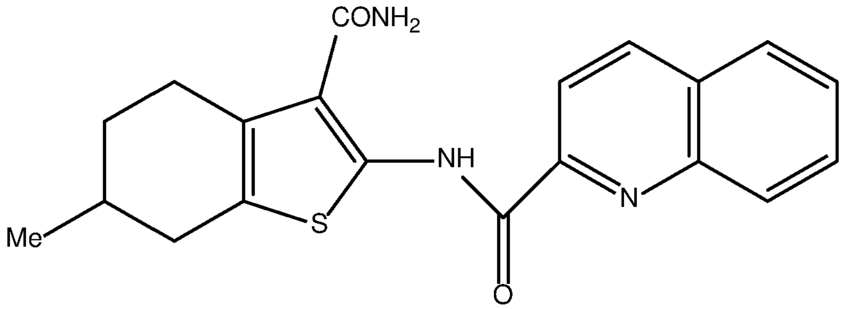

or

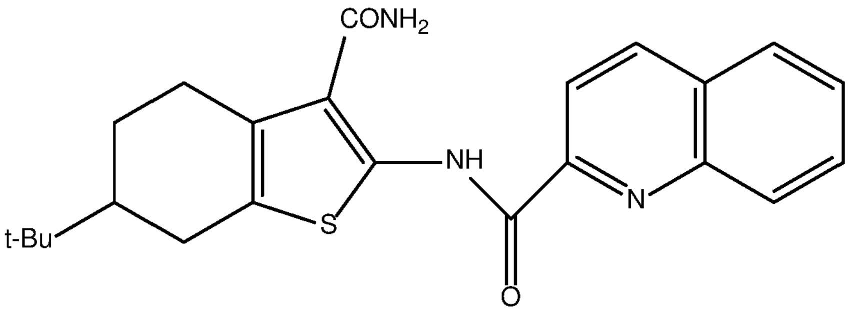

.

The invention also provides a compound of formula (III):

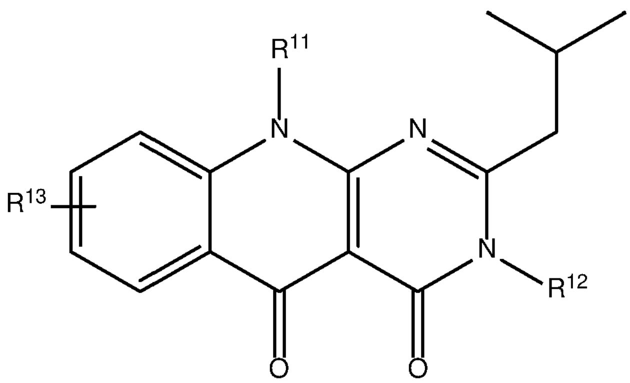

(III)

wherein $R^{11}$ is $C_1$-$C_6$ alkyl,

$R^{12}$ is phenyl, optionally substituted with one or more substituents selected from the group consisting of halo, $C_1$-$C_6$ alkyl, or $C_1$-$C_6$ alkoxy, and
$R^{13}$ is H, halo, or $C_1$-$C_6$ alkoxy,

or a pharmaceutically acceptable salt thereof.

In an embodiment, $R^{13}$ is H.

In certain preferred embodiments, the compound of formula (III) is:

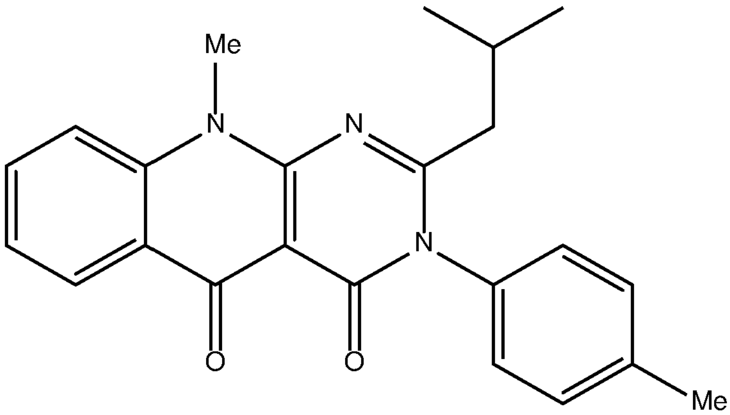,

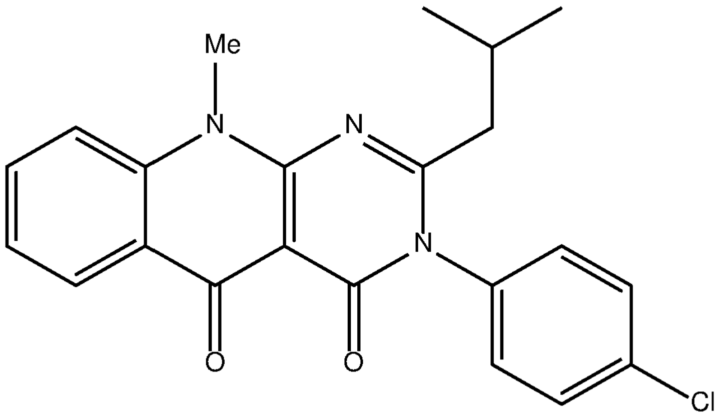,

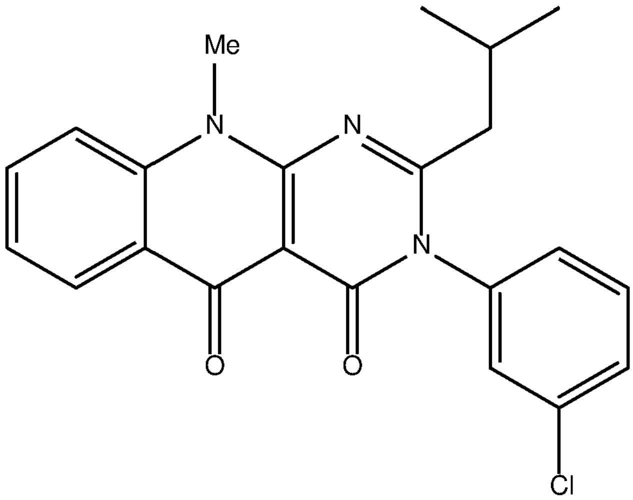,

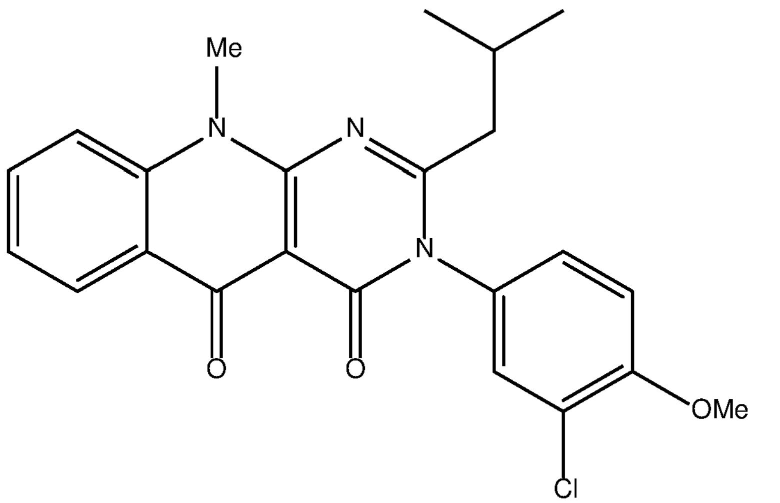

,

or

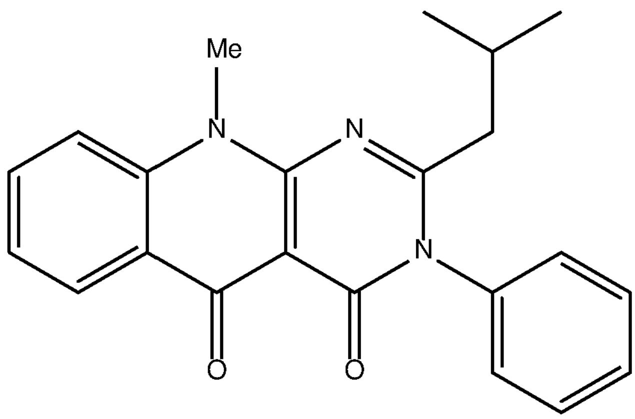

.

Referring now to terminology used generically herein, the term "alkyl" means a straight-chain or branched alkyl substituent containing from, for example, 1 to about 6 carbon atoms, preferably from 1 to about 4 carbon atoms, more preferably from 1 to 2 carbon atoms. When the alkyl contains 1 or 2 carbon atoms, the alkyl must be straight-chain. When the alkyl contains from 3 to about 6 carbon atoms, the alkyl can be straight-chain or branched. Examples of such substituents include methyl, ethyl, propyl, isopropyl, *n*-butyl, *sec*-butyl, isobutyl, *tert*-butyl, pentyl, isoamyl, hexyl, and the like.

The term "aryl" refers to an unsubstituted or substituted aromatic carbocyclic substituent, as commonly understood in the art, and the term "$C_6$-$C_{10}$ aryl" includes phenyl and naphthyl. It is understood that the term aryl applies to cyclic substituents that are planar and comprise $4n+2$ $\pi$ electrons, according to Hückel's Rule.

The term "alkoxy" means a straight-chain or branched alkoxy substituent containing from, for example, 1 to about 6 carbon atoms, preferably from 1 to about 4 carbon atoms, more preferably from 1 to 2 carbon atoms. Examples of such substituents include methoxy, ethoxy, propoxy, isopropoxy, *n*-butoxy, *sec*-butoxy, isobutoxy, *tert*-butoxy, pentyloxy, isoamyloxy, hexyloxy, and the like.

The term "halo" or "halogen," as used herein, means a substituent selected from Group VIIA, such as, for example, fluorine, bromine, chlorine, and iodine.

Whenever a range of the number of atoms in a structure is indicated (e.g., a $C_1$-$C_6$, $C_1$-$C_4$, or $C_2$-$C_6$, $C_2$-$C_4$ alkyl, alkenyl, alkynyl, etc.), it is specifically contemplated that any sub-range or individual number of carbon atoms falling within the indicated range also can be used. Thus, for instance, the recitation of a range of 1-6 carbon atoms (e.g., $C_1$-$C_6$), 1-4 carbon atoms (e.g., $C_1$-$C_4$), 1-3 carbon atoms (e.g., $C_1$-$C_3$), or 2-6 carbon atoms (e.g., $C_2$-$C_6$) as used with respect to any chemical group (e.g., alkyl, alkylamino, etc.) referenced herein encompasses and specifically describes 1, 2, 3, 4, 5, and/or 6 carbon atoms, as appropriate, as well as any sub-range thereof (e.g., 1-2 carbon atoms, 1-3 carbon atoms, 1-4 carbon atoms, 1-5 carbon atoms, 1-6 carbon atoms, 2-3 carbon atoms, 2-4 carbon atoms, 2-5 carbon atoms, 2-6 carbon atoms, 3-4 carbon atoms, 3-5 carbon atoms, 3-6 carbon atoms, 4-5 carbon atoms, and/or 4-6 carbon atoms,

etc., as appropriate). Similarly, the recitation of a range of 6-10 carbon atoms (e.g., $C_6$-$C_{10}$) as used with respect to any chemical group (e.g., aryl) referenced herein encompasses and specifically describes 6, 7, 8, 9, and/or 10 carbon atoms, as appropriate, as well as any sub-range thereof (e.g., 6-10 carbon atoms, 6-9 carbon atoms, 6-8 carbon atoms, 6-7 carbon atoms, 7-10 carbon atoms, 7-9 carbon atoms, 7-8 carbon atoms, 8-10 carbon atoms, and/or 8-9 carbon atoms, etc., as appropriate).

The phrase "pharmaceutically acceptable salt" is intended to include nontoxic salts synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, nonaqueous media such as ether, ethyl acetate, ethanol, isopropanol, or acetonitrile are preferred. Lists of suitable salts are found in Remington's Pharmaceutical Sciences, 18th ed., Mack Publishing Company, Easton, PA, 1990, p. 1445, and Journal of Pharmaceutical Science, 66, 2-19 (1977).

Suitable bases include inorganic bases such as alkali and alkaline earth metal bases, e.g., those containing metallic cations such as sodium, potassium, magnesium, calcium and the like. Non-limiting examples of suitable bases include sodium hydroxide, potassium hydroxide, sodium carbonate, and potassium carbonate. Suitable acids include inorganic acids such as hydrochloric acid, hydrobromic acid, hydroiodic acid, sulfuric acid, phosphoric acid, and the like, and organic acids such as p-toluenesulfonic, methanesulfonic acid, benzenesulfonic acid, oxalic acid, p-bromophenylsulfonic acid, carbonic acid, succinic acid, citric acid, benzoic acid, acetic acid, maleic acid, tartaric acid, fatty acids, long chain fatty acids, and the like. Preferred pharmaceutically acceptable salts of inventive compounds having an acidic moiety include sodium and potassium salts. Preferred pharmaceutically acceptable salts of inventive compounds having a basic moiety (e.g., a dimethylaminoalkyl group) include hydrochloride and hydrobromide salts. The compounds of the present invention containing an acidic or basic moiety are useful in the form of the free base or acid or in the form of a pharmaceutically acceptable salt thereof.

It should be recognized that the particular counterion forming a part of any salt of this invention is usually not of a critical nature, so long as the salt as a whole is pharmacologically acceptable and as long as the counterion does not contribute undesired qualities to the salt as a whole.

It is further understood that the above compounds and salts may form solvates, or exist in a substantially uncomplexed form, such as the anhydrous form. As used herein, the term "solvate" refers to a molecular complex wherein the solvent molecule, such as the crystallizing solvent, is incorporated into the crystal lattice. When the solvent incorporated in the solvate is water, the molecular complex is called a hydrate. Pharmaceutically acceptable solvates include hydrates, alcoholates such as methanolates and ethanolates, acetonitrilates and the like. These compounds can also exist in polymorphic forms.

The present invention further provides a pharmaceutical composition comprising a compound as described above and a pharmaceutically acceptable carrier. The present invention provides a pharmaceutical composition comprising a pharmaceutically acceptable carrier and an effective amount, e.g., a therapeutically effective amount, including a prophylactically effective amount, of one or more of the aforesaid compounds, or salts thereof, of the present invention.

The pharmaceutically acceptable carrier can be any of those conventionally used and is limited only by chemico-physical considerations, such as solubility and lack of reactivity with the compound, and by the route of administration. It will be appreciated by one of skill in the art that, in addition to the following described pharmaceutical compositions; the compounds of the present invention can be formulated as inclusion complexes, such as cyclodextrin inclusion complexes, or liposomes.

The pharmaceutically acceptable carriers described herein, for example, vehicles, adjuvants, excipients, or diluents, are well known to those who are skilled in the art and are readily available to the public. It is preferred that the pharmaceutically acceptable carrier be one which is chemically inert to the active compounds and one which has no detrimental side effects or toxicity under the conditions of use.

The choice of carrier will be determined in part by the particular active agent, as well as by the particular method used to administer the composition. Accordingly, there is a wide variety of suitable formulations of the pharmaceutical composition of the present invention. The following formulations for oral, aerosol, parenteral, subcutaneous, intravenous, intraarterial, intramuscular, interperitoneal, intrathecal, rectal, and vaginal administration are merely exemplary and are in no way limiting.

Formulations suitable for oral administration can consist of (a) liquid solutions, such as an effective amount of the compound dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and cornstarch. Tablet forms can include one or more of lactose, sucrose, mannitol, corn starch,

potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such carriers as are known in the art.

The compounds of the present invention, alone or in combination with other suitable components, can be made into aerosol formulations to be administered via inhalation. These aerosol formulations can be placed into pressurized acceptable propellants, such as dichlorodifluoromethane, propane, nitrogen, and the like. They also may be formulated as pharmaceuticals for non-pressured preparations, such as in a nebulizer or an atomizer.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters. Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylene-polypropylene copolymers, (d) amphoteric detergents such as, for example, alkyl-beta-aminopropionates, and 2-alkyl-imidazoline quaternary ammonium salts, and (3) mixtures thereof.

The parenteral formulations will typically contain from about 0.5 to about 25% by weight of the active ingredient in solution. Suitable preservatives and buffers can be used in such formulations. In order to minimize or eliminate irritation at the site of injection, such compositions may contain one or more nonionic surfactants having a hydrophile-lipophile balance (HLB) of from about 12 to about 17. The quantity of surfactant in such formulations ranges from about 5 to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multidose sealed containers, such as ampoules and vials, and can be stored in a freeze-dried (lyophilized) condition requiring only the addition of the sterile liquid carrier, for example, water, for injections, immediately prior to use. Extemporaneous injection solutions and suspensions can be prepared from sterile powders, granules, and tablets of the kind previously described.

The compounds of the present invention may be made into injectable formulations. The requirements for effective pharmaceutical carriers for injectable compositions are well known to those of ordinary skill in the art. See Pharmaceutics and Pharmacy Practice, J. B. Lippincott Co., Philadelphia, Pa., Banker and Chalmers, eds., pages 238-250 (1982), and ASHP Handbook on Injectable Drugs, Toissel, 4th ed., pages 622-630 (1986).

Topical formulations, including those that are useful for transdermal drug release, are well-known to those of skill in the art and are suitable in the context of the invention for application to skin. Topically applied compositions are generally in the form of liquids, creams, pastes, lotions and gels. Topical administration includes application to the oral mucosa, which includes the oral cavity, oral epithelium, palate, gingival, and the nasal mucosa. In some embodiments, the composition contains at least one active component and a suitable vehicle or carrier. It may also contain other components, such as an anti-irritant. The carrier can be a liquid, solid or semi-solid. In embodiments, the composition is an aqueous solution. Alternatively, the composition can be a dispersion, emulsion, gel, lotion or cream vehicle for the various components. In one embodiment, the primary vehicle is water or a biocompatible solvent that is substantially neutral or that has been rendered substantially neutral. The liquid vehicle can include other materials, such as buffers, alcohols, glycerin, and mineral oils with various emulsifiers or dispersing agents as known in the art to obtain the desired pH, consistency and viscosity. It is possible that the compositions can be produced as solids, such as powders or granules. The solids can be applied directly or dissolved in water or a biocompatible solvent prior to use to form a solution that is substantially neutral or that has been rendered substantially neutral and that can then be applied to the target

site. In embodiments of the invention, the vehicle for topical application to the skin can include water, buffered solutions, various alcohols, glycols such as glycerin, lipid materials such as fatty acids, mineral oils, phosphoglycerides, collagen, gelatin and silicone based materials.

Additionally, the compounds of the present invention may be made into suppositories by mixing with a variety of bases, such as emulsifying bases or water-soluble bases. Formulations suitable for vaginal administration may be presented as pessaries, tampons, creams, gels, pastes, foams, or spray formulas containing, in addition to the active ingredient, such carriers as are known in the art to be appropriate.

The dose administered to a mammal, particularly, a human, in accordance with the present invention should be sufficient to effect the desired response. Such responses include reversal or prevention of the adverse effects of the disease for which treatment is desired or to elicit the desired benefit. One skilled in the art will recognize that dosage will depend upon a variety of factors, including the age, condition, and body weight of the human, as well as the source, particular type of the disease, and extent of the disease in the human. The size of the dose will also be determined by the route, timing and frequency of administration as well as the existence, nature, and extent of any adverse side-effects that might accompany the administration of a particular compound and the desired physiological effect. It will be appreciated by one of skill in the art that various conditions or disease states may require prolonged treatment involving multiple administrations.

Suitable doses and dosage regimens can be determined by conventional range-finding techniques known to those of ordinary skill in the art. Generally, treatment is initiated with smaller dosages that are less than the optimum dose of the compound. Thereafter, the dosage is increased by small increments until the optimum effect under the circumstances is reached. The present inventive method typically will involve the administration of about 0.1 to about 300 mg of one or more of the compounds described above per kg body weight of the animal or mammal.

The therapeutically effective amount of the compound or compounds administered can vary depending upon the desired effects and the factors noted above. Typically, dosages will be between 0.01 mg/kg and 250 mg/kg of the subject's body weight, and more typically between about 0.05 mg/kg and 100 mg/kg, such as from about 0.2 to about 80 mg/kg, from about 5 to about 40 mg/kg or from about 10 to about 30 mg/kg of the subject's body weight. Thus, unit dosage forms can be formulated based upon the suitable ranges recited above and the subject's body weight. The term "unit dosage form" as used herein refers to a physically discrete unit of therapeutic agent appropriate for the subject to be treated.

Alternatively, dosages are calculated based on body surface area and from about 1 $mg/m^2$ to about 200 $mg/m^2$, such as from about 5 $mg/m^2$ to about 100 $mg/m^2$ will be administered to the subject per day. In particular embodiments, administration of the therapeutically effective amount of the compound or compounds involves administering to the subject from about 5 $mg/m^2$ to about 50 $mg/m^2$, such as from about 10 $mg/m^2$ to about 40 $mg/m^2$ per day. It is currently believed that a single dosage of the compound or compounds is suitable, however a therapeutically effective dosage can be supplied over an extended period of time or in multiple doses per day. Thus, unit dosage forms also can be calculated using a subject's body surface area based on the suitable ranges recited above and the desired dosing schedule.

Chemistry

Representative routes to the compounds of the invention, for example, those set forth in Tables 1-3, are described below.

The compound of formula (I) can be prepared using the following exemplary reaction scheme:

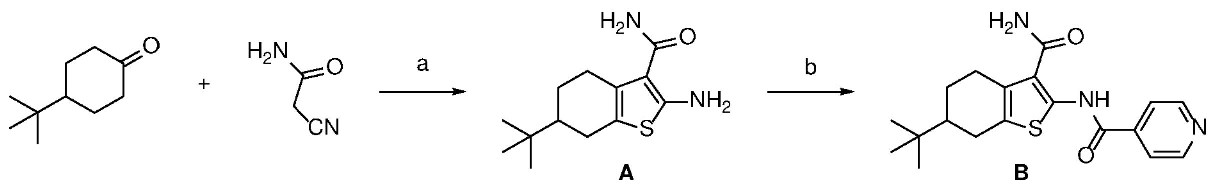


Reagents and conditions: (a) sulfur (1 equiv), morpholine (1.5 equiv), ethanol, 120°C (microwave), 15 min; (b) isonicotinoyl chloride, pyridine, $CH_2Cl_2$, rt, 16 h

Reaction of a substituted cycloalkanone such as 4-t-butylcyclohexanone with cyanoacetamide in the presence of elemental sulfur and base such as morpholine in a solvent such as ethanol with heating under microwave irradiation provides aminothiophene **A.** Acylation of aminothiophene **A** with an acylating agent such as an acyl chloride (e.g., isonicotinoyl chloride) in the presence of a base such as pyridine in a solvent such as dichloromethane provides a compound of formula (I), exemplified as compound **B**.

The compound of formula (I) can be prepared using the following exemplary reaction scheme:

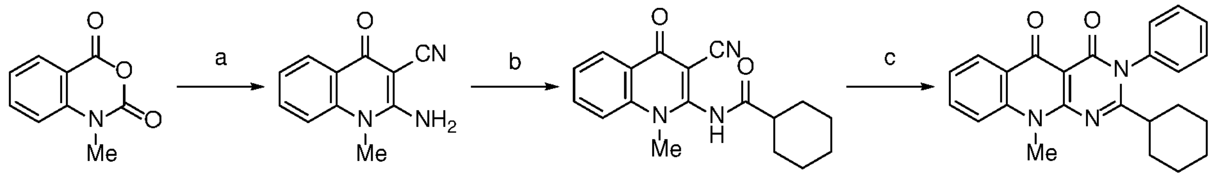


C D E

Reagents and conditions: (a) malononitrile (1.1 equiv), triethylamine (0.2 equiv), DMF, 120°C (microwave), 45 min; (b) cyclohexylcarbonyl chloride, pyridine, rt, 16 h; (c) aniline (5.0 equiv), $Cu(OAc)_2$ (0.5 equiv), water, 100°C (microwave), 2 h

Reaction of isatoic anhydride with malononitrile in the presence of a base such as trimethylamine in a solvent such as N,N-dimethylformamide (DMF) with heating under microwave irradiation provides 2-amino-1-methyl-4-oxo-1,4-dihydroquinoline-3-carbonitrile **C.** Acylation of compound **C** with an acylating agent such as an acid chloride (e.g., cyclohexylcarbonyl chloride) in the presence of a base such as pyridine provides amide compound **D.** Reaction of compound **D** with an amine such as aniline in the presence of $Cu(OAc)_2$ and water with heating under microwave irradiation provides tricyclic compound **E.**

The invention further provides a compound of formula (I):

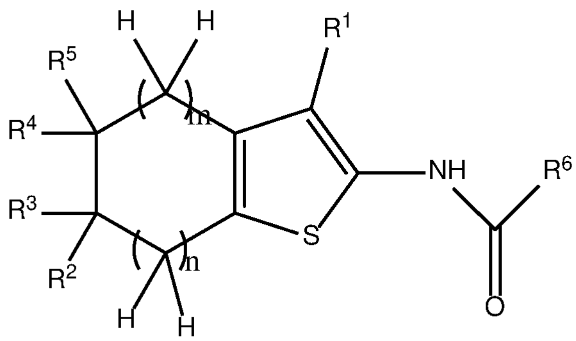

(I)

wherein $R^1$ is -CN or -$CONR^7R^8$,

$R^2$ is $C_1$-$C_6$ alkyl or $C_6$-$C_{10}$ aryl and $R^3$-$R^5$ are all H or wherein $R^4$ is $C_1$-$C_6$ alkyl or $C_6$-$C_{10}$ aryl and $R^2$, $R^3$, and $R^5$ are all H,

m and n are independently 1 or 2,

$R^6$ is 2-pyridyl, 3-pyridyl, 4-pyridyl, or 2-quinolinyl, and

$R^7$ and $R^8$ are independently H or $C_1$-$C_6$ alkyl,

wherein $R^6$ is optionally substituted with one or more groups selected from the group consisting of halo, $C_1$-$C_6$ alkyl, and $C_1$-$C_6$ alkoxy,

or a pharmaceutically acceptable salt thereof, and/or

a compound of formula (II):

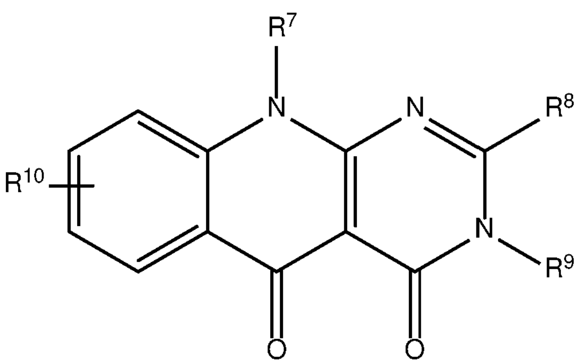

(II)

wherein $R^7$ is $C_1$-$C_6$ alkyl,
$R^8$ is $C_1$-$C_6$ alkyl, $C_3$-$C_8$-cycloalkyl, or $C_3$-$C_8$-cycloalkyl-$C_1$-$C_6$-alkylene,
$R^9$ is phenyl, optionally substituted with one or more substituents selected from the group consisting of halo, $C_1$-$C_6$ alkyl, or $C_1$-$C_6$ alkoxy, and
$R^{10}$ is H, halo, or $C_1$-$C_6$ alkoxy,
or a pharmaceutically acceptable salt thereof for use in treating or preventing a disease or disorder responsive to activation of a D1 dopamine receptor agonist in a mammal in need thereof.

In an embodiment, the compound is of formula (I).
In an embodiment, m and n are both 1.
In an embodiment, $R^1$ is -$CONR^7R^8$.
In an embodiment, $R^7$ and $R^8$ are both H
In certain embodiments, $R^2$ is $C_1$-$C_6$ alkyl or $C_6$-$C_{10}$ aryl and $R^3$-$R^5$ are all H.
In certain embodiments, $R^6$ is 2-pyridyl, 3-pyridyl, or 4-pyridyl.
In certain preferred embodiments, the compound is selected from the group consisting of:

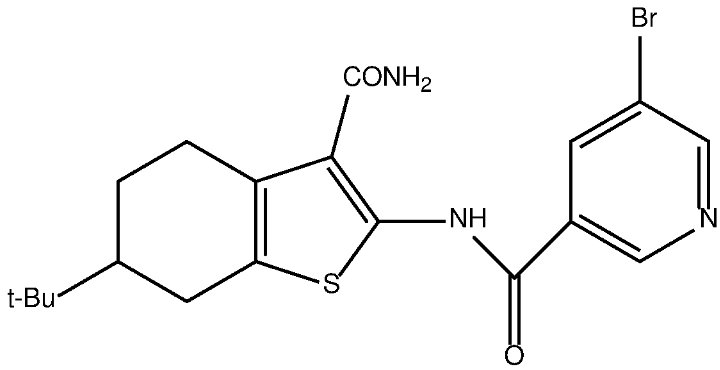
,

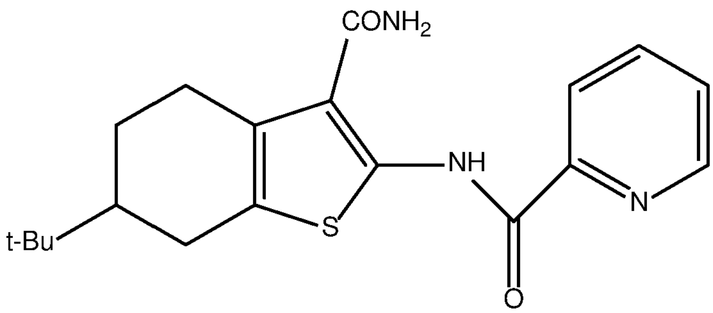
,

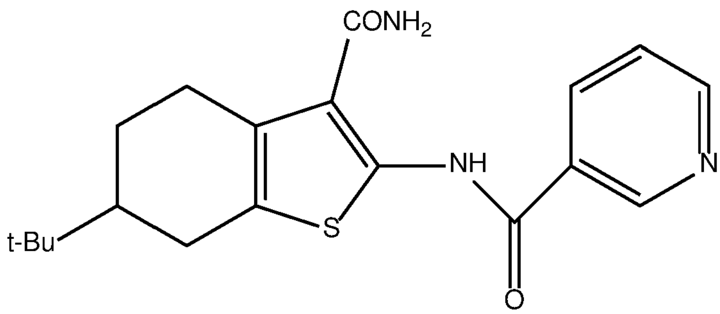
,

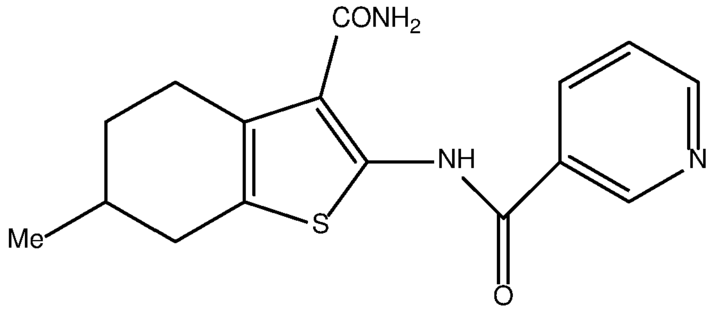

,

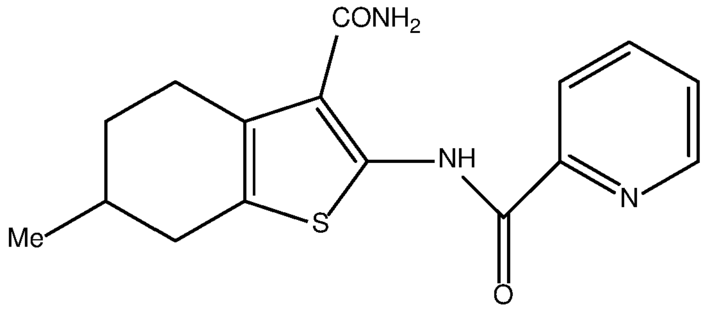

In certain embodiments, $R^6$ is 2-quinolinyl.

In certain particular embodiments, the compound is:

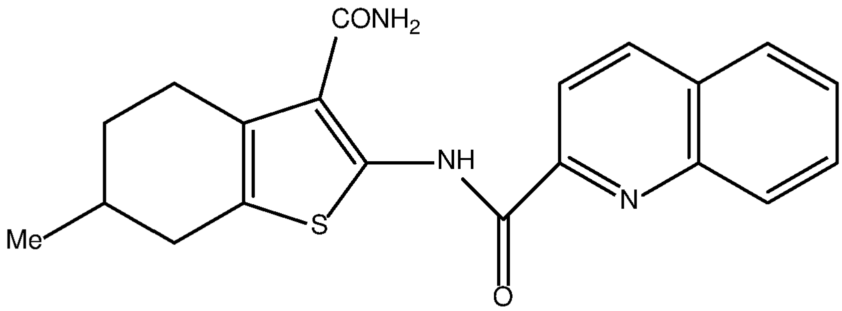

or

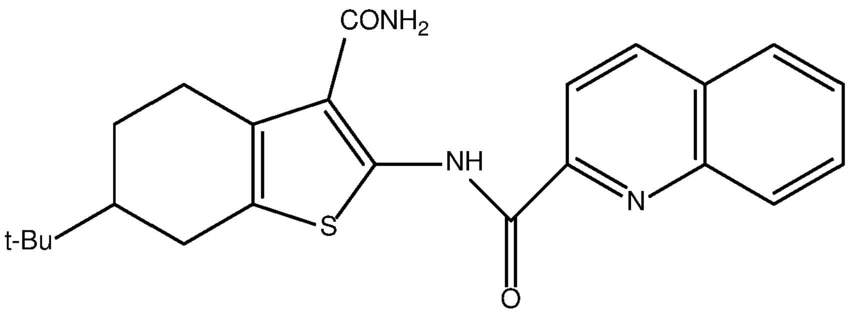

.

In an embodiment, the compound is formula (II).

In certain embodiments, $R^7$ is methyl.

In certain embodiments, $R^{10}$ is H.

In certain embodiments, $R^8$ is $C_1$-$C_6$ alkyl.

In certain preferred embodiments, the compound is selected from the group consisting of:

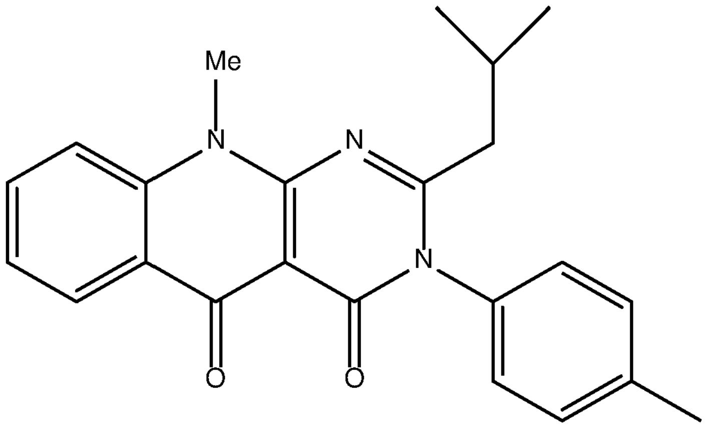

,

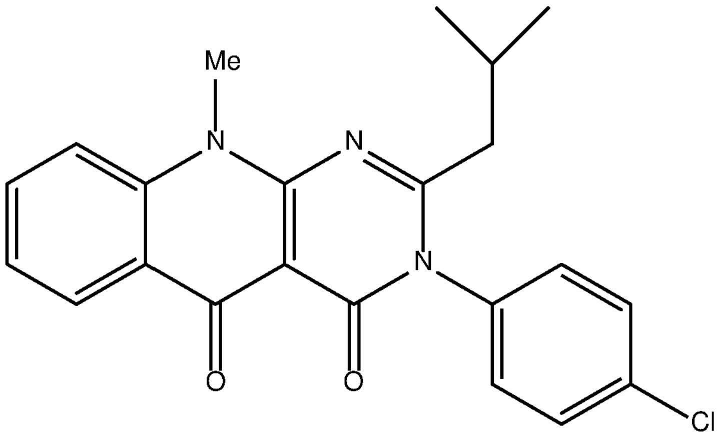

,

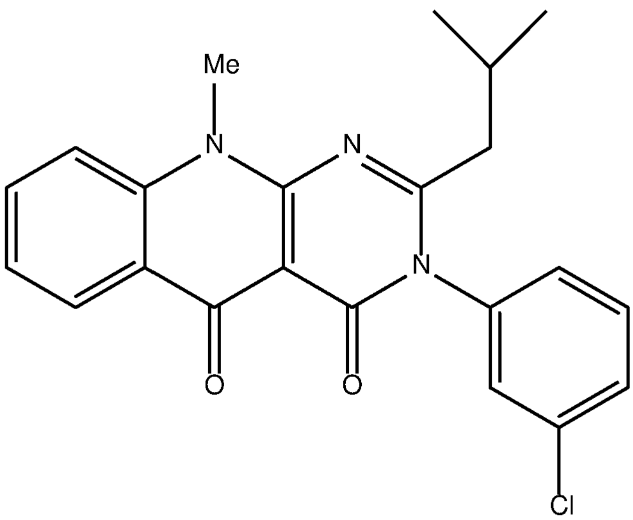

,

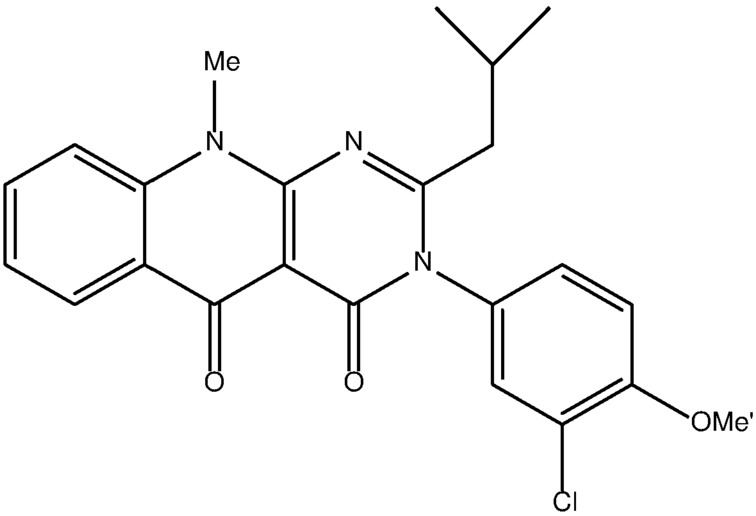

,

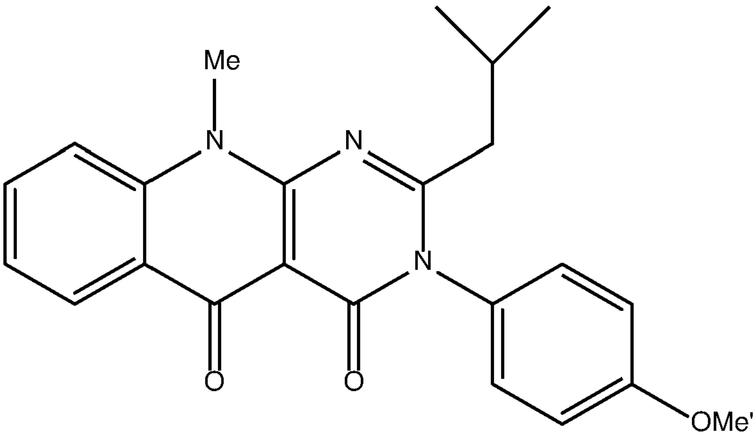

,

and

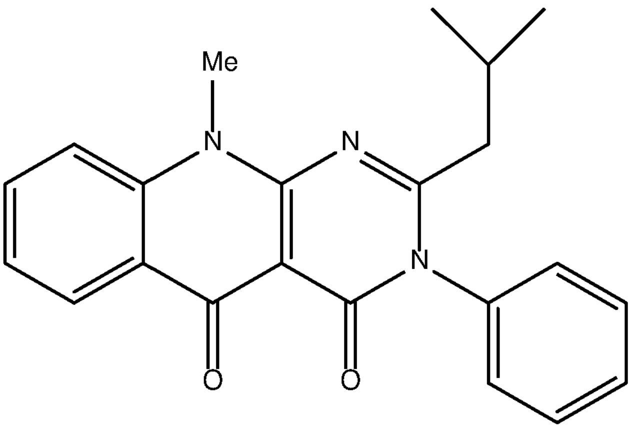

.

In certain embodiments, $R^8$ is $C_3$-$C_8$ cycloalkyl.

In certain preferred embodiments, the compound is selected from the group consisting of:

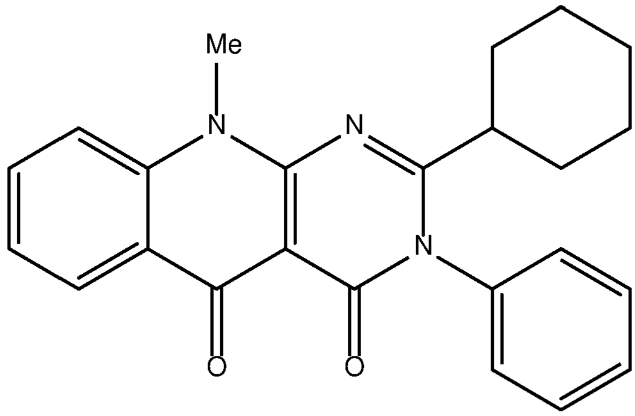

,

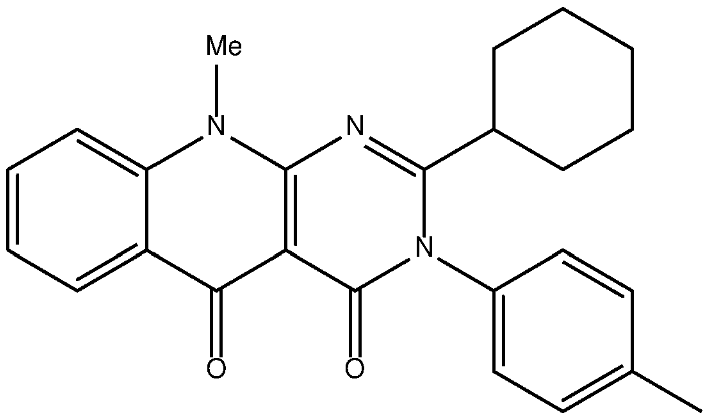

,

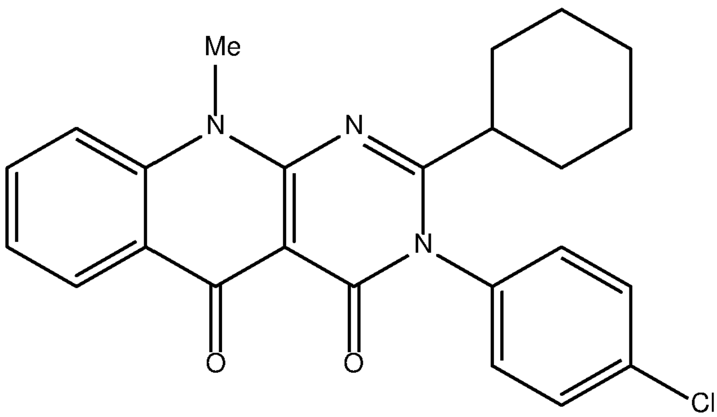

,

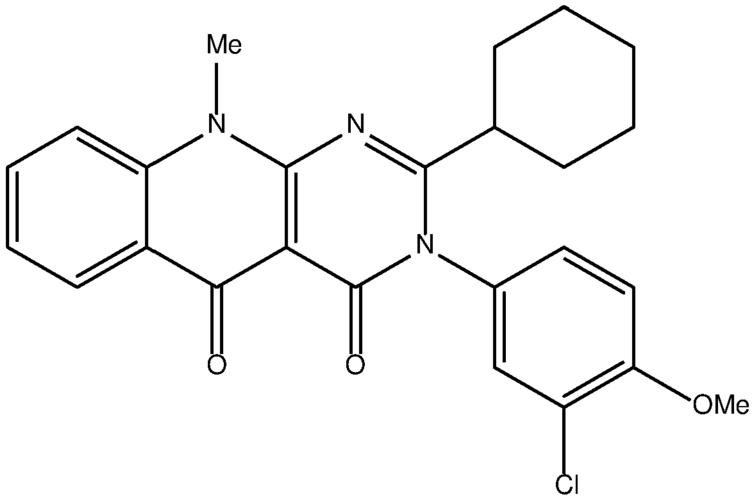

,

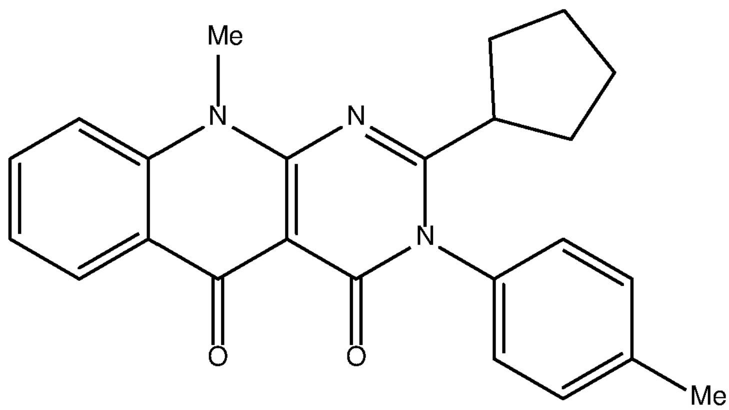

,

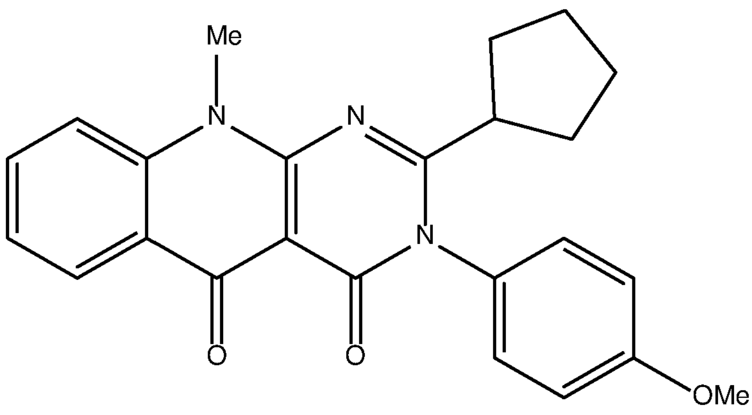

,

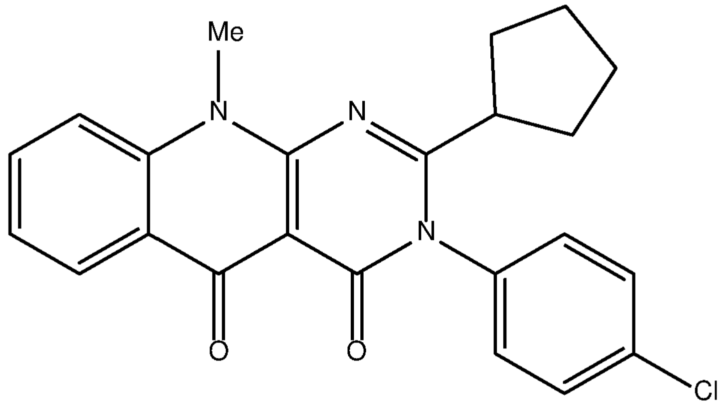
,

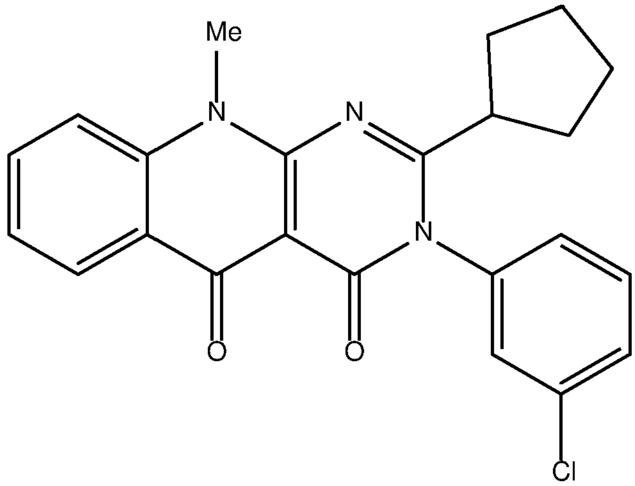
,

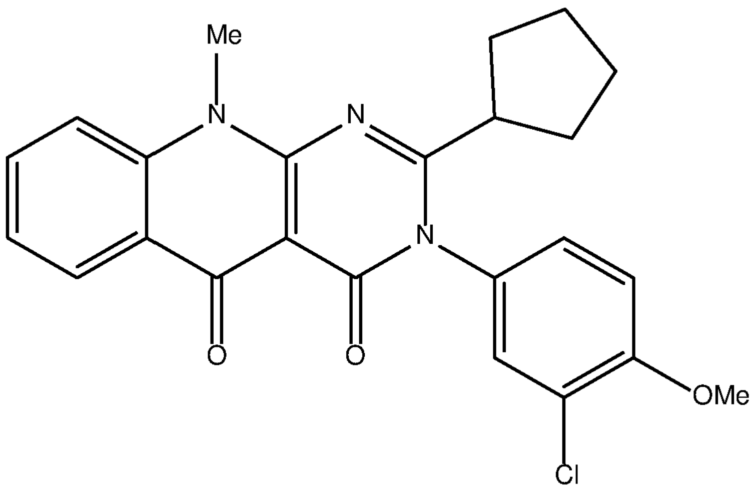
,

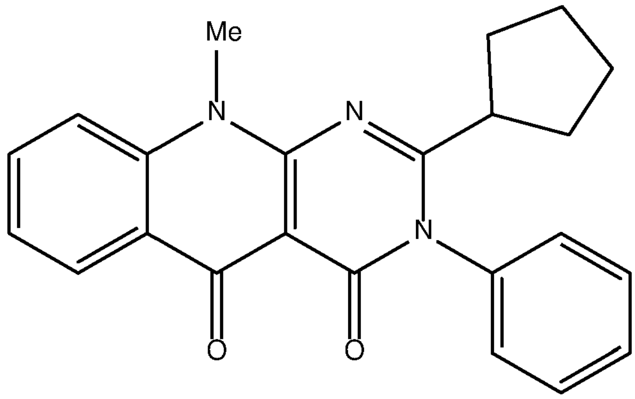

,

and

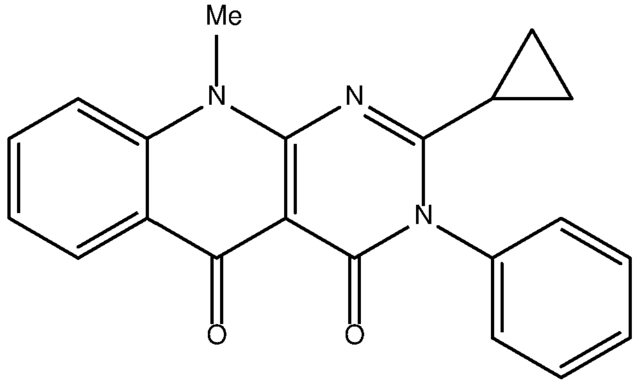

.

In certain embodiments, $R^8$ is $C_3$-$C_8$ cycloalkyl-$C_1$-$C_6$ alkylene.

In certain preferred embodiments, the compound is selected from the group consisting of:

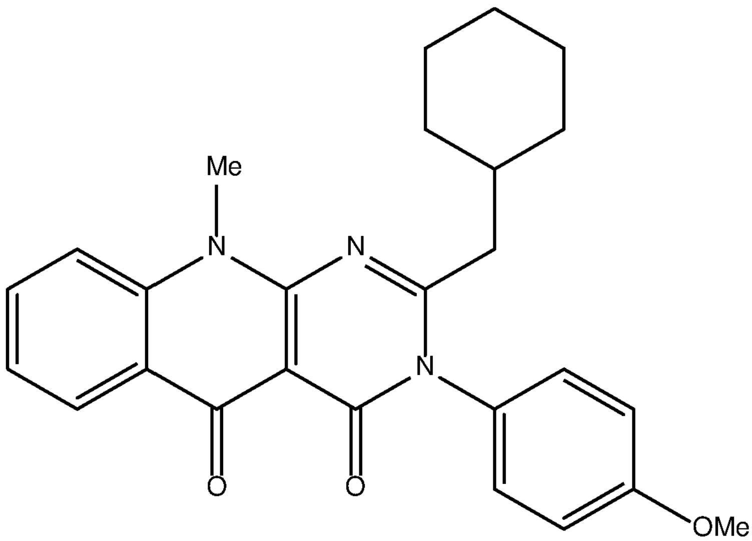

,

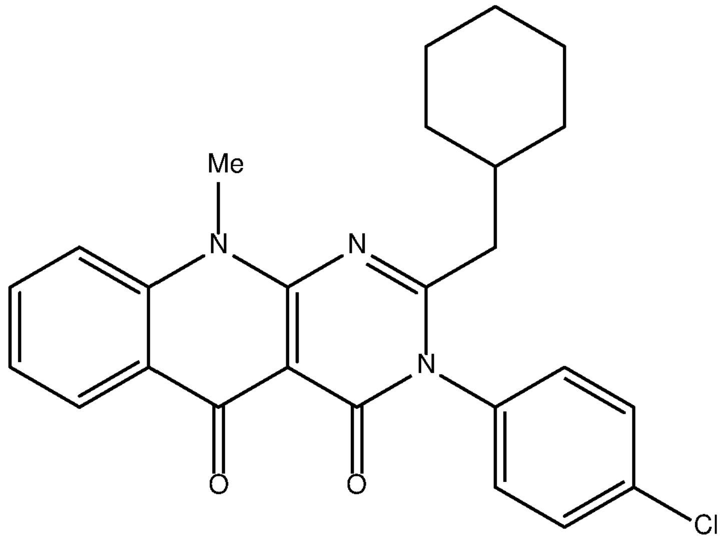

,

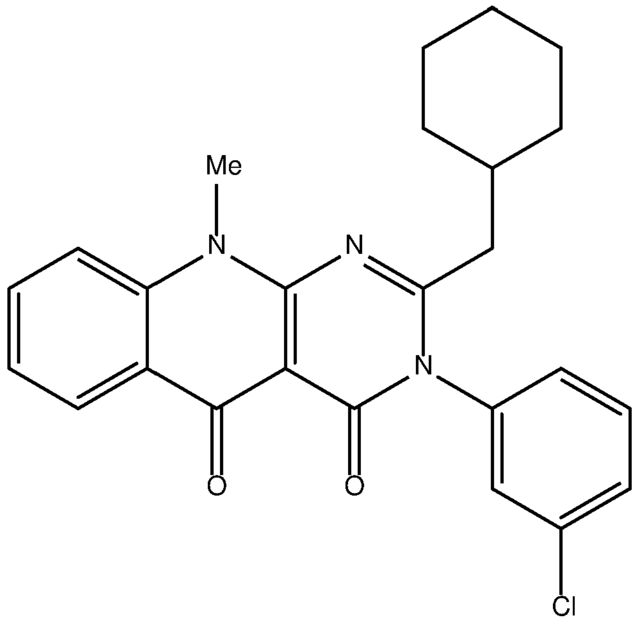

,

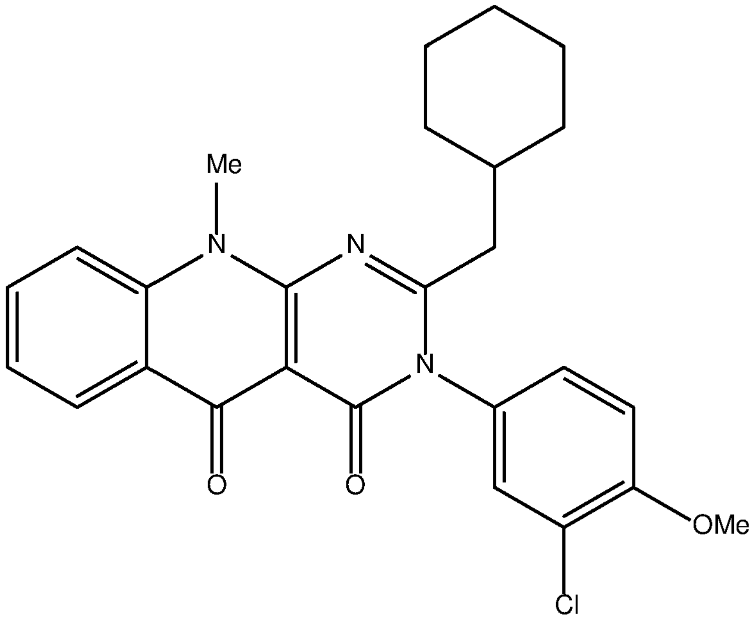

,

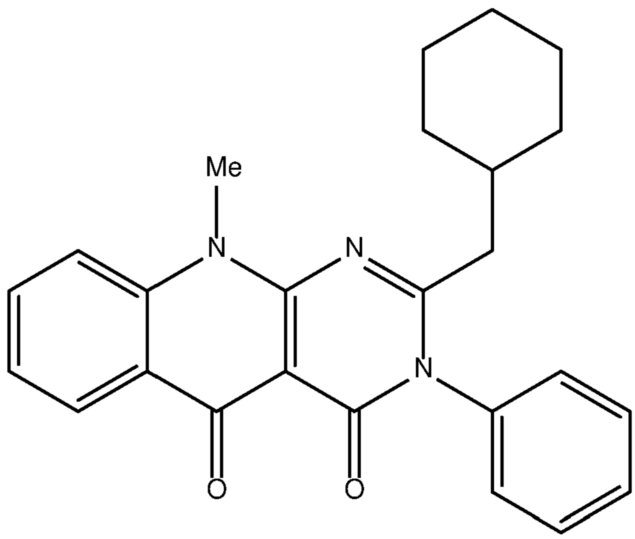

,

and

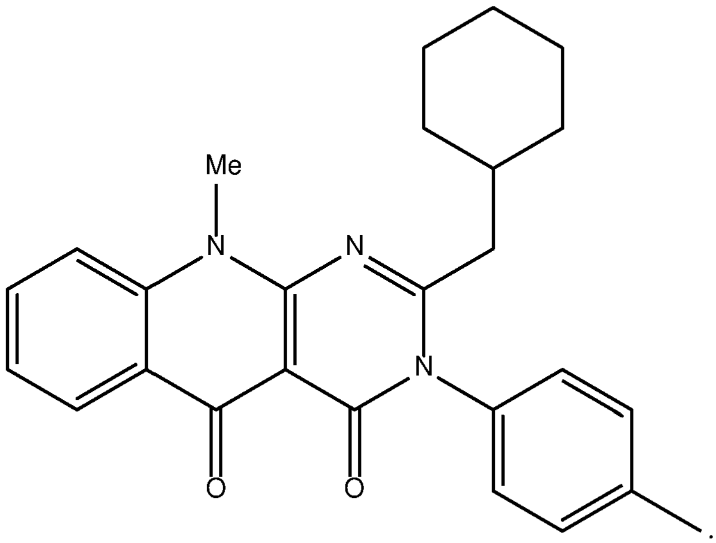

In certain embodiments, the dopamine D1 receptor agonist is dopamine.

In a particular embodiment, the dopamine is endogenous dopamine.

In certain embodiments, the disease or disorder involves an impairment of mood, cognition, memory, movement or motor skills, or a combination thereof.

In these embodiments, the administration improves mood, cognition, memory, movement or motor skills, or a combination thereof.

In preferred embodiments, the disease or disorder is Alzheimer's Disease, schizophrenia, Parkinson's disease, a dyskinesia, or Huntington's disease.

In a particular embodiment, the dyskinesia is an L-DOPA-induced dyskinesia.

In certain embodiments, the compound for use as described above further comprises administering to the mammal a dopamine D1 receptor agonist other than dopamine. Non-limiting examples of suitable dopamine D1 receptor agonists include apomorphine, fenoldopam, SKF38393, SKF77434, and L-DOPA. As is well known in the art, L-DOPA can be converted to dopamine *in vivo* and is thus a prodrug of dopamine. This embodiment is based on the discovery that the compound of the invention acts as a positive allosteric modulator of the dopamine D1 receptor and potentiates dopamine's affinity on both G-protein and β-arrestin mediated signaling as well as increasing the maximum dopamine-stimulated response or responses, without displaying any intrinsic agonist action.

In a particular embodiment, the dopamine D1 receptor agonist is the prodrug L-DOPA and the disease or disorder is Parkinson's disease.

The following examples further illustrate the invention but, of course, should not be construed as in any way limiting its scope.

***Materials*** - Original screening quantities of compounds **1** and **28** were obtained from the Molecular Libraries Screening Center Network Library. Compounds were subsequently purchased from MolPort (Riga, Latvija), for follow-up triage studies. Finally, both compounds **1** and **28** were synthesized in-house at the Kansas Specialized Chemistry Center, as described. Compounds were tested in-house via NMR to confirm purity (>99%) and structural accuracy. Identical results were obtained with all batches from all suppliers. All other chemicals were obtained from Sigma-Aldrich (St. Louis, MO) unless otherwise indicated within the methods. All tissue culture media and components were obtained from Mediatech, inc. (Manassas, VA).

***Calcium Mobilization Assay* (high-throughput screening assay).** HEK293T cells were stably transfected with human D1R and G15 protein using the Flp-In T-Rex expression system (Life Technologies, Grand Island, NY). Cells were first stably transfected with the human D1R in pcDNA3.1+ and selected with G418. Colonies were validated by radioligand binding assay for D1R expression. Cells were then stably transfected with G15 protein in G15/pIREShygro (Clonetech) vector that imparted hygromycin resistance and subsequently selected with hygromycin. G15 expression was validated from individual colonies using the $Ca^{2+}$ mobilization assay. Cells lines giving the most robust calcium response were selected for screening assays. D1R-stimulated calcium mobilization was measured using methods similar to those previously published (Chun et al., 2013). Briefly, D1R-G15 cells (4,000 cells/well and 3 μL/well) were added directly to the culture media and plated in 1536-well, optical, clear bottom, black-walled plates (Greiner Bio-one, Monroe, NC). The following day, cells were incubated for 60 min at room temperature in the dark with Fluo-8 NW calcium dye in the presence of an extracellular signal quencher (Screen Quest™ Fluo-8 NW Calcium Assay Kit, AAT Bioquest, Inc., Sunnyvale, CA), as recommended by the manufacturer. The plates were then treated with 40 μM of test compound and

read kinetically in real time (every 0.6 sec) both before compound addition, and for two minutes after compound addition. Compound additions were done in unison using an onboard 1536-pintool while continuously reading at an excitation wavelength of 480 nm and an emission wavelength of 540 nm on an FDSS 7000 (Hamamatsu, Bridgewater, NJ). For potentiation assays, cells were first treated with test compound as described above followed by a second addition of an $EC_{20}$ concentration of DA (~300 nM) to give a small response that allows for measurement of the potentiation of the dopamine response. In this paradigm both agonist activity and potentiation can be examined in a single read. Data were recorded and quantified as maximum minus minimum (max-min) relative florescence units (RFU) within the assay window using FDSS software. Hit compounds were defined as compounds that significantly (>3 SD) potentiated the $EC_{20}$ response of DA, and were chosen for further study.

***cAMP Accumulation Assay.*** Assays were performed on D1R-HEK293 (Codex Biosolutions, Gaithersburg, MD) cells stably expressing the human D1R. HEK293 cell lines were maintained in Dulbecco's modified Eagles medium, supplemented with 10% fetal bovine serum, 100 U/ml penicillin, 100 $\mu$g/ml streptomycin, 1 mM sodium pyruvate, and 250 $\mu$g/ml G418 and incubated at 37°C, 5% $CO_2$, and 90% humidity. For the assay, cells were seeded in 384-well black, clear-bottomed plates at a density of 5,000 cells/well, 10 $\mu$L/well. After 18-24 h incubation at 37°C, 5% $CO_2$, and 90% humidity, the media was removed and replaced with 5 $\mu$L/well PBS. Cells were then treated with 2.5 $\mu$L of varying concentrations of compound diluted in PBS containing 25 $\mu$M 4-(3-Butoxy-4-methoxybenzyl)imidazolin-2-one (Ro 20-1724), 1 $\mu$M propranolol, and 0.2 mM sodium metabisulfite and incubated for 30 min at 37°C, 5% $CO_2$, and 90% humidity. cAMP was measured using the DiscoverX HitHunter kit (DiscoverX, Fremont, CA) according to the manufacturer's recommendations. Briefly, antibody and working solution was added to each well according to the manufacturer's protocol, and incubated in the dark at room temperature for 60 min. Following incubation, enzyme acceptor reagent was added to the plates and luminescence (RLU) was measured (FDSS $\mu$Cell, Hamamatsu Photonics K. K., Bridgewater, NJ) following a 3 h incubation in the dark at room temperature. Data are represented as a percentage of the control maximum dopamine-stimulated cAMP signal.

***β-arrestin Recruitment Assay.*** Agonist-mediated recruitment of β-arrestin-2 to all five DARs was determined using the DiscoveRx PathHunter complementation assay (DiscoverX Inc, Fremont, CA), as previously described (Free, et. al., 2014; Conroy, et. al., 2015). Briefly, CHO-K1 cells stably expressing either human D1R, D2R, D3R, D4R or D5R, as indicated, were seeded in cell plating (CP) media (DiscoverX) at a density of 2,625 cells/well and 7.5 $\mu$L/well in 384-well black, clear-bottom plates. Following 18-24 h of incubation, the cells were treated with the indicated concentrations of compound in PBS buffer containing 0.2 mM sodium metabisulfite, and incubated at 37°C for 90 min. DiscoverX reagent was added to cells according to the manufacturer's protocol, followed by a 60 min incubation in the dark at room temperature. Luminescence was measured on a Hamamatsu FDSS $\mu$-Cell reader (Hamamatsu, Bridgewater, NJ) and data were collected using the FDSS software. Data were collected as relative luminescence units (RLUs) and normalized to a percentage of the control luminescence seen with a maximum concentration of dopamine, with zero percent being RLUs produced in the absence of any compound.

***Radioligand Binding Assays.*** Radioligand competition binding assays were conducted with slight modifications as previously described (Chun et al., 2013). HEK293 cells stably transfected with human D1R (Codex Biosolutions, Inc., Gaithersburg, MD) were dissociated from plates using EBSS lacking calcium and magnesium, and intact cells were collected by centrifugation at 1,000 × g for 10 min. Cells were re-suspended and lysed using 5 mM Tris-HCl and 5 mM $MgCl_2$ at pH 7.4 at 4°C. Cell lysate was pelleted by centrifugation at 30,000 × g for 30 min and re-suspended in 50 mM Trizma + 5 mM $MgCl_2$ at pH 7.4. Cell membrane preparations (100 $\mu$l, containing ~25 $\mu$g protein) were incubated for 90 min at room temperature with the indicated concentrations of MLS1082 or MLS6585 in the presence or absence of dopamine and 0.5 nM [$^3$H]-SCH23390 in a final reaction volume of 250 $\mu$l. Non-specific binding was determined in the presence of 4 $\mu$M (+)-butaclamol. Bound ligand was separated from free by filtration through a PerkinElmer Unifilter-96 GF/C 96-well micro-plate using the PerkinElmer Unifilter-96 Harvester, washing 3 times, 1 ml per well in ice-cold assay buffer. After drying, 50 $\mu$l of liquid scintillation cocktail (MicroScint PS, Perkin Elmer, Waltham, MA) was added to each well, plates were sealed, and analyzed on a PerkinElmer Topcount NXT™.

***Allosteric Operational Models.*** Binding-interaction studies with allosteric ligands were fitted to the following allosteric ternary complex model (equation 1):(May, Leach, Sexton, & Christopoulos, 2007)

$$Y = \frac{B_{max}[A]}{[A] + \left(\frac{K_A K_B}{\alpha'[B] + K_B}\right)\left(1 + \frac{[I]}{K_I} + \frac{[B]}{K_B} + \frac{\alpha[I][B]}{K_I K_B}\right)} \qquad (1)$$

where Y is percentage (vehicle control) binding, $B_{max}$ is the total number of receptors, [A], [B] and [I] are the concentrations of radioligand, allosteric modulator and the orthosteric ligand, respectively, $K_A$ and $K_B$ and $K_I$ are the equilibrium dissociation constants of the radioligand, allosteric modulator orthosteric ligand, respectively, $\alpha'$ and $\alpha$ are the binding cooperativities between the allosteric ligand and [$^3$H]-SCH23390 and the allosteric modulator and the agonist dopamine, respectively. Saturation binding experiments were used to determine the value of p$K_A$ for [$^3$H]-SCH23390 (p$K_A$ = 9.30,

$K_A$ = 0.5 nM). Values of $\alpha$ (or $\alpha'$) > 1 denote positive cooperativity; values < 1 (but > 0) denote negative cooperativity, and value = 1 denotes neutral cooperativity. For compound MLS6585, a complete inhibition of [$^3$H]-SCH23390 binding by the allosteric modulator was observed, which is consistent with a very high level of negative cooperativity. In this case to allow fitting of the data, log$\alpha'$ was fixed to -3 to reflect this high negative cooperativity. For compound MLS 1082, no displacement of [$^3$H]-SCH23390 binding was observed, which is consistent with neutral cooperativity (log$\alpha'$ = 0). The dissociation constant of dopamine ($K_I$) was not fixed in these analyses but rather determined for each separate experiment.

Concentration-response curves for the interaction between the allosteric ligand and the orthosteric ligand in the β-arrestin recruitment assays were globally fitted to the following operational model of allosterism and agonism (equation 2):(Leach, Sexton, & Christopoulos, 2007)

$$E = \frac{E_m(\tau_A[A](K_B+\alpha\beta[B])+\tau_B[B]K_A)^n}{([A]K_B+K_AK_B+[B]K_A+\alpha[A][B])^n+(\tau_A[A](K_B+\alpha\beta[B])+\tau_B[B]K_A)^n} \quad (2)$$

where $E_m$ is the maximum possible cellular response, [A] and [B] are the concentrations of orthosteric and allosteric ligands, respectively, $K_A$ and $K_B$ are the equilibrium dissociation constant of the orthosteric and allosteric ligands, respectively, $\tau_A$ and $\tau_B$ are operational measures of orthosteric and allosteric ligand efficacy, respectively, $\alpha$ is the binding cooperativity parameter between the orthosteric and allosteric ligand, $\beta$ denotes the magnitude of the allosteric effect of the modulator on the efficacy of the orthosteric agonist and n denotes the transducer slope that describes the underlying stimulus-response coupling of the ligand-occupied receptor to the signal pathway. This parameter was constrained to be shared between all curves within a fitted dataset for each interaction study. In many instances, the individual model parameters of equation 2 could not be directly estimated via the nonlinear regression algorithm by analysis of the functional data alone due to parameter redundancy. To facilitate model convergence, therefore, we fixed the equilibrium dissociation constant of each ligand to that determined from the whole cell binding assays. For all compounds, no agonism was observed so log$\tau_B$ was fixed to -3.

***Statistical Analysis.*** Data were analyzed using GraphPad Prism 6.01 (GraphPad Software, Inc., La Jolla, CA). All results are expressed as mean ± SEM and are normalized to dopamine control. Statistical significance was determined using paired, two-tailed Student's t-tests when two groups were compared and one-way ANOVA with Bonferroni post-test when multiple groups were compared. $p$ < 0.05 was used as the cutoff for statistical significance.

***Bioluminescence Resonance Energy Transfer (BRET) Assays.*** Experiments were performed in HEK239 cells transiently transfected with D1R-RLuc8 and β-arrestin-mVenus (β-arrestin BRET) or Gαs-mVenus + β1 + γ2 (Gs BRET) using the polyethylenimine (PEI) transfection method. Briefly, 4 × $10_6$ cells/plate were seeded on 10 cm dishes and incubated overnight. Appropriate amounts of DNA were combined with 3 μg/μL PEI per μg of DNA in nonsupplemented DMEM and incubated with the cells overnight. Experiments were performed 48 hours post-transfection. On experiment day, cells were collected and resuspended in Dulbecco's Phosphate-Buffered Saline (DPBS) with $Ca_{2+}$ and $Mg_{2+}$ + 0.2 mM sodium metabisulfite + 5.5 mM glucose. Cells were plated in 96-well white, solid-bottom plates (Greiner Bio-One, Monroe, NC) and incubated at room temperature for 45 min. Coelenterazine H (5 μM, NanoLight Technology, Pinetop, AZ) was added to the cells and incubated for 5 minutes at room temperature protected from light, followed by incubation with the indicated concentration of compounds for 5 minutes in > 3% DMSO. Luminescence and fluorescence signals were measured using a PheraSTAR plate reader (BMG Labtech, Cary, NC). BRET ratio was calculated by dividing the fluorescence signal by the luminescence signal for each well and normalized to a percentage of the control BRET ratio with a maximum concentration of dopamine, with zero percent being the BRET ratio produced in the absence of any compound.

***Internalization Assay.*** Agonist-mediated D1R internalization was assessed using the PathHunter Total GPCR Internalization Assay System (DiscoverX, Inc., Fremont, CA) which utilizes a U2OS cell line stably expressing the D1R tagged with a Prolink tag, and an enzyme acceptor tag fused to an endosomal marker protein. Trafficking of the tagged receptor to the endosomes results in complementation of the two enzyme fragments and a subsequent chemiluminescent signal. The assay was conducted according to the manufacturer's recommendation as described in Conroy et al. (2015).

EXAMPLE 1

This example demonstrates that D1R mediated β-arrestin recruitment is potentiated by compounds **1** and **28.**

β -arrestin recruitment was measured following stimulation by the indicated concentrations of dopamine either alone (DA) or in the presence of 50 μM of either compounds **1** (Fig. 1A) and 28 (Fig. 1B). Potential PAM agonist activity also determined via a concentration response with the PAM as indicated. Compound **1** increases dopamine potency by 7-fold (p = 0.013), and efficacy by 20% (p = 0.029). Compound **28** causes an 8-fold increase in potency (p = 0.007), and a 34% increase in efficacy (p = 0.024). Neither PAM displayed any measurable agonist activity. Data are displayed as

a percentage of the maximum control stimulation seen with dopamine, mean +/- SEM, n=5.

EXAMPLE 2

This example demonstrates that determination of PAM affinity via dopamine curve shift analysis.

Increasing concentrations of compound **1** (Fig. 2A) or compound **28** (Fig. 2B) were used to potentiate dopamine concentration response curves in a β-arrestin recruitment assay. The fold changes in dopamine EC50 and Emax for each PAM concentration were calculated and used to determine the affinity of each PAM.

EXAMPLE 3

This example demonstrates that compound **1** and compound **28** potentiate dopamine-stimulated cAMP accumulation.

Compound **1** (Fig. 3A) increases dopamine potency for cAMP accumulation by 3-fold ($p = 0.024$), but importantly does not significantly increase forskolin's affinity for cAMP accumulation in non D1R containing control cells (Fig. 3B). Compound **28** (Fig. 3C) increases dopamine potency for cAMP accumulation by 5-fold ($p = 0.023$), but also shows no increase in forskolin stimulated cAMP from control cells (Fig. 3D); indicating that both compounds potentiate cAMP accumulation though action on the D1R. Neither compound **1** (Figs. 3A and 3B) nor compound **28** (Figs. 3C and 3D) demonstrated any agonist activity for cAMP accumulation. Data are displayed as a percentage of the maximum control stimulation seen with dopamine (Figs. 3A and 3C) or with forskolin (Figs. 3B and 3D), mean +/- SEM, n=5.

EXAMPLE 4

This example demonstrates that compound **1** and compound **28** increase dopamine's affinity for the D1R.

Dopamine competition binding with [3H]-SCH23390 was used to determine PAM effects on dopamine binding affinity to the D1R orthosteric site. Increasing concentrations of compound **1** (Fig. 4A) shifted the dopamine competition curve, by ~3-fold leftward indicating an increase in dopamine affinity in the presence of MLS 1082. Increasing compound **28** concentrations shifted the dopamine competition curve ~7-fold (Fig. 4B). Decreases in the maximum binding in the DA + MLS6585 curves is due to MLS6585 blocking [3H]-SCH23390 at higher concentrations. Radioligand competition displacement binding demonstrate MLS1082 slightly blocks [3H]-SCH23390 binding at the highest concentration tested by ~17%; whereas, MLS6585 had a greater effect on [3H]-SCH23390 binding, decreasing binding by -66% at 50 μM (Fig. 4C). Data are represented as a percentage of the control specific [3H]-SCH23390 binding seen in the absence of competitor.

EXAMPLE 5

This example demonstrates that compound **1** and compound **28** increase the efficacy of D1R partial agonists.

β-arrestin recruitment assays were performed using dose responses of known agonists of the D1R, both in the presence and absence of the PAM compounds. Compounds **1** and **28** both increased the efficacy of the partial agonists fenoldopam Fig. 5(A) (1082: 48% increase, $p < 0.05$; 6585: 83% increase, $p < 0.0001$) and apomorphine (Fig. 5B), 1082: 95% increase, $p < 0.01$; 6585: 134% increase, $p < 0.0001$). Furthermore, the G-protein biased partial agonist SKF38393 (Fig. 5C) was potentiated to weak partial agonism by concurrent treatment with the PAM compounds (**1**: 111% increase, $p < 0.05$; 6585: 151% increase, $p < 0.001$). Both compounds **1** and **28** also potentiated SKF77434-stimulated cAMP accumulation (Fig. 5D, 1082: 130% increase, $p < 0.0001$; 6585: 96% increase, $p < 0.01$). Neither PAM altered the potency of any D1R partial agonist tested. Data are displayed as a percentage of the maximum control stimulation seen with dopamine.

EXAMPLE 6

This example demonstrates that compounds **1** and **28** show additive potentiation, suggesting divergent binding sites.

Potentiation of β-arrestin recruitment (A) and cAMP accumulation (B) were determined using MLS1082 and MLS6585 separately and in combination. Fig. 6A shows concentration-response curve of DA plus either 50 μM compound **1** or compound **28** potentiated DA potency for β-arrestin recruitment by 5- and 8-fold, respectively (PAM fold change EC50 vs. DA control, compound **1:** $p = 0.032$; compound **28;** $p = 0.021$, paired two-tailed Student's t-test, n = 5). The two PAMs in combination (50 μM compound **1** + 50 μM compound **28**) potentiated DA potency for recruiting β -arrestin 74-fold vs. DA control and an additional 20- and 11-fold vs. compound **1** or compound **28,** respectively, with no effect on maximum efficacy (combo fold change EC50 vs. DA control, $p = 0.045$; vs. compound **1**, $p = 0.052$; vs. compound

**28,** p = 0.06; paired two-tailed Student's t-test, n = 5). Fig. 6B shows that 50 μM compound **1** or 50 μM compound **28** plus increasing concentrations of DA potentiated DA potency for stimulating cAMP accumulation by 3- and 4-fold, respectively (PAM fold change EC50 vs. DA control, compound **1:** p = 0.13; compound **28**; p = 0.018, paired two-tailed Student's t-test, n = 3). In combination (50 μM compound **1** + 50 μM compound **28**), the PAMs potentiated DA potency for stimulation cAMP 15-fold vs. DA control and an additional 6- and 4-fold vs. compound **1** or compound **28**, respectively, with no effect on maximum efficacy (combo fold change EC50 vs. DA control, p = 0.031; vs. compound **1,** p = 0.021; vs. compound **28**, p = 0.042; paired two-tailed Student's t-test, n = 3). Taken together, these results suggest the two PAMs act at distinct binding locations.

EXAMPLE 7

This example demonstrates the improved selectivity for the dopamine D1 receptor versus the dopamine D5 receptor exhibited by compounds of formula (III), in accordance with an embodiment of the invention.

(a) D1R Competition Binding

Competition binding of [$^3$H]-SCH23390 at the D1 dopamine receptor were conducted as follows. Membranes from HEK cells stably expressing the D1R were incubated with, a D1R orthosteric antagonist, and increasing concentrations of either unlabeled SCH23390 (positive control) or the test compound. The results are shown graphically for compounds **5, 6,** and **7** in FIGS. 7A, 7B, and 7C, respectively.

As is apparent from the results shown in FIGS. 7A-7C, compounds **5, 6,** and **7** had no effect on [$^3$H]-SCH23390 binding at concentrations as high as 50 μM.

(b) D1R β-Arrestin Recruitment

The DiscoverX PathHunter β-Arrestin Assay was used to measure β-arrestin reuitment to the D1R following the manufacturer's instructions. Briefly, CHO cells stably expressing the D1R fused to a ProLink tag and β-arrestin fused to a proprietary enzyme acceptor were incubated with a dopamine concentration-response curve ± 50 μM compound **1** or compounds **5, 6,** and **7**. β-arrestin recruitment was measured in raw luminescent units and was normalized to dopamine control. The results are shown graphically for **5, 6,** and **7** in Figs. 8A-8C, respectively. Compound **1** increased dopamine $EC_{50}$ 4.6-fold and $E_{max}$ by 20%. Compound **5** increased dopamine $EC_{50}$ by 3-fold and $E_{max}$ by 2%. Compound **6** increased dopamine $EC_{50}$ by 3-fold but did not increase $E_{max}$. Compound **7** increased dopamine $EC_{50}$ by 2-fold but did not increase $E_{max}$.

(c) Recruitment of β-Arrestin to the D5R

Dopamine-stimulated β-arrestin recruitment to the D5 dopamine receptor (D5R) was measured similarly as described for D1R, using CHO cells stably expressing the D5R fused to a ProLink tag and β-arrestin fused to a proprietary enzyme acceptor. The results are shown for compounds **5, 6,** and **7** in Figs. 9A-9C, respectively. Compound **1** increased dopamine $EC_{50}$ by 2.5-fold and $E_{max}$ by 22%. Compound **5** had very minimal effect on potentiating the dopamine-stimulated recruitment of β-arrestin to the D5R, potentiating dopamine $EC_{50}$ by 1.3-fold, but decreasing $E_{max}$ by 12%. Compound **6** had a slight deleterious effect on potentiating the dopamine-stimulated recruitment of β-arrestin to the D5R, decreasing dopamine $EC_{50}$ by 0.8-fold and decreasing $E_{max}$ by 37%. Compound **7** had a slight deleterious effect on potentiating the dopamine-stimulated recruitment of β-arrestin to the D5R, decreasing dopamine $EC_{50}$ by 0.8-fold and decreasing $E_{max}$ by 29%.

These result show that compounds **5, 6,** and **7** potentiate the dopamine stimulation of beta-arrestin recruitment to the D1 receptor, though not to a greater extent than compound **1**. However, none of these analogs have PAM activity at the D5 receptor, which is an improvement over compound **1**.

EXAMPLE 8

This example shows the activity of various representative compounds of the invention in the D1 β-arrestin assay, the D1 cAMP assay, the D5 β-arrestin assay, the D4 β-arrestin assay, the D5 cAMP assay, and the SCH23390 assay. The results are set forth in Tables 1-3.

Table 1: D1 β-arrestin and D1 cAMP assays

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 1 | 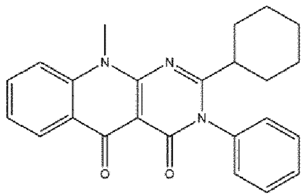 | 2.50E-07 | 4.64 | 120% | 7 | 7.20 E-08 | 1.83 | 111% | 3 |
| 2 | 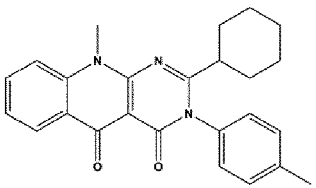 | 5.00E-07 | 2.32 | 89% | 4 | 1.10 E-07 | 1.20 | 115% | 2 |
| 3 | 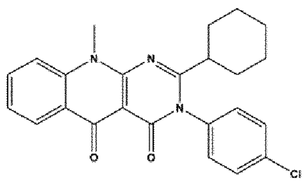 | 8.00E-07 | 1.45 | 110% | 4 | 1.60 E-07 | 0.83 | 105% | 2 |
| 4 | 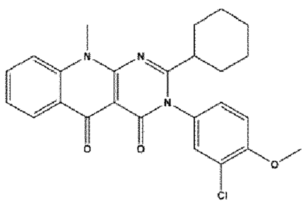 | 7.50E-07 | 1.55 | 108% | 4 | 1.50 E-07 | 0.88 | 87% | 2 |

(continued)

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 5 | 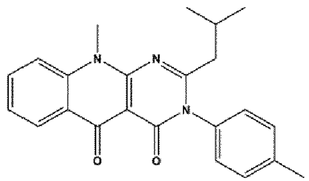 | 3.50E-07 | 3.31 | 102% | 4 | 1.85 E-07 | 1.19 | 98% | 3 |
| 6 | 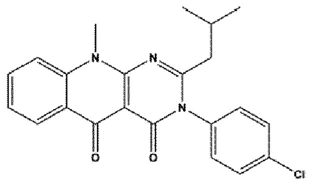 | 3.90E-07 | 2.97 | 87% | 4 | 7.70 E-08 | 1.71 | 115% | 2 |
| 7 | 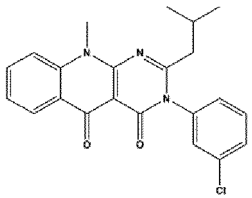 | 6.10E-07 | 1.90 | 105% | 4 | 1.9 0E-07 | 0.69 | 105% | 2 |
| 8 | 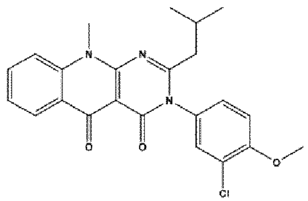 | 5.20E-07 | 2.23 | 96% | 4 | 8.40 E-08 | 1.57 | 119% | 2 |

(continued)

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 9 | 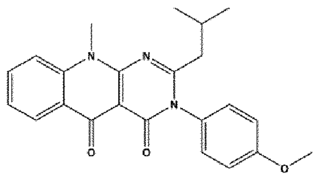 | 1.39E-07 | 7.37 | 91% | 5 | 3.70 E-08 | 3.57 | 151% | 2 |
| 10 | 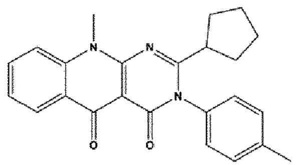 | 1.4E-07 | 8.29 | 115% | 4 | 9.00 E-08 | 1.47 | 116% | 2 |
| 11 | 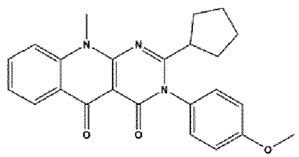 | 1.30E-07 | 8.92 | 116% | 4 | 9.60 E-08 | 1.38 | 111% | 2 |
| 12 | 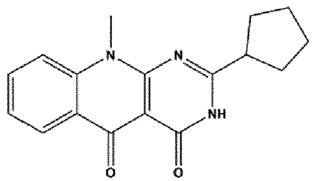 | 6.30E-07 | 1.84 | 83% | 4 | 6.00 E-08 | 2.20 | 102% | 2 |
| 13 | 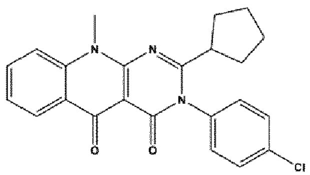 | 3.40E-07 | 3.41 | 108% | 4 | 7.40 E-08 | 1.78 | 120% | 2 |

(continued)

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 14 | 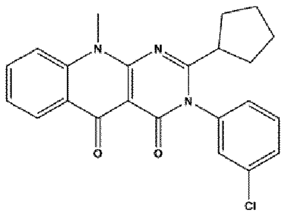 | 1.80E-07 | 6.44 | 78% | 2 | 1.20 E-07 | 1.10 | 117% | 2 |
| 15 | 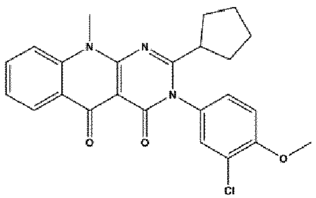 | 6.40E-07 | 1.81 | 88% | 2 | 4.30 E-08 | 3.07 | 143% | 2 |
| 16 | 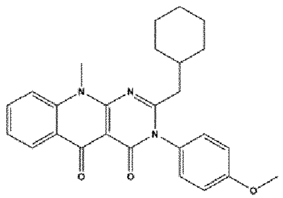 | 2.60E-06 | 0.45 | 114% | 2 | 3.9 E-07 | 0.34 | 66% | 2 |
| 17 | 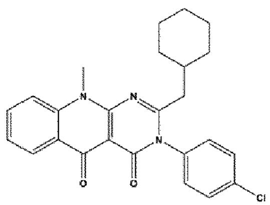 | 1.50E-06 | 0.77 | 107% | 2 | 1.8 E-07 | 0.73 | 100% | 2 |

(continued)

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 18 | 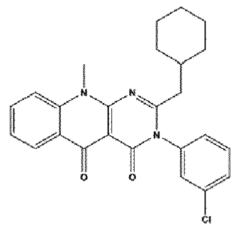 | 1.10E-06 | 1.05 | 92% | 2 | 1.8 E-07 | 0.73 | 104% | 2 |
| 19 | 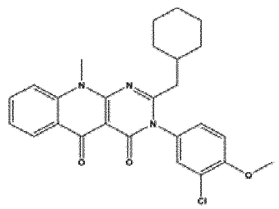 | 2.20E-06 | 0.53 | 107% | 2 | 1.9 E-07 | 0.69 | 107% | 2 |
| 20 | 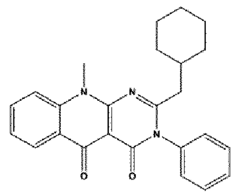 | 1.10E-06 | 1.05 | 104% | 2 | 1.1 E-07 | 1.18 | 91% | 2 |
| 21 | 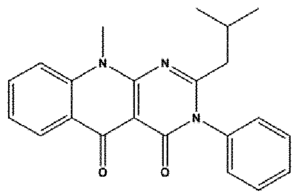 | 2.00E-07 | 5.80 | 105% | 2 | 5.4 E-08 | 3.41 | 89% | 3 |

(continued)

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 22 | 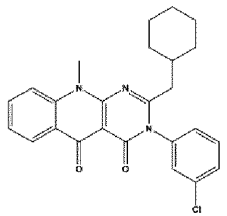 | 6.40E-07 | 1.81 | 99% | 2 | 2.0 E-07 | 0.83 | 117% | 2 |
| 23 | 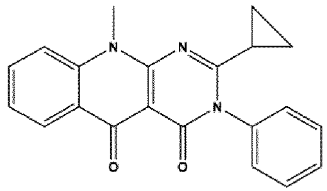 | 6.30E-07 | 1.84 | 106% | 2 | 1.1 E-07 | 1.50 | 106% | 2 |
| 24 | 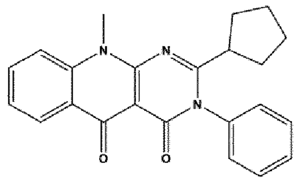 | 2.70E-07 | 4.30 | 101% | 2 | 1.86 E-08 | 6.10 | 107% | 2 |
| 25 | 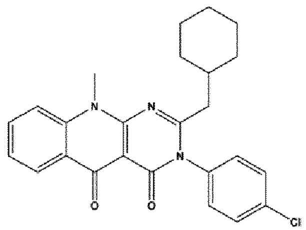 | 1.20E-06 | 0.97 | 74% | 2 | 2.50 E-07 | 0.56 | 130% | 1 |

(continued)

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 26 | 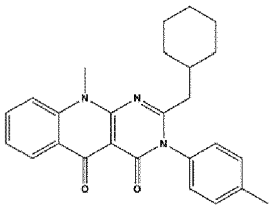 | 1.10E-06 | 1.05 | 102% | 2 | 1.09 E-07 | 1.04 | 113% | 2 |
| 27 | 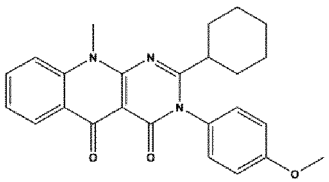 | 9.00E-07 | 1.29 | 82% | 2 | 6.00 E-08 | 2.33 | 117% | 1 |
| 28 | 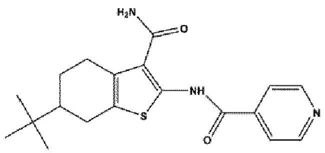 | 1.40E-07 | 8.29 | 134% | 12 | 4.50E-08 | 2.93 | 102% | 2 |
| 29 | 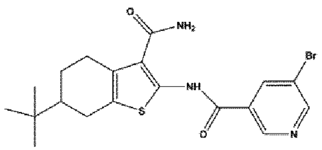 | 1.10E-06 | 1.05 | 121 % | 6 | 1.10E-07 | 1.30 | 111% | 2 |
| 30 | 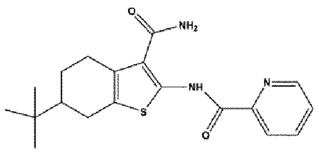 | 1.10E-06 | 1.05 | 151% | 6 | 6.73E-08 | 1.31 | 109% | 2 |

(continued)

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 31 | 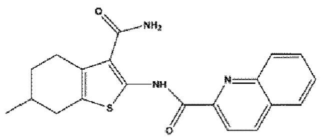 | 8.60E-07 | 1.35 | 126% | 6 | 7.37E-08 | 1.20 | 118% | 2 |
| 32 | 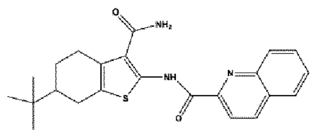 | 1.50E-06 | 0.77 | 128% | 6 | 6.89E-08 | 1.28 | 120% | 2 |
| 33 | 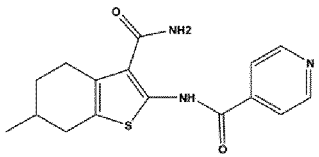 | 1.20E-06 | 0.97 | 124% | 5 | 2.32E-07 | 1.53 | 117% | 2 |
| 34 | 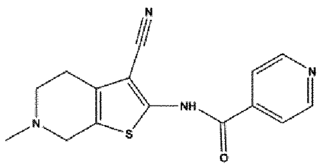 | 1.90E-06 | 0.61 | 121 % | 5 | 2.74E-07 | 1.30 | 102% | 2 |
| 35 | 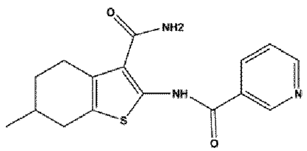 | 1.20E-06 | 0.97 | 136% | 5 | 1.98E-07 | 1.79 | 112% | 1 |

(continued)

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 36 | 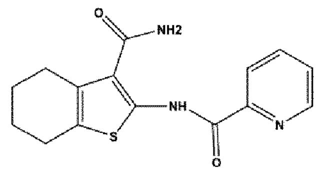 | 1.40E-06 | 0.83 | 136% | 5 | 1.41E-07 | 2.50 | 105% | 1 |
| 37 | 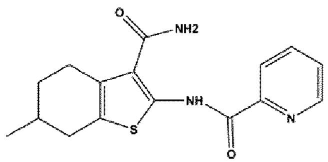 | 1.10E-06 | 1.05 | 114% | 5 | 1.06E-07 | 1.61 | 101% | 2 |
| 38 | 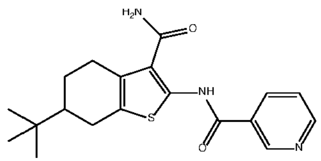 | 3.84E-07 | 10.35 | 130% | 6 | 6.29E-08 | 2.29 | 103% | 3 |
| 39 | 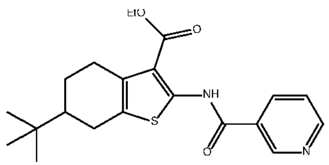 | 1.32E-06 | 3.01 | 108% | 6 | 1.39E-07 | 1.04 | 100% | 3 |
| 40 | 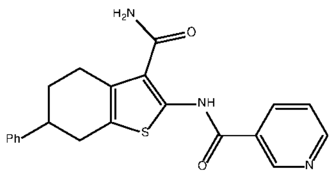 | 2.01E-06 | 1.97 | 123% | 6 | 2.05E-07 | 0.7 | 109% | 3 |

(continued)

| | | D1 Beta-Arrestin | | | | D1 cAMP | | | |
|---|---|---|---|---|---|---|---|---|---|
| Cmpd | Structure | DA + 50 μM PAM, EC50 (M) | DA + 50 μM PAM, Fold Chg EC50 | DA + 50 μM PAM, Emax (%DA) | N | DA + 50 μM PAM, EC50 (M) | DA + 50 μM PAM, Fold Chg EC50 | DA + 50 μM PAM, Emax (%DA) | N |
| 41 | 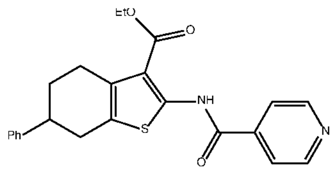 | | N.D | | | | N.D. | | |

Table 2: D5 β-arrestin and D4 β-arrestin assays

| | | D5 Beta-Arrestin | | | | D4 Beta-Arrestin | | | |
|---|---|---|---|---|---|---|---|---|---|
| Cmpd | Structure | DA+50 μM PAM, EC50 (M) | DA + 50 μM PAM, Fold Change EC50 | DA + 50 μM PAM, Emax (%DA) | N | DA+50 μM PAM, EC50 (M) | DA + 50 μM PAM, Fold Change EC50 | DA + 50 μM PAM, Emax (%DA) | N |
| 1 | 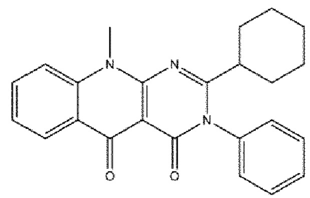 | 7.00E-07 | 2.56 | 122% | 5 | 2.60E-07 | 1.06 | 115% | 5 |
| 2 | 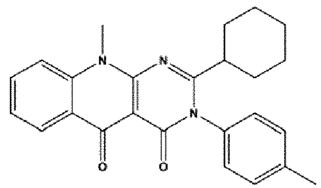 | 2.20E-08 | 2.64 | 82% | 2 | 1.70E-07 | 0.64 | 89% | 2 |
| 3 | 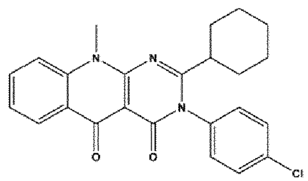 | 2.80E-08 | 2.03 | 94% | 2 | 1.80E-07 | 0.61 | 109% | 2 |
| 4 | 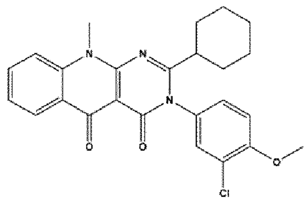 | 7.50E-08 | 0.77 | 107% | 2 | 1.80E-07 | 0.61 | 104% | 2 |

(continued)

| | | D5 Beta-Arrestin | | | | D4 Beta-Arrestin | | | |
|---|---|---|---|---|---|---|---|---|---|
| Cmpd | Structure | DA+50 μM PAM, EC50 (M) | DA + 50 μM PAM, Fold Change EC50 | DA + 50 μM PAM, Emax (%DA) | N | DA+50 μM PAM, EC50 (M) | DA + 50 μM PAM, Fold Change EC50 | DA + 50 μM PAM, Emax (%DA) | N |
| 5 | 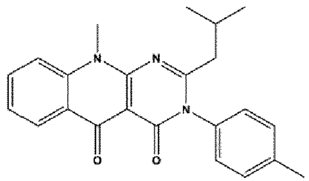 | 3.30E-08 | 1.32 | 88% | 2 | 1.10E-07 | 1.06 | 83% | 3 |
| 6 | 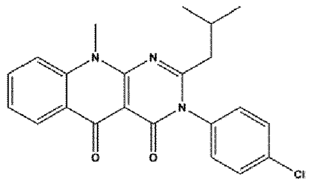 | 7.10E-08 | 0.81 | 63% | 2 | 2.30E-07 | 0.47 | 81% | 2 |
| 7 | 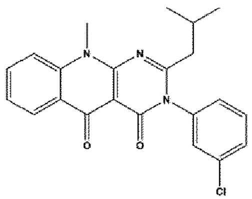 | 7.20E-08 | 0.80 | 71% | 2 | 1.80E-07 | 0.57 | 69% | 2 |
| 8 | 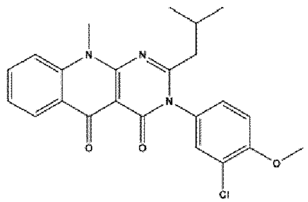 | 3.70E-08 | 1.53 | 148% | 2 | 1.30E-07 | 1.20 | 118% | 2 |

(continued)

| | | D5 Beta-Arrestin | | | | D4 Beta-Arrestin | | | |
|---|---|---|---|---|---|---|---|---|---|
| Cmpd | Structure | DA+50 μM PAM, EC50 (M) | DA + 50 μM PAM, Fold Change EC50 | DA + 50 μM PAM, Emax (%DA) | N | DA+50 μM PAM, EC50 (M) | DA + 50 μM PAM, Fold Change EC50 | DA + 50 μM PAM, Emax (%DA) | N |
| 9 | 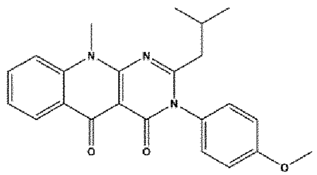 | 1.90E-08 | 2.93 | 182% | 2 | 9.80E-08 | 1.58 | 128% | 2 |
| 10 | 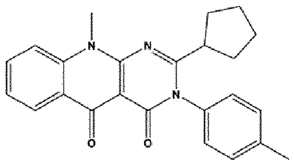 | 1.80E-08 | 3.11 | 212% | 2 | 1.20E-07 | 1.33 | 164% | 2 |
| 11 | 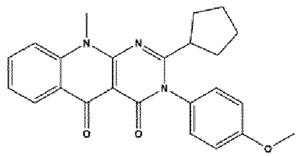 | 1.40E-08 | 4.10 | 207% | 2 | 1.10E-07 | 1.46 | 169% | 2 |
| 12 | 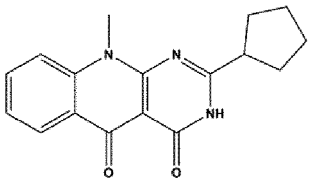 | 8.50E-08 | 0.66 | 121% | 2 | 2.20E-07 | 0.71 | 102% | 2 |
| 13 | 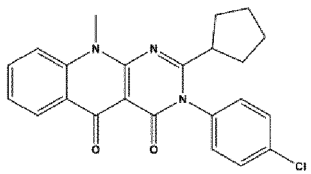 | 3.20E-08 | 1.77 | 172% | 2 | 1.00E-07 | 1.44 | 153% | 2 |

(continued)

| | | D5 Beta-Arrestin | | | | D4 Beta-Arrestin | | | |
|---|---|---|---|---|---|---|---|---|---|
| Cmpd | Structure | DA+50 μM PAM, EC50 (M) | DA + 50 μM PAM, Fold Change EC50 | DA + 50 μM PAM, Emax (%DA) | N | DA+50 μM PAM, EC50 (M) | DA + 50 μM PAM, Fold Change EC50 | DA + 50 μM PAM, Emax (%DA) | N |
| 14 | 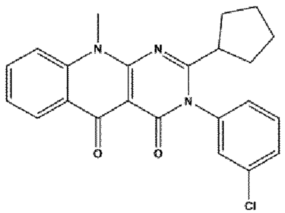 | 3.72E-08 | 0.91 | 101% | 2 | 9.87E-08 | 1.90 | 81% | 3 |
| 15 | 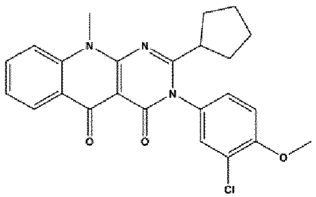 | 2.20E-08 | 1.51 | 105% | 2 | 1.64E-07 | 1.33 | 90% | 2 |
| 16 | 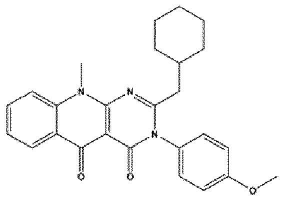 | 4.59E-08 | 0.73 | 154% | 2 | 1.50E-07 | 1.45 | 100% | 2 |
| 17 | 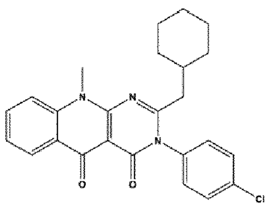 | 3.09E-08 | 1.09 | 81% | 2 | 1.60E-07 | 1.38 | 79% | 2 |

(continued)

| | | D5 Beta-Arrestin | | | | D4 Beta-Arrestin | | | |
|---|---|---|---|---|---|---|---|---|---|
| Cmpd | Structure | DA+50 μM PAM, EC50 (M) | DA + 50 μM PAM, Fold Change EC50 | DA + 50 μM PAM, Emax (%DA) | N | DA+50 μM PAM, EC50 (M) | DA + 50 μM PAM, Fold Change EC50 | DA + 50 μM PAM, Emax (%DA) | N |
| 18 | 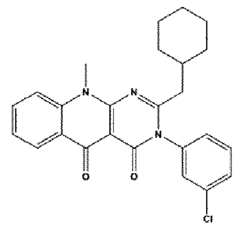 | 1.30E-07 | 1.35 | 93% | 2 | 2.81E-07 | 1.57 | 102% | 2 |
| 19 | 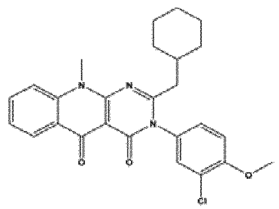 | 1.37E-07 | 1.29 | 94% | 2 | 2.47E-07 | 1.78 | 95% | 2 |
| 20 | 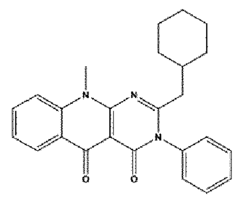 | 4.05E-08 | 2.38 | 126% | 2 | 2.06E-07 | 2.14 | 93% | 2 |
| 21 | 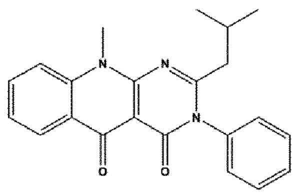 | 4.35E-08 | 4.56 | 120% | 2 | 1.40E-07 | 0.62 | 133% | 3 |

(continued)

| | | D5 Beta-Arrestin | | | | D4 Beta-Arrestin | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 22 | 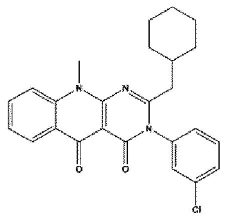 | 6.89E-08 | 1.40 | 128% | 2 | 2.65E-07 | 1.67 | 96% | 2 |
| 23 | 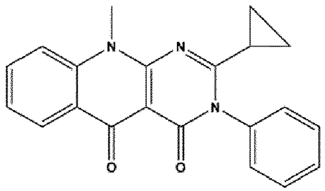 | 6.58E-08 | 2.58 | 122% | 2 | 1.23E-07 | 1.32 | 118% | 2 |
| 24 | 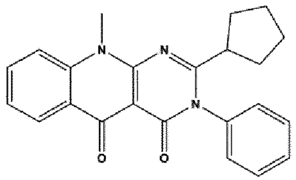 | 4.98E-08 | 4.16 | 138% | 2 | 6.92E-08 | 1.26 | 112% | 2 |
| 25 | 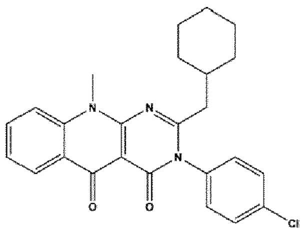 | 7.58E-08 | 2.25 | 83% | 2 | 4.77E-07 | 0.77 | 87% | 1 |

(continued)

| | | D5 Beta-Arrestin | | | | D4 Beta-Arrestin | | | |
|---|---|---|---|---|---|---|---|---|---|
| Cmpd | Structure | DA+50 μM PAM, EC50 (M) | DA + 50 μM PAM, Fold Change EC50 | DA + 50 μM PAM, Emax (%DA) | N | DA+50 μM PAM, EC50 (M) | DA + 50 μM PAM, Fold Change EC50 | DA + 50 μM PAM, Emax (%DA) | N |
| 26 | 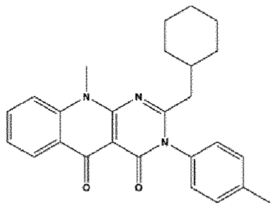 | 2.78E-07 | 1.15 | 112% | 1 | 1.15E-07 | 1.40 | 100% | 2 |
| 27 | 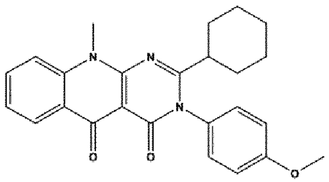 | 9.70E-08 | 3.35 | 154% | 1 | 3.08E-07 | 1.19 | 81% | 1 |
| 28 | 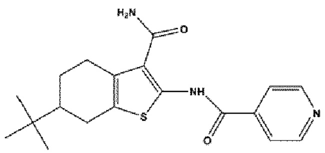 | 1.14E-07 | 1.25 | 139% | 9 | 1.24E-07 | 1.72 | 160% | 6 |
| 29 | 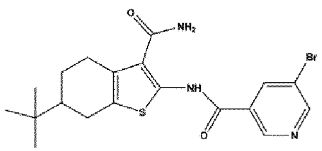 | 1.10E-07 | 1.68 | 99% | 3 | 1.20E-07 | 1.20 | 100% | 2 |
| 30 | 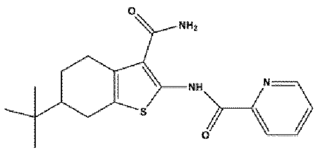 | 3.90E-07 | 0.48 | 154% | 3 | 3.20E-07 | 0.43 | 149% | 2 |

(continued)

| | | D5 Beta-Arrestin | | | | D4 Beta-Arrestin | | | |
|---|---|---|---|---|---|---|---|---|---|
| Cmpd | Structure | DA+50 μM PAM, EC50 (M) | DA + 50 μM PAM, Fold Change EC50 | DA + 50 μM PAM, Emax (%DA) | N | DA+50 μM PAM, EC50 (M) | DA + 50 μM PAM, Fold Change EC50 | DA + 50 μM PAM, Emax (%DA) | N |
| 31 | 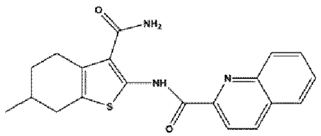 | 2.20E-07 | 0.83 | 106% | 3 | 1.00E-07 | 1.34 | 108% | 2 |
| 32 | 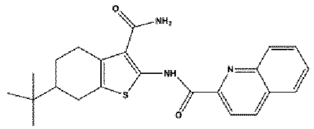 | 2.00E-07 | 0.92 | 87% | 3 | 1.90E-07 | 0.73 | 112% | 2 |
| 33 | 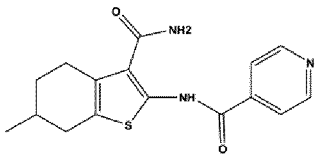 | 9.90E-08 | 1.86 | 101% | 3 | 1.70E-07 | 0.81 | 109% | 2 |
| 34 | 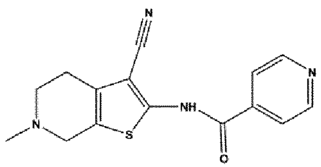 | 1.40E-07 | 1.28 | 95% | 3 | 1.50E-07 | 0.96 | 107% | 2 |
| 35 | 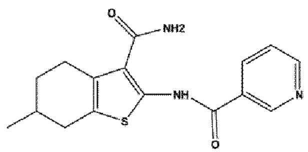 | 1.70E-07 | 1.06 | 94% | 3 | 1.30E-07 | 1.07 | 128% | 2 |

(continued)

| | | D5 Beta-Arrestin | | | | D4 Beta-Arrestin | | | |
|---|---|---|---|---|---|---|---|---|---|
| Cmpd | Structure | DA+50 μM PAM, EC50 (M) | DA + 50 μM PAM, Fold Change EC50 | DA + 50 μM PAM, Emax (%DA) | N | DA+50 μM PAM, EC50 (M) | DA + 50 μM PAM, Fold Change EC50 | DA + 50 μM PAM, Emax (%DA) | N |
| 36 | 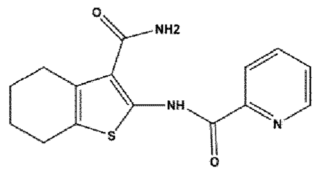 | 7.00E-07 | 0.26 | 97% | 3 | 1.20E-07 | 1.19 | 112% | 2 |
| 37 | 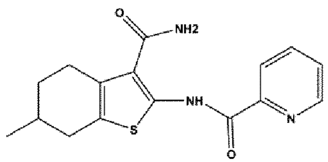 | 3.90E-07 | 0.47 | 87% | 3 | 1.30E-07 | 1.06 | 111% | 2 |
| 38 | 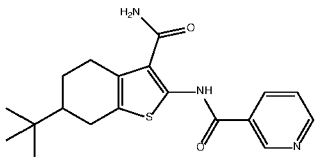 | 1.68E-07 | 0.78 | 177% | 3 | 5.75E-08 | 2.26 | 153% | 2 |
| 39 | 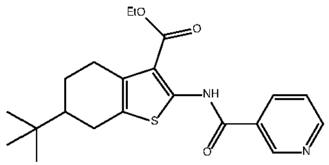 | 1.57E-07 | 0.65 | 107% | 3 | 8.11E-08 | 2.03 | 125% | 2 |
| 40 | 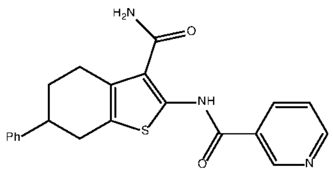 | 2.07E-07 | 0.48 | 140% | 3 | 2.32E-07 | 0.68 | 163% | 2 |

(continued)

| | | D5 Beta-Arrestin | | | | D4 Beta-Arrestin | | | |
|---|---|---|---|---|---|---|---|---|---|
| Cmpd | Structure | DA+50 μM PAM, EC50 (M) | DA + 50 μM PAM, Fold Change EC50 | DA + 50 μM PAM, Emax (%DA) | N | DA+50 μM PAM, EC50 (M) | DA + 50 μM PAM, Fold Change EC50 | DA + 50 μM PAM, Emax (%DA) | N |
| 41 | 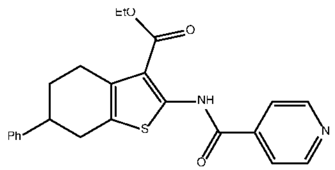 | | N.D. | | | | N.D. | | |

Table 3: D5 cAMP and SCH23390 competition binding assays

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 1 | 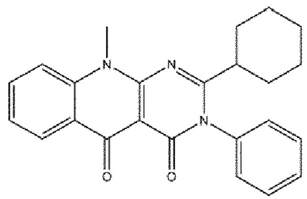 | 3.11E-08 | 2.14 | 112 | 6 | ~0.9 | 17 | 5 | 3.11E-08 |
| 2 | 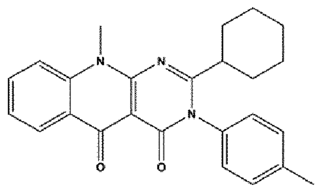 | | | | | -- | -- | 1 | |
| 3 | 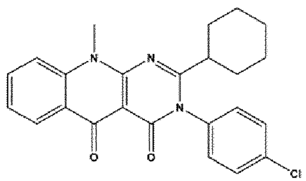 | | | | | -- | -- | 1 | |
| 4 | 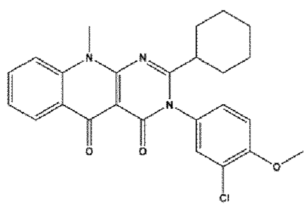 | | | | | 1.20E-04 | 10% | 1 | |

(continued)

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 5 | 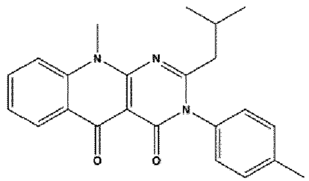 | 3.84E-08 | 2.23 | 103 | 3 | 9.795 | 24% | 2 | 3.84E-08 |
| 6 | 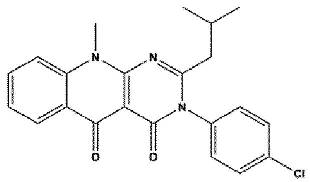 | | | | | ~0.99 | 20% | 2 | |
| 7 | 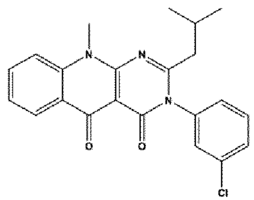 | | | | | ~0.91 | 20% | 2 | |
| 8 | 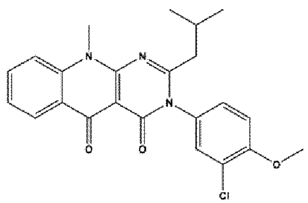 | | | | | -- | -- | 2 | |

(continued)

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 9 | 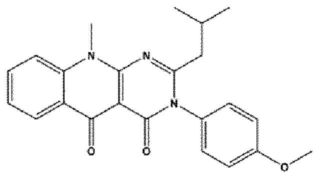 | | | | | ~0.04 | 47% | 2 | |
| 10 | 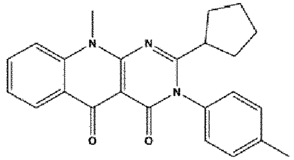 | | | | | 5.23E-05 | 28% | 2 | |
| 11 | 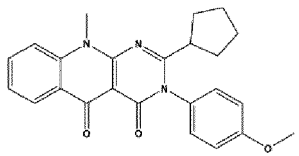 | | | | | 3.45E-05 | 21% | 2 | |
| 12 | 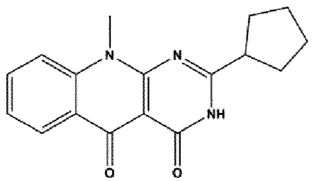 | | | | | ~6.91 | 30% | 2 | |
| 13 | 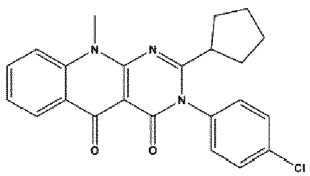 | | | | | 1.77E-06 | 20% | 1 | |

(continued)

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 14 | 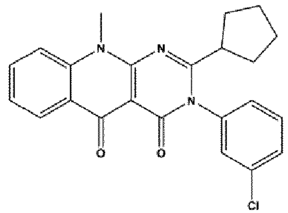 | | | | | -- | -- | 2 | |
| 15 | 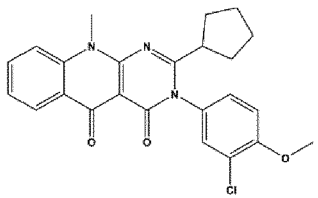 | | | | | -- | -- | 1 | |
| 16 | 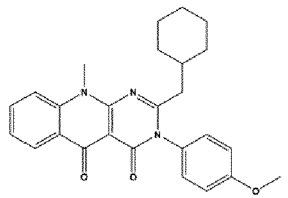 | 1.09E-07 | 0.67 | 38% | 2 | -- | -- | 1 | 1.09E-07 |
| 17 | 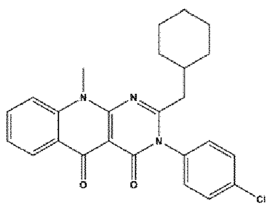 | 5.06E-08 | 1.43 | 104% | 2 | 3.80E-05 | 50% | 1 | 5.06E-08 |

(continued)

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 18 | 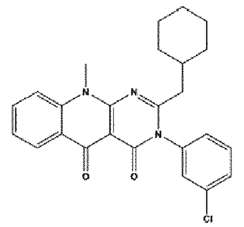 | 1.97E-07 | 0.34 | 103% | 2 | ~0.01 | 30% | 1 | 1.97E-07 |
| 19 | 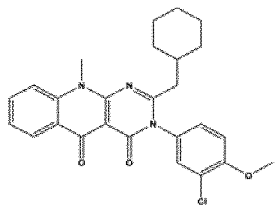 | 6.55E-08 | 1.04 | 108% | 2 | ~0.045 | 24% | 1 | 6.55E-08 |
| 20 | 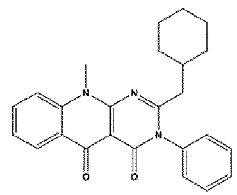 | 3.79E-08 | 1.30 | 121% | 2 | 6.50E-05 | 13% | 1 | 3.79E-08 |
| 21 | 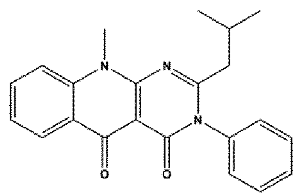 | 2.83E-08 | 2.22 | 105% | 4 | 4.42E-05 | 56% | 2 | 2.83E-08 |

(continued)

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 22 | 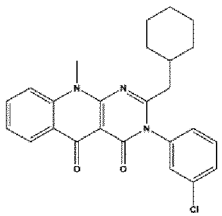 | 6.77E-08 | 0.73 | 79% | 2 | -- | -- | 1 | 6.77E-08 |
| 23 | 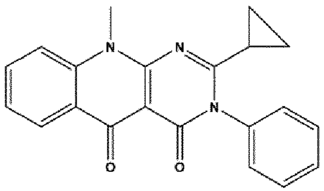 | 8.60E-08 | 0.57 | 108% | 2 | -- | -- | 1 | 8.60E-08 |
| 24 | 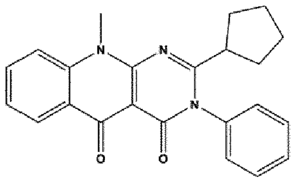 | 6.25E-08 | 1.26 | 108 | 1 | 1.70E-05 | 60% | 1 | 6.25E-08 |
| 25 | 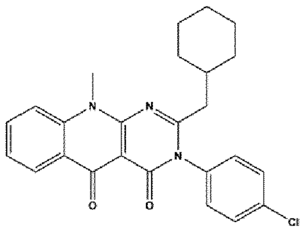 | | | | | -- | -- | 1 | |

(continued)

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 26 | 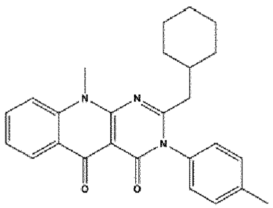 | | | | | -- | -- | 1 | |
| 27 | 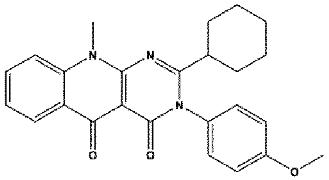 | | | | | -- | -- | 1 | |
| 28 | 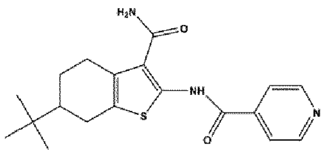 | 5.04E-08 | 1.63 | 99% | 9 | 1.25E-05 | 66% | 5 | 5.04E-08 |
| 29 | 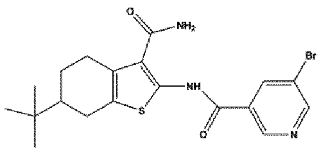 | 9.95E-08 | 0.66 | 134% | 1 | ~0.02 | 40% | 2 | 9.95E-08 |
| 30 | 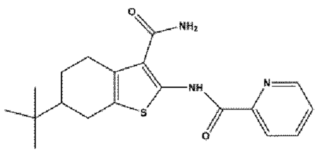 | 4.22E-08 | 0.60 | 123% | 1 | -- | -- | 2 | 4.22E-08 |

(continued)

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 31 | 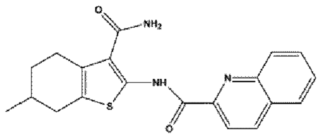 | 5.55E-08 | 0.45 | 124% | 1 | -- | -- | 2 | 5.55E-08 |
| 32 | 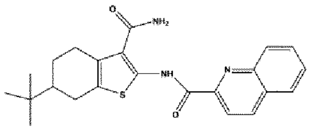 | 4.75E-08 | 1.25 | 130% | 1 | -- | -- | 2 | 4.75E-08 |
| 33 | 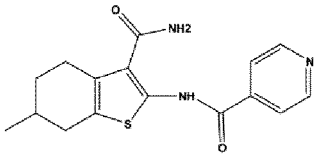 | 2.40E-08 | 1.57 | 97% | 2 | -- | -- | 2 | 2.40E-08 |
| 34 | 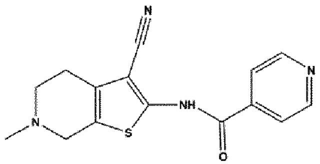 | 2.10E-08 | 1.45 | 97% | 2 | -- | -- | 2 | 2.10E-08 |
| 35 | 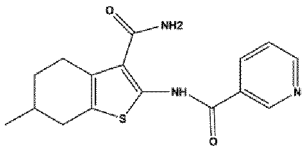 | 1.06E-07 | 0.76 | 82% | 4 | -- | -- | 2 | 1.06E-07 |

(continued)

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 36 | 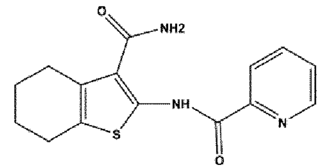 | 6.35E-08 | 0.86 | 72% | 4 | -- | -- | 2 | 6.35E-08 |
| 37 | 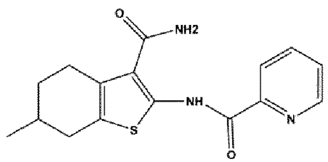 | 2.30E-08 | 1.64 | 116% | 2 | -- | -- | 2 | 2.30E-08 |
| 38 | 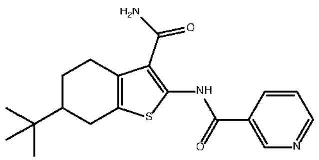 | | | | | 8.67E-06 | 50% | 1 | |
| 39 | 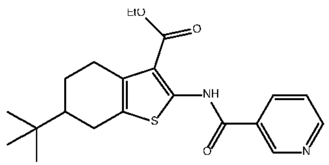 | | | | | 0.21 | -- | 1 | |
| 40 | 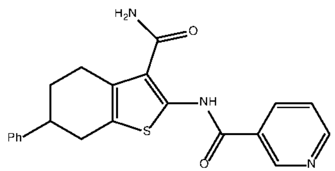 | | | | | -- | -- | 1 | |

(continued)

| | | D5 cAMP | | | | SCH23390 Competition Binding | | | |
|---|---|---|---|---|---|---|---|---|---|
| Cmpd | Structure | DA + 50 μM PAM, EC50 (M) | DA + 50 μM PAM, Fold Change EC50 | DA + 50 μM PAM, Emax (%DA) | N | DA + 50 μM PAM, EC50 (M) | DA + 50 μM PAM, Fold Change EC50 | DA + 50 μM PAM, Emax (%DA) | N |
| 41 | 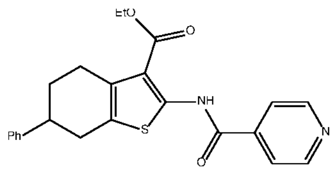 | | N.D. | | | | N.D. | | |

EXAMPLE 9

This example demonstrates methods of preparing compounds in accordance with an embodiment of the invention.

**Example 1: general procedure for thiophene synthesis via the Gewald reaction.** To a slurry of sulfur (1.0 equiv) in ethanol (3 mL) in a 10 mL microwave vial were added the ketone component (1.0 equiv), α-cyanocarbonyl component (1.50 mmol) and morpholine (1.50 equiv). The reaction was heated at 120° C for 15 minutes under microwave irradiation then cooled to rt. The solvent was removed in vacuo and the resulting residue purified by medium pressure silica gel chromatography to afford the thiophene product.

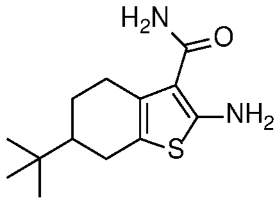

**Example 1.1: 2-Amino-6-(tert-butyl)-4,5,6,7-tetrahydrobenzo[b]thiophene-3-carboxamide.** 4-*t*-Butylcyclohexanone (500 mg, 3.24 mmol) and 2-cyanoacetamide (409 mg, 4.86 mmol) were reacted according to general procedure A to afford the thiophene product as a light yellow solid (227 mg, 0.90 mmol, 28% yield). $R_f$ = 0.48 (10% MeOH in $CH_2Cl_2$); mp = 223-226 °C; $^1$H NMR (401 MHz, DMSO-$d_6$) δ 0.87 (s, 9H), 1.16 (dq, *J* = 5.2, 12.1 Hz, 1H), 1.34-1.41 (m, 1H), 1.90 (dd, *J* = 2.5, 12.4 Hz, 1H), 2.22 (t, *J* = 14.8 Hz, 1H), 2.41-2.58 (m, 2H), 2.66 (dd, *J* = 4.9, 16.0 Hz, 1H); $^{13}$C NMR (101 MHz, DMSO-$d_6$) δ 24.6, 26.1, 27.4, 27.6, 32.6, 45.0, 107.5, 116.5, 130.4, 159.8, 168.3; IR 3304, 2952, 1628, 1556 $cm^{-1}$.

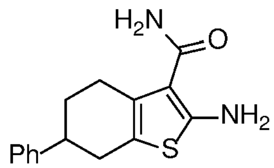

**Example 1.2: 2-Amino-6-phenyl-4,5,6,7-tetrahydrobenzo[b]thiophene-3-carboxamide.** 4-Phenylcyclohexanone (351 mg, 2.02 mmol) and 2-cyanoacetamide (263 mg, 3.12 mmol, 1.55 equiv) were reacted according to general procedure A to afford the previously described thiophene product (Org. Biomol. Chem. 2014, 12, 1942-1956) as a light yellow solid (315 mg, 1.16 mmol, 57% yield). $R_f$ = 0.58 (10% MeOH in $CH_2Cl_2$); mp = 229-233 °C; $^1$H NMR (401 MHz, DMSO-$d_6$) δ 1.76-1.87 (m, 1H), 1.91-1.98 (m, 1H), 2.47-2.80 (complex, 4H), 2.86-2.95 (m, 1H), 6.54 (br s, 2H), 6.95 (br s, 2H), 7.16-7.22 (m, 1H), 7.27-7.31 (complex, 4H); $^{13}$C NMR (101 MHz, DMSO-$d_6$, APT pulse sequence) δ d (CH, $CH_3$): 40.5, 126.6, 127.3, 128.8; u (C, $CH_2$): 26.8, 30.2, 32.5, 107.7, 115.7, 130.2, 146.4, 159.9, 168.3; IR 3443, 3139, 1646, 1557 $cm^{-1}$.

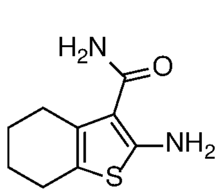

**Example 1.3: 2-Amino-4,5,6,7-tetrahydrobenzo[b]thiophene-3-carboxamide.** Cyclohexanone (mg, mmol) and 2-cyanoacetamide (mg, mmol, equiv) were reacted according to general procedure A to afford the thiophene product (Chem. Ber. 1966, 99, 94-100) as a light yellow solid (mg, mmol, % yield). $R_f$ = 0.26 (50% EtOAc in hexanes); $^1$H NMR (401 MHz, DMSO-$d_6$) δ 1.60-1.72 (complex, 4H), 2.38-2.45 (m, 2H), 2.53-2.59 (m, 2H), 6.48 (br s, 2H), 6.87 (br s, 2H); $^{13}$C NMR (101 MHz, DMSO-$d_6$, APT pulse sequence) δ u (C, $CH_2$): 23.0, 23.2, 24.5, 26.4, 108.0, 116.0, 130.5, 159.4, 168.3; IR 3147, 2927, 1639, 1550 $cm^{-1}$.

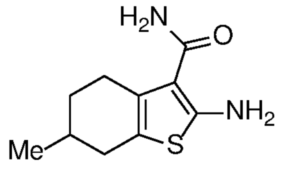

**Example 1.4: 2-Amino-6-methyl-4,5,6,7-tetrahydrobenzo[b]thiophene-3-carboxamide.** 4-Methylcyclohexanone (1.510 g, 13.46 mmol) and 2-cyanoacetamide (1.245 g, 14.81 mmol, 1.1 equiv) were reacted according to general procedure A to afford the thiophene product as a light yellow solid (0.819 g, 3.89 mmol, 29% yield).b $R_f$ = 0.26 (50% EtOAc in hexanes); $^1$H NMR (401 MHz, DMSO-$d_6$) δ 0.98 (d, *J* = 6.5 Hz, 3H), 1.20-1.30 (m, 1H), 1.70-1.80 (m, 2H), 2.01-2.10 (m, 1H), 2.45-2.51 (m, 1H), 2.56-2.62 (m, 2H), 6.47 (br s, 2H), 6.87 (br s, 2H); $^{13}$C NMR (101 MHz, DMSO-$d_6$, APT pulse sequence) δ d (CH, $CH_3$): 21.3, 28.9; u (C, $CH_2$): 25.7, 30.8, 32.2, 107.4, 115.2, 129.6, 159.2, 167.9.

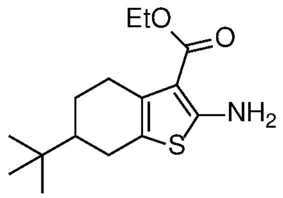

**Example 1.5: Ethyl 2-amino-6-phenyl-4,5,6,7-tetrahydrobenzo[b]thiophene-3-carboxylate.** 4-t-butylcyclohexanone (mg, mmol) and ethyl 2-cyanoacetate (mg, mmol, equiv) were reacted according to general procedure A to afford the previously described thiophene product (Org. Biomol. Chem. 2014, 12, 1942-1956) as a yellow oil (mg, mmol, % yield). $R_f$ = 0.80 (50% EtOAc in hexanes); $^1$H NMR (401 MHz, $CDCl_3$) δ 0.91 (s, 9H), 1.19-1.28 (m, 1H), 1.32 (t, *J* = 7.1 Hz, 3H), 1.41-1.50 (m, 1H), 1.91-1.98 (m, 1H), 2.24-2.32 (m, 1H), 2.44-2.54 (m, 2H), 2.92-3.00 (m, 1H), 4.25 (q, *J* = 7.2 Hz, 2H), 5.93 (br s, 2H); $^{13}$C NMR (101 MHz, $CDCl_3$, APT pulse sequence) δ d (CH, $CH_3$): 14.6, 27.4, 45.3; u (C, $CH_2$): 24.5, 26.1, 28.2, 32.5, 59.5, 105.5, 118.2, 132.5, 162.0, 166.2; IR 3328, 2958, 1660, 1577 $cm^{-1}$.

**Example 2: general procedure for coupling of amine scaffold and acid chloride.** To a solution of the amine-containing thiophene scaffold in pyridine (2 mL) and $CH_2Cl_2$ (4 mL) was added the acid chloride (1.5 equiv) and the reaction stirred at rt for 18 h. The solvents were removed in vacuo and the residue partitioned between aqueous NaOH (1 N, 5 mL) and a 10% solution of MeOH in $CH_2Cl_2$ (2 × 10 mL). The combined organic layers were dried ($Na_2SO_4$), evaporated in vacuo and purified by medium pressure silica gel chromatography to afford the amide product.

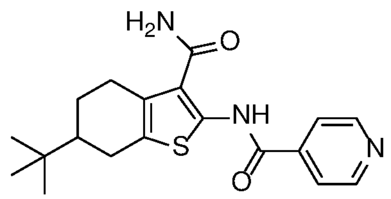

**Example 2.1: *N*-(6-(Tert-butyl)-3-carbamoyl-4,5,6,7-tetrahydrobenzo[b]thiophen-2-yl)isonicotinamide.** 2-Amino-6-(tert-butyl)-4,5,6,7-tetrahydrobenzo[b]thiophene-3-carboxamide (90.5 mg, 0.359 mmol) and isonicotinoyl chloride hydrochloride (77.0 mg, 0.430 mmol, 1.2 equiv) were reacted according to general procedure B to afford the amide product as a light yellow solid (33.0 mg, 0.092 mmol, 26% yield). $R_f$ = 0.47 (10% MeOH in $CH_2Cl_2$); $^1$H NMR (401 MHz, DMSO-$d_6$) δ 0.93 (s, 9H), 1.14-1.29 (m, 1H), 1.45 (dt, *J* = 4.1, 11.1 Hz, 1H), 2.00 (dd, *J* = 1.3, 12.1 Hz, 1H), 2.39-2.54 (m, 1H), 2.67-2.87 (m, 3H), 7.77 (d, *J* = 6.0 Hz, 2H), 8.84 (d, *J* = 5.2 Hz, 2H), 13.17 (br s, 1H); $^{13}$C NMR (101 MHz, DMSO-$d_6$, APT pulse sequence) δ d (CH, $CH_3$): 27.1, 44.3, 120.7, 150.9; u (C, $CH_2$): 24.0, 25.5, 26.2, 32.1, 116.5, 127.8, 129.3, 139.4, 142.6, 160.8, 167.6. HPLC purity 100%.

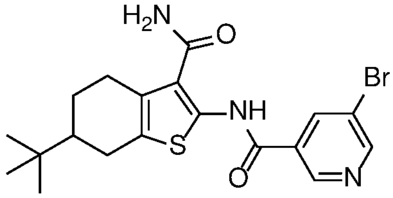

**Example 2.2: 5-Bromo-*N*-(6-(tert-butyl)-3-carbamoyl-4,5,6,7-tetrahydrobenzo[b]thiophen-2-yl)nicotinamide.** 2-Amino-6-(tert-butyl)-4,5,6,7-tetrahydrobenzo[b]thiophene-3-carboxamide (25.0 mg, 0.099 mmol), HOBt (22.8 mg, 0.149 mmol, 1.5 equiv), 3-bromonicotinicl acid (30.0 mg, 0.149 mmol, 1.5 equiv) and diisopropylcarbodiimide (31.3 mg, 0.248 mmol, 2.5 equiv) were charged to a microwave vial with MeCN (8 mL). The vial was sealed and heated at 100 °C for 10 minutes under microwave irradiation. The solvent was removed in vacuo and the residue partitioned between saturated, aqueous $NaHCO_3$ (10 mL) and $CH_2Cl_2$ (3 × 10 mL). The combined organic layers were evaporated and purified by reverse-phase chromatography to afford the amide product as an off-white solid (23.1 mg, 0.064 mmol, 65% yield). $R_f$ = 0.39 (50% EtOAc in hexanes);;; HRMS calcd. for $C_{19}H_{23}{}^{81}BrN_3O_2S$ [M + H]$^+$ 438.0668, found 438.0666. HPLC purity 100%.

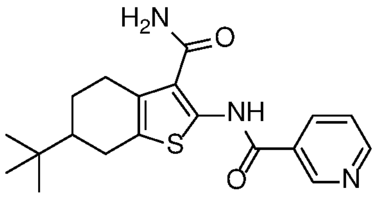

**Example 2.3: *N*-(6-(Tert-butyl)-3-carbamoyl-4,5,6,7-tetrahydrobenzo[b]thiophen-2-yl)nicotinamide.** 2-Amino-6-(tert-butyl)-4,5,6,7-tetrahydrobenzo[b]thiophene-3-carboxamide (55 mg, 0.22 mmol) and nicotinoyl chloride hydrochloride (58 mg, 0.33 mmol, 1.5 equiv) were reacted according to general procedure B and subsequently recrystallized from MeCN to afford the amide product as a light yellow solid (59 mg, 0.17 mmol, 76% yield). $R_f$ = 0.46 (10% MeOH in $CH_2Cl_2$); mp = 195-209 °C; $^1$H NMR (401 MHz, DMSO-$d_6$) δ 0.90 (s, 9H), 1.22 (dq, *J* = 5.2, 12.0 Hz, 1H), 1.42 (dt, *J* = 4.8, 7.9 Hz, 1H), 1.97 (d, *J* = 10.9 Hz, 1H), 2.39 (t, *J* = 14.6 Hz, 1H), 2.66-2.84 (m, 3H), 7.61 (dd, *J* = 4.8, 7.9 Hz, 1H), 8.20 (td, *J* = 1.8, 8.1 Hz, 1H), 8.77-8.81 (m, 1H), 9.03 (s, 1H), 13.08 (s, 1H); $^{13}$C NMR (101 MHz, DMSO-$d_6$, APT pulse sequence) δ d (CH, $CH_3$): 27.1, 44.3, 124.1, 134.8, 148.1, 152.9; u (C, $CH_2$): 24.0, 25.5, 26.2, 32.2, 116.2, 127.5, 128.2, 129.2, 142.9, 160.9, 167.7; IR 2950, 1663, 1633, 1586 cm$^{-1}$; HRMS calcd. for $C_{19}H_{24}N_3O_2S$ [M + H]$^+$ 358.1584, found 358.1594. HPLC purity 100%.

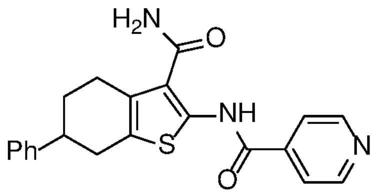

**Example 2.4: *N*-(3-Carbamoyl-6-phenyl-4,5,6,7-tetrahydrobenzo[b]thiophen-2-yl)nicotinamide.** 2-Amino-6-phenyl-4,5,6,7-tetrahydrobenzo[b]thiophene-3-carboxamide (43 mg, 0.16 mmol) and nicotinoyl chloride hydrochloride (42 mg, 0.24 mmol, 1.5 equiv) were reacted according to general procedure B and subsequently recrystallized from ethanol and $CH_2Cl_2$ to afford the amide product as a light yellow solid (26 mg, 0.07 mmol, 43% yield). $R_f$ = 0.52 (10% MeOH in $CH_2Cl_2$); mp = 235-239 °C; $^1$H NMR (401 MHz, DMSO-$d_6$) δ 1.84-1.95 (m, 1H), 1.99-2.07 (m, 1H), 2.72-2.84 (m, 1H), 2.84-3.01 (complex, 4H), 7.18-7.24 (m, 1H), 7.28-7.34 (complex, 4H), 7.62 (dd, *J* = 4.8, 7.8 Hz, 1H), 8.21 (td, *J* = 2.2, 8.1 Hz, 1H), 8.80 (dd, *J* = 1.4, 4.8 Hz, 1H), 9.04 (d, *J* = 1.9 Hz, 1H), 13.08 (s, 1H); $^{13}$C NMR (101 MHz, DMSO-$d_6$, APT pulse sequence) δ d (CH, $CH_3$): 40.2, 124.5, 126.7, 127.3, 128.9, 135.3, 148.5, 153.4; u (C, $CH_2$): 26.0, 30.0, 32.2, 116.9, 127.0, 128.7, 129.5, 143.3, 146.1, 161.5, 168.1. HPLC purity 95.7%.

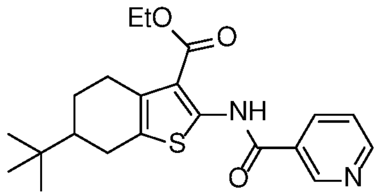

**Ethyl 6-(*tert*-butyl)-2-(nicotinamido)-4,5,6,7-tetrahydrobenzo[b]thiophene-3-carboxylate.** Ethyl 2-amino-6-(tert-butyl)-4,5,6,7-tetrahydrobenzo[b]thiophene-3-carboxylate (73 mg, 0.26 mmol) and nicotinoyl chloride hydrochloride (69 mg, 0.39 mmol, 1.5 equiv) were reacted according to general procedure B and subsequently recrystallized from ethanol and $CH_2Cl_2$ to afford the amide product as a light yellow solid (37 mg, 0.10 mmol, 37% yield). $R_f$ = 0.78 (10% MeOH in $CH_2Cl_2$); mp = 136-138 °C; $^1$H NMR (401 MHz, $CDCl_3$) δ 0.95 (s, 9H), 1.31 (dq, *J* = 5.2, 12.5 Hz, 1H), 1.40 (t, *J* = 7.1 Hz, 3H), 1.45-1.53 (m, 1H), 2.04 (dd, *J* = 3.7, 12.8 Hz, 1H), 2.44 (t, *J* = 13.2 Hz, 1H), 2.55-2.65 (m, 1H), 2.74 (dd, *J* = 4.8, 15.9 Hz, 1H), 3.07 (dd, *J* = 5.1, 17.4 Hz, 1H), 4.37 (q, *J* = 7.1 Hz, 2H), 7.50 (dd, *J* = 4.9, 8.0 Hz, 1H), 8.32 (td, *J* = 1.8, 8.1 Hz, 1H), 8.81 (d, *J* = 4.1 Hz, 1H), 9.26 (s, 1H), 12.44 (s, 1H); $^{13}$C NMR (101 MHz, $CDCl_3$, APT pulse sequence) δ d (CH, $CH_3$): 14.3, 27.3, 45.0, 123.8, 135.5, 148.3, 152.4; u (C, $CH_2$): 24.4, 25.9, 27.5, 32.4, 112.3, 128.4, 128.6, 131.2, 147.3, 161.3, 167.0. HPLC purity 96.7%.

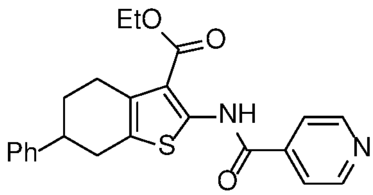

**Ethyl 2-(isonicotinamido)-6-phenyl-4,5,6,7-tetrahydrobenzo[b]thiophene-3-carboxylate.** Ethyl 2-amino-6-phenyl-4,5,6,7-tetrahydrobenzo[*b*]thiophene-3-carboxylate (81 mg, 0.27 mmol) and isonicotinoyl chloride hydrochloride (72 mg, 0.40 mmol, 1.5 equiv) were reacted according to general procedure B to afford the amide product as a light yellow solid (50 mg, 0.12 mmol, 46% yield). $R_f$ = 0.78 (10% MeOH in $CH_2Cl_2$); mp = 248-251 °C; $^1$H NMR (401 MHz, $CDCl_3$) δ 1.42 (t, *J* = 7.1 Hz, 3H), 1.95 (dq, *J* = 5.7, 12.2 Hz, 1H), 2.13-2.20 (m, 1H), 2.78-2.88 (m, 2H), 2.96-3.12 (complex, 3H), 4.40 (q, *J* = 7.1 Hz, 2 H), 7.26-7.85 (complex, 5H), 7.84 (d, *J* = 6.1 Hz, 2H), 8.84 (d, *J* = 5.8 Hz, 2H), 12.52 (s, 1H); $^{13}$C NMR (101 MHz, $CDCl_3$, APT pulse sequence) δ d (CH, $CH_3$): 14.3, 40.5, 120.9, 126.5, 126.9, 128.5, 151.0; u (C, $CH_2$): 26.7, 30.0, 32.3, 61.0, 112.6, 127.4, 131.1, 139.5, 145.6, 147.4, 161.5, 167.0. HPLC purity 96.9%.

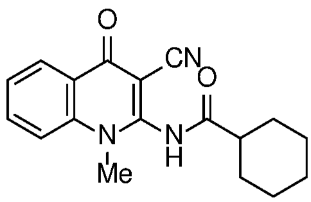

**Example 3.1: *N*-(3-Cyano-1-methyl-4-oxo-1,4-dihydroquinolin-2-yl)cyclohexanecarboxamide.** To a solution of 2-amino-1-methyl-4-oxo-1,4-dihydroquinoline-3-carbonitrile (J. Heterocyclic Chem. **1979,** *16*, 1605-1610)(450 mg, 2.26 mmol) in pyridine (3 mL) was added cyclohexanecarbonyl chloride (0.60 mL, 4.52 mmol, 2.0 equiv) in four portions. The reaction was stirred at rt for 16 h and quenched with water (2 mL). The residue was extracted with chloroform (3 × 20 mL) and the combined organics dried ($Na_2SO_4$). The solvent was removed in vacuo and the residue purified by silica gel chromatographed to afford the amide product as an off-white solid (550 mg, 1.78 mmol, 79% yield). $^1$H NMR (500 MHz, DMSO-$d_6$) δ 1.17-1.36 (complex, 3H), 1.45 (dq, *J* = 2.7, 12.0 Hz, 1H), 1.63-1.69 (m, 1H), 1.76-1.81 (m, 2H), 1.92-1.98 (m, 2H), 2.51-2.56 (m, 1H), 3.73 (s, 3H), 7.57-7.60 (m, 1H), 7.88-7.92 (m, 1H), 7.92-7.96 (m, 1H), 8.22 (dd, *J* = 1.5, 7.9 Hz, 1H), 10.99 (br s, 1H); $^{13}$C NMR (126 MHz, DMSO-$d_6$) δ 25.0, 25.3, 28.6, 35.3, 43.8, 93.0, 115.3, 118.5, 124.9, 125.4, 125.8, 133.9, 139.7, 151.3, 174.5, 174.9; HRMS calcd. for $C_{19}H_{24}N_3O_2S$ [M + H]$^+$ 358.1584, found 358.1594.

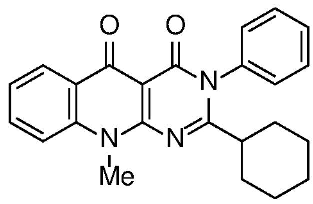

**Example 3.2: 2-Cyclohexyl-10-methyl-3-phenylpyrimido[4,5-b]quinoline-4,5(3H,10H)-dione.** *N*-(3-Cyano-1-methyl-4-oxo-1,4-dihydroquinolin-2-yl)cyclohexanecarboxamide (30.0 mg, 0.097 mmol), aniline (45.2 mg, 0.485 mmol, 5.0 equiv) and copper (II) acetate (17.6 mg, 0.097 mmol, 1.0 equiv) were combined in water (1 mL) in a microwave vial. The reaction was sealed and heated to 100 °C for 2 h under microwave irradiation. The solvent was removed in vacuo and the residue purified by preparative, mass-directed, reverse-phase HPLC to afford the pyrimidinone product as a light brown solid (7.5 mg, 0.019 mmol, 20% yield). $^1H$ NMR (400 MHz, DMSO-$d_6$) δ 0.80-0.94 (m, 2H), 1.11-1.31 (m, 1H), 1.51-1.71 (complex, 5H), 1.79-1.87 (m, 2H), 2.16 (tt, *J* = 3.1, 11.2 Hz, 1H), 4.04 (s, 3H), 7.39-7.43 (m, 3H), 7.50-7.60 (m, 3H), 7.77-7.84 (m, 2H), 8.18-8.22 (m, 1H); HRMS calcd. for $C_{24}H_{24}N_3O_2$ $[M + H]^+$ 386.1863, found 386.1836. HPLC purity 100%.

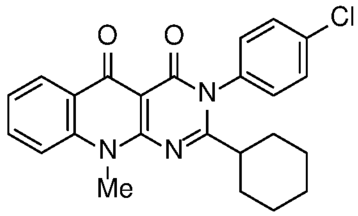

**Example 3.3: 3-(4-Chlorophenyl)-2-cyclohexyl-10-methylpyrimido[4,5-b]quinoline-4,5(3H,10H)-dione.** *N*-(3-Cyano-1-methyl-4-oxo-1,4-dihydroquinolin-2-yl)cyclohexanecarboxamide (50.0 mg, 0.162 mmol), 4-chloroaniline (103.0 mg, 0.808 mmol, 5.0 equiv) and copper (II) acetate (14.7 mg, 0.081 mmol, 0.5 equiv) were combined in water (0.5 mL) in a microwave vial. The reaction was sealed and heated to 100 °C for 2 h under microwave irradiation. The solvent was removed in vacuo and the residue purified by preparative, mass-directed, reverse-phase HPLC to afford the pyrimidinone product as a light brown solid (6.6 mg, 0.016 mmol, 10% yield). $^1H$ NMR (500 MHz, $CDCl_3$) δ 1.03-1.13 (m, 2H), 1.23-1.32 (m, 1H), 1.64-1.86 (complex, 7H), 2.28-2.36 (m, 1H), 4.09 (s, 3H), 7.18-7.22 (m, 2H), 7.41 (t, *J* = 7.0 Hz, 1H), 7.53 (d, *J* = 8.2 Hz, 2H), 7.57-7.64 (m, 1H), 7.73 (t, *J* = 7.5 Hz, 1H), 8.49-8.56 (m, 1H); HRMS calcd. for $C_{24}H_{23}ClN_3O_2$ $[M + H]^+$ 420.1473, found 420.1473. HPLC purity 99.6%.

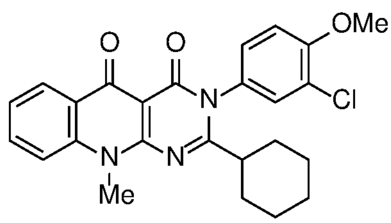

**Example 3.4: 3-(3-Chloro-4-methoxyphenyl)-2-cyclohexyl-10-methylpyrimido[4,5-b]quinoline-4,5(3H,10H)-dione.** *N*-(3-Cyano-1-methyl-4-oxo-1,4-dihydroquinolin-2-yl)cyclohexanecarboxamide (50.0 mg, 0.162 mmol), 3-chloro-4-methoxyaniline (127.0 mg, 0.808 mmol, 5.0 equiv) and copper (II) acetate (14.7 mg, 0.081 mmol, 0.5 equiv) were combined in water (0.5 mL) in a microwave vial. The reaction was sealed and heated to 100 °C for 2 h under microwave irradiation. The solvent was removed in vacuo and the residue purified by preparative, mass-directed, reverse-phase HPLC to afford the pyrimidinone product as a light brown solid (8.9 mg, 0.020 mmol, 12% yield). $^1H$ NMR (500 MHz, $CDCl_3$) δ 1.04-1.17 (m, 2H), 1.23-1.32 (m, 1H), 1.61-1.89 (complex, 7H), 2.34-2.41 (m, 1H), 4.00 (s, 3H), 4.09 (s, 3H), 7.06-7.08 (m, 1H), 7.13 (dd, *J* = 2.3, 8.7 Hz, 1H), 7.27 (d, *J* = 2.5 Hz, 1H), 7.41 (t, *J* = 7.4 Hz, 1H), 7.59 (d, *J* = 8.5 Hz, 2H), 7.73 (t, *J* = 8.3 Hz, 1H), 8.53 (d, *J* = 7.6 Hz, 1H); HRMS calcd. for $C_{25}H_{25}ClN_3O_3$ $[M + H]^+$ 450.1579, found 450.1587. HPLC purity 95.5%.

EXAMPLE 10

This example demonstrates that compound 1 and compound 28 potentiate dopamine-induced D1R internalization.

Receptor internalization was measured using the DiscoverX GPCR internalization assay as described in Materials and Methods. Cells were treated with the indicated concentrations of dopamine (DA) for 3 hours in the absence or presence of 50 μM compound **1**, or 50 μM compound **28.** The results are shown in FIG. 10 as a plot of $D_1$R internalization versus dopamine concentration. Both compound **1** and compound **28** potentiated DA's potency for inducing receptor internalization ($EC_{50}$ [95% CI]: DA = 2.79 μM [1.4 - 5.6], DA + compound **1** = 0.53 μM [0.16 - 1.8], *p* = 0.004), DA + compound **28** 0.46 μM [0.15 - 1.45], *p* = 0.04). Further, compound **1** increased DA's efficacy for internalization *(p* = 0.04), but compound **28** showed no potentiation of efficacy (Emax ± SEM: DA = 97.4% ± 4.7, DA + compound **1** = 113.5% ± 2.8, DA + compound **28** = 97.2% ± 3.0,). Statistical comparisons via Students t-test, n=5.

EXAMPLE 11

This example demonstrates that compound **1** and compound **28** increase the efficacy and potency of the D1R agonist dihydrexidine.

β-arrestin recruitment was measured following stimulation by the indicated concentrations of dihydrexidine in the absence or presence of 50 μM of either compound **1** or compound **28.** DA was run as a control in every experiment and the data are plotted in FIG. 11 as the percentage of the maximum DA response observed. Both compound **1** and compound **28** increase the efficacy and potency of dihydrexidine: ($EC_{50}$ [95% CI]) dihydrexidine = 73.3 nM [42.8 -125.2], dihydrexidine + compound **1 =** 20.9 nM [9.7 - 45.5], *p* < 0.0001, dihydrexidine ± compound **28** = 18.6 nM [11.3 - 30.6], p < 0.0001; (Emax ± SEM) dihydrexidine = 70.5% ± 2.5; dihydrexidine + compound **1** = 95.3% ± 3.8, *p* < 0.001; dihydrexidine + compound **28** = 96.9 % ± 3.2, *p* < 0.001. Data are displayed as a percentage of the maximum control stimulation seen with dopamine, mean ± SEM, and statistical comparisons are via paired two-tailed Students t-test, n =5.

EXAMPLE 12

This example demonstrates that compound **1** and known D1R positive allosteric modulator Compound B (Lewis, et al., 2015) act at the same site on D1R which is separate from that of compound **28.**

β-arrestin recruitment was measured following stimulation with dopamine in the absence (DA) or in the presence of 50 μM compound **1**, 50 μM compound **28,** 100 μM Compound B, or the indicated combination of the three compounds. The structure of compound B is shown in FIG. 12A. FIG. 12B shows that compound **1** and Compound B both potentiate DA's potency ($EC_{50}$ [95% CI]: DA = 4.19 μM [2.4 - 7.5]; DA + compound **1** = 0.68 μM [0.36 - 1.3], *p* = 0.004; DA + Compound B = 0.56 μM [0.22 - 1.4], *p* = 0.01). Addition of compound **1** and Compound B together caused the same level of potentiation as either compound alone ($EC_{50}$ [95% CI]: DA + compound **1** + Compound B = 0.5 μM [0.29 - 0.86]), suggesting that Compound B and compound **1** act at similar sites on the D1R. FIG. 12C shows that compound **28** and Compound B both potentiated DA's potency for β-arrestin recruitment ($EC_{50}$ [95% CI]: DA = 4.2 μM [2.4 - 7.5]; DA + compound **28** = 0.39 μM [0.27 - 0.54], *p* =0.0002); DA + Compound B = 0.56 μM [0.22 - 1.4], *p* = 0.01). Addition of compound **28** and Compound B together resulted in a greater potentiation of DA's potency than either compound alone ($EC_{50}$ ± SEM: DA + compound **28** + Compound B = 0.09 μM [0.02 - 0.46], *p* = 0.004), suggesting the two compounds act via separate binding sites on the D1R. Statistical comparisons via paired two-tailed Students t-test, n = 5.EXAMPLE 13

This example demonstrates that a R130Q point mutation in D1R abolishes compound **1**, but not compound **28** PAM activity, suggesting a binding location for compound **1.**

Dopamine stimulated β-arrestin recruitment and G protein ($G\alpha_s$) engagement were measured using BRET assays as described in the Methods. Briefly, cells were transfected with either the wild-type D1R or the R130Q mutant along with the indicated biosensor. Cells were then stimulated with the indicated concentrations of dopamine alone (DA) or in the presence of 50 μM compound **1** or 50 μM compound **28.** FIG. 13A shows that both compound **1** and compound **28** potentiated DA's potency for β-arrestin recruitment to the wild type D1R ($EC_{50}$ [95% CI]: DA = 1.76 μM [0.69- 4.5]; DA + compound **1** = 0.45 μM [0.2 - 1.0], p < 0.001; DA + compound **28** = 0.43 μM [0.21 - 0.86], p < 0.001). Further, both compound **1** and compound **28** increased DA's efficacy (Emax ± SEM: DA = 99.9% ± 1.2; DA + compound **1** = 132.7% ± 2.2); DA + compound **28** = 114.5% +/- 1.6, *p* < 0.01). FIG. 13B shows that with the mutant R130Q receptor, compound **28,** but not compound **1,** potentiated DA's potency ($EC_{50}$ [95% CI]: DA = 1.67 μM [0.79 - 3.5]; DA + compound **1 =** 1.23 μM [0.44 - 3.5]; DA + compound **28** = 0.38 μM [0.19 - 0.75], *p* < 0.0001). Further, compound **1** did not potentiate DA's efficacy for activating the R130Q mutant (Emax ± SEM: DA = 98.4% ± 5.2; DA + compound **1 =** 105.4% ± 2.9), however, compound **28** did potentiate the Emax (DA + compound **28** = 111.3 +/- 3.1, *p* <0.03). FIG. 13C shows that with the WT receptor, both compound **1** and compound **28** enhanced DA's potency for stimulating D1R-Gαs interactions ($EC_{50}$ [95% CI]: DA = 0.37 μM [0.24 - 0.57]; DA + compound **1** = 0.12 μM [0.09 -0.16], *p* = 0.0001; DA + compound **28** (0.07 μM [0.04 - 0.12], *p* = 0.001). Compound **1** also promoted a measurable increase in DA efficacy, while compound **28** did not (Emax ± SEM: DA = 100.3% ± 2.1; DA + compound **1** = 109.4% ± 2.2; DA + compound **28** = 101.7% ± 3.4). FIG. 13D shows that with the mutant R130Q receptor, compound **28,** but not compound **1,** enhanced DA's potency ($EC_{50}$ [95%

CI]: DA = 0.39 μM [0.25 - 0.6]; DA + compound **1** = 0.23 μM [0.17 - 0.31]; DA + compound **28** = 0.069 μM [0.05 - 0.11], $p < 0.0001$). Further, neither compound increased DA's efficacy at the R130Q receptor (Emax ± SEM: DA = 100.3% ± 49; DA+compound **1** = 98.2% ± 4.8; DA+compound **28** = 107.3% ± 5.3). Together, these data indicate that the binding site for compound **1** on the D1R includes the R130 residue, while compound **28** acts at a separate, yet unknown site. Statistical comparisons via paired two-tailed Students t-test, and one-way ANOVA; n = 5-6.

EXAMPLE 14

This example demonstrates that compound **1** and compound **28** potentiate D5R-mediated β-arrestin recruitment.

β-arrestin recruitment was measured following stimulation by the indicated concentrations of dopamine either alone (DA) or in the presence of 50 μM of either compound **1** or compound **28** in the DiscoverX assay, as described in the Methods, using cells stably expressing human D5R. The results are shown in FIG. 14. Each PAM was also examined in the absence of DA as an agonist, as indicated. Both PAM compounds were found to potentiate both the affinity and efficacy of dopamine stimulation of β-arrestin recruitment to the D5R. Neither PAM produced an agonist response when tested alone. $EC_{50}$ (Mean [95% CI]): DA = 0.16 μM [0.13 - 0.19], DA + 50 μM compound **1** = 0.094 μM [0.07-0.13] (*$p < 0.01$* vs. DA control), DA + 50 μM compound **28** = 0.1 μM [0.082-0.13] (*$p < 0.01$* vs. DA control). Emax (Mean ± SEM): DA = 92.5% ± 1.5, DA + 50 μM compound **1** = 117% ± 2.7 (*$p < 0.0001$* vs. DA control), DA + 50 μM compound **28** = 130% ± 2.4 ($p < 0.0001$ vs. DA control) Data are displayed as a percentage of the maximum control stimulation seen with dopamine, mean ± SEM, n = 3 of experiments run in quadruplicate.

EXAMPLE 15

This example demonstrates that neither compound **1** nor compound **28** potentiate D2-like dopamine receptor- or β2 adrenergic receptor-mediated β-arrestin recruitment.

β-arrestin recruitment was measured following stimulation by the indicated concentrations of either dopamine (DA) or epinephrine (EPI) in the presence of 50 μM of either compound **1** or compound **28** in the β-arrestin BRET assay, as described in the methods. Neither PAM produced any measurable potentiation of any agonist response at these receptors. FIGS. 15A-15C show the $D_2$R-arrestin BRET ratios, $D_3$R-arrestin BRET ratios, and $D_4$R-arrestin BRET ratios versus dopamine concentration, respectively. FIG. 15D shows the β2AR- arrestin BRET ratios versus epinephrine concentration. Data are displayed as a percentage of the maximum control stimulation seen with either dopamine or epinephrine, mean ± SEM, n = 3 of experiments run in triplicate.

EXAMPLE 16

This example describes allosteric modeling data, in accordance with an embodiment of the invention.

The curve shift binding data (EXAMPLE 4, FIGS. 4A-4C) were fit to the allosteric ternary complex model to estimate the affinity of each PAM for the D1R in the absence of the endogenous ligand (*K*b) and the binding cooperativity between the PAM and dopamine ($\alpha$), where $\alpha > 1$ indicates positive cooperativity. For compound **1**, the model estimated an affinity in the submicromolar range (pKb = 6.27 ± 0.19, Kb = 0.54 μM) and an $\alpha$ of 3.09 (log$\alpha$ = 0.49 ± 0.07). Compound **28** had a low micromolar affinity (pKb = 5.27 ± 0.03, Kb = 5.37 μM) but a greater $\alpha$ of 6.61 (log$\alpha$ = 0.82 ± 0.11).

Data from β-arrestin recruitment curve shift experiments (EXAMPLE 2, FIGS. 2A-2B) were used in conjunction with the outputs from the allosteric ternary complex model described above to assess the effect of compound **1** and compound **28** on dopamine's efficacy using an operation model of allosterism. This model estimates modulator affinity in the absence of dopamine (*K*b), cooperativity with dopamine affinity ($\alpha$), and modulatory effect of the compound upon the efficacy of the orthosteric agonist ($\beta$). For compound **1**, *K*b was determined to be 0.46 μM (pKb = 6.34 ± 0.08) and for compound **28,** *K*b was determined to be 5.4 μM (pKb = 5.27 ± 0.24). $\alpha$ cooperativity constants were fixed to those determined via the allosteric ternary complex model described above. Both compound **1** and **28** were found to have $\beta$ factors greater than one, indicating a positive modulatory effect on dopamine efficacy [log$\beta$ ($\beta$): compound **1** = 0.41 ± 0.06 (2.57); compound **28** = 0.32 ± 0.1 (2.09)]

EXAMPLE 17

This example demonstrates the pharmacological activities of compound **1** and compound **28** on select catecholamine receptors (EXAMPLE 15, FIGS. 15A-15D), in accordance with an embodiment of the invention. The results are set forth in Table 4.

Table 4

| | Agonist | | Agonist + 50 μM compound **1** | | Agonist + 50 μM compound **28** | |
|---|---|---|---|---|---|---|
| | $EC_{50}$ | Emax (% Ctrl) | $EC_{50}$ | Emax (% Ctrl) | $EC_{50}$ | Emax (% Ctrl) |
| D2R⁺ | 42.6 nM [21.1-85.9] | 100 ± 12.48 | 39.9 nM [23.5 - 67.9] | 105 ± 14.9 | 38.7 nM [22.4 - 66.9] | 106 ± 14.4 |
| D3R⁺ | 2.7 nM [1.6 - 4.6] | 100 ± 32.4 | 2.3 nM [1 - 5.8] | 92.1 ± 22.3 | 2.5 nM [1.1 - 5.7] | 105 ± 36.5 |
| D4R⁺ | 48.2 nM [32.6 - 70.4] | 99.9 ±2.5 | 47.0nM [13.6 - 159] | 92.3 ±7.4 | 37.6 nM [19.3 - 71.7] | 105 ± 4.0 |
| β2AR⁺⁺ | 0.7 μM [0.53 - 0.93] | 99.9 ± 1.18 | 0.84 μM [0.5 - 1.4] | 104 ± 1.3 | 1.04 μM [0.35 - 3.08] | 99.1 ± 1.89 |

$EC_{50}$ and Emax values were derived from the D2-like and β2 receptor selectivity experiments. Curve parameters were determined via non-linear regression of individual experiments normalized to a dopamine or epinephrine control. $EC_{50}$ values expressed as geometric mean [95% confidence interval]; Emax values expressed as mean ± SEM. N = 3-5 experiments run in triplicate. Statistical significance determined via t-test.
⁺ Agonist = Dopamine
⁺⁺ Agonist = Epinephrine

EXAMPLE 18

This example demonstrates activity of compounds in the dopamine D1-(β-arrestin assay, dopamine D5-β-arrestin assay, and dopamine D4-(β-arrestin assay, in accordance with an embodiment of the invention.

Tables 5-7:

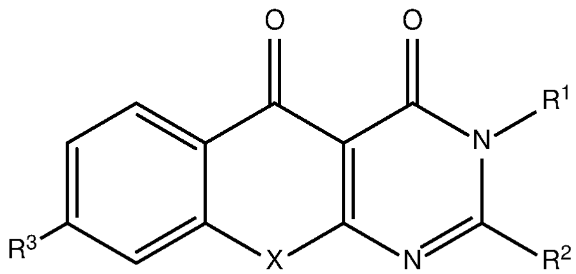

Table 5: Dopamine D1-(β-arrestin Assay

| $R^1$ | $R^2$ | $R^3$ | X | DA + 50 μM PAM, EC50 (M) | DA + 50 μM PAM, Fold Change EC50 | DA + 50 μM PAM, Emax (%DA) | n (# of experiments) |
|---|---|---|---|---|---|---|---|
| Ph | Cyclohexyl | H | NMe | 2.50E-07 | 4.64 | 120% | 7 |
| Cycloheptyl | Cyclohexyl | H | NMe | 1.43E-07 | 3.85 | 109% | 1 |
| Cycloheptyl | n-Propyl | H | NMe | 1.60E-07 | 3.47 | 87% | 1 |
| 3,5-diMePh | n-Propyl | H | NMe | 3.92E-08 | 14.1 | 57% | 1 |
| Phenylethyl | i-Propyl | H | NMe | 3.51E-07 | 1.57 | 109% | 1 |
| 4-FPh | i-Propyl | H | NMe | 2.15E-07 | 1.69 | 110% | 1 |
| Ph | 4-t-BuPh | H | NMe | 2.72E-07 | 1.33 | 117% | 1 |
| i-Bu | 2-furyl | H | NMe | 5.44E-07 | 2.06 | 76% | 1 |
| Cycloheptyl | n-Pentyl | H | NMe | 3.23E-07 | 1.12 | 120% | 1 |

(continued)

| $R^1$ | $R^2$ | $R^3$ | X | DA + 50 μM PAM, EC50 (M) | DA + 50 μM PAM, Fold Change EC50 | DA + 50 μM PAM, Emax (%DA) | n (# of experiments) |
|---|---|---|---|---|---|---|---|
| Ph | 4-MePh | MeO | O | 4.26E-07 | 0.85 | 121% | 1 |
| Bn | 4-FPh | H | NMe | 8.51E-07 | 1.32 | 123% | 1 |

Table 6: Dopamine D5-β-arrestin Assay

| $R^1$ | $R^2$ | $R^3$ | X | DA + 50 μM PAM, EC50 (M) | DA + 50 μM PAM, Fold Change EC50 | DA + 50 μM PAM, Emax (%DA) | n |
|---|---|---|---|---|---|---|---|
| Ph | Cyclohexyl | H | NMe | 7.00E-07 | 2.56 | 122% | 5 |
| Cycloheptyl | Cyclohexyl | H | NMe | 4.52E-08 | 1.71 | 178% | 1 |
| Cycloheptyl | n-Propyl | H | NMe | 9.19E-09 | 7.78 | 111% | 1 |
| 3,5-diMePh | n-Propyl | H | NMe | 2.03E-08 | 3.81 | 134% | 1 |
| Phenylethyl | i-Propyl | H | NMe | 1.47E-08 | 20.5 | 144% | 1 |
| 4-FPh | i-Propyl | H | NMe | 3.17E-08 | 2.44 | 160% | 1 |
| Ph | 4-t-BuPh | H | NMe | 8.46E-09 | 35.49 | 144% | 1 |
| i-Bu | 2-furyl | H | NMe | 1.75E-08 | 17.2 | 83% | 1 |
| Cycloheptyl | n-Pentyl | H | NMe | 1.99E-08 | 3.89 | 168% | 1 |
| Ph | 4-MePh | MeO | O | 6.08E-08 | 4.94 | 148% | 1 |
| Bn | 4-FPh | H | NMe | 4.46E-08 | 6.74 | 169% | 1 |

Table 7: Dopamine D4-β-arrestin Assay

| $R^1$ | $R^2$ | $R^3$ | X | DA + 50 μM PAM, EC50 (M) | DA + 50 μM PAM, Fold Change EC50 | DA + 50 μM PAM, Emax (%DA) | n |
|---|---|---|---|---|---|---|---|
| Ph | Cyclohexyl | H | NMe | 2.60E-07 | 1.06 | 115% | 5 |
| Cycloheptyl | Cyclohexyl | H | NMe | 1.48E-07 | 0.95 | 110% | 1 |
| Cycloheptyl | n-Propyl | H | NMe | 1.76E-07 | 0.8 | 101% | 1 |
| 3,5-diMePh | n-Propyl | H | NMe | 1.49E-07 | 0.95 | 103% | 1 |
| Phenylethyl | i-Propyl | H | NMe | 1.26E-07 | 1.27 | 115% | 1 |
| 4-FPh | i-Propyl | H | NMe | 8.04E-08 | 1.76 | 120% | 1 |
| Ph | 4-t-BuPh | H | NMe | 1.99E-07 | 0.8 | 117% | 1 |
| i-Bu | 2-furyl | H | NMe | 9.44E-08 | 1.7 | 100% | 1 |
| Cycloheptyl | n-Pentyl | H | NMe | 9.68E-08 | 1.46 | 97% | 1 |
| Ph | 4-MePh | MeO | O | 1.61E-07 | 0.99 | 154% | 1 |
| Bn | 4-FPh | H | NMe | 1.29E-07 | 1.24 | 128% | 1 |

Tables 8-10: 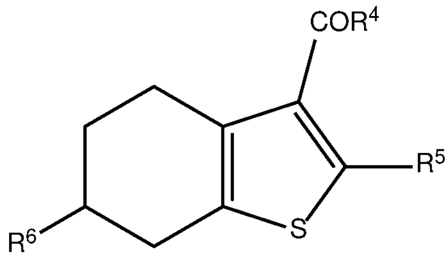

Table 8: Dopamine D1-(β-arrestin Assay

| $R^4$ | $R^5$ | $R^6$ | DA + 50 μM PAM, EC50 (M) | DA + 50 μM PAM, Fold Change EC50 | DA + 50 μM PAM, Emax (%DA) | n |
|---|---|---|---|---|---|---|
| $NH_2$ | NHCO-4-pyridyl | t-Bu | 1.40E-07 | 8.29 | 134% | 12 |
| $NH_2$ | 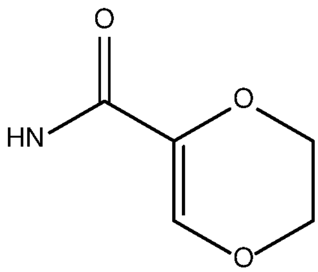 | Me | 8.90E-07 | 1.41 | 106% | 3 |
| $NH_2$ | NHCO-2-furanyl | t-Bu | 4.69E-07 | 2.19 | 126% | 3 |
| $NH_2$ | NHCO-3-pyridyl | H | 5.47E-07 | 1.88 | 104% | 3 |
| NHCH2-3-pyridyl | H | Me | 8.04E-07 | 1.27 | 129% | 3 |
| NH-2-MePh | NHCO-4-pyridyl | Me | 6.05E-07 | 2.09 | 110% | 3 |
| $NH_2$ | NHCO-4-HOPh | Me | 5.54E-07 | 1.76 | 137% | 3 |
| $NH_2$ | NHCO2-FPh | Me | 8.76E-07 | 1.39 | 108% | 3 |
| 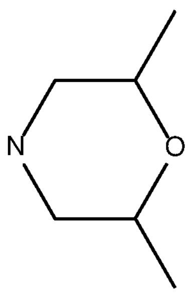 | NHCO-4-Cl | t-Bu | 6.47E-07 | 1.89 | 108% | 3 |
| $NH_2$ | 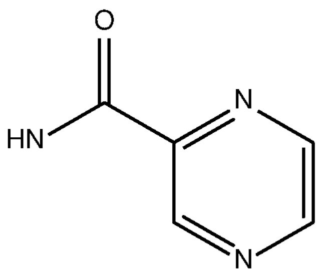 | H | 5.54E-07 | 1.76 | 125% | 3 |

Table 9: Dopamine D5-β-arrestin Assay

| $R^4$ | $R^5$ | $R^6$ | DA + 50 μM PAM, EC50 (M) | DA + 50 μM PAM, Fold Change EC50 | DA + 50 μM PAM, Emax (%DA) | n |
|---|---|---|---|---|---|---|
| $NH_2$ | NHCO-4-pyridyl | t-Bu | 1.14E-07 | 1.25 | 139% | 9 |
| $NH_2$ | 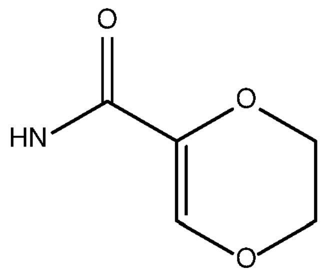 | Me | 9.02E-08 | 0.89 | 122% | 2 |
| $NH_2$ | NHCO-2-furanyl | t-Bu | 5.22E-08 | 1.14 | 144% | 2 |
| $NH_2$ | NHCO-3-pyridyl | H | 5.76E-08 | 1.03 | 108% | 2 |
| NHCH2-3-pyridyl | H | Me | 9.18E-08 | 0.65 | 147% | 2 |
| NH-2-MePh | NHCO-4-pyridyl | Me | 9.72E-08 | 0.83 | 117% | 2 |
| $NH_2$ | NHCO-4-HOPh | Me | 6.86E-08 | 0.87 | 174% | 2 |
| $NH_2$ | NHCO2-FPh | Me | 1.31E-07 | 0.61 | 131% | 2 |
| 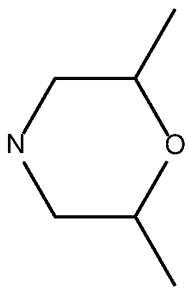 | NHCO-4-Cl | t-Bu | 7.20E-08 | 0.77 | 114% | 2 |
| $NH_2$ | 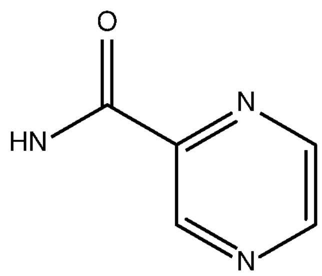 | H | 9.06E-08 | 0.59 | 136% | 2 |

Table 10: Dopamine D4-β-arrestin Assay

| $R^4$ | $R^5$ | R6 | DA + 50 μM PAM, EC50 (M) | DA + 50 μM PAM, Fold Change EC50 | DA + 50 μM PAM, Emax (%DA) | n |
|---|---|---|---|---|---|---|
| $NH_2$ | NHCO-4-pyridyl | t-Bu | 1.24E-07 | 1.72 | 160% | 6 |

(continued)

| $R^4$ | $R^5$ | R6 | DA + 50 μM PAM, EC50 (M) | DA + 50 μM PAM, Fold Change EC50 | DA + 50 μM PAM, Emax (%DA) | n |
|---|---|---|---|---|---|---|
| $NH_2$ | 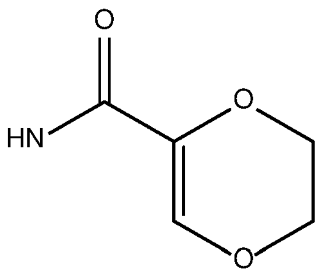 | Me | 2.49E-07 | 0.74 | 120% | 2 |
| $NH_2$ | NHCO-2-furanyl | t-Bu | 1.66E-07 | 1.17 | 119% | 2 |
| $NH_2$ | NHCO-3-pyridyl | H | 1.60E-07 | 1.21 | 100% | 2 |
| NHCH2-3-pyridyl | H | Me | 2.65E-07 | 0.73 | 129% | 2 |
| NH-2-MePh | NHCO-4-pyridyl | Me | 1.70E-07 | 1.09 | 114% | 2 |
| $NH_2$ | NHCO-4-HOPh | Me | 1.61E-07 | 1.2 | 163% | 2 |
| $NH_2$ | NHCO2-FPh | Me | 2.72E-07 | 0.68 | 135% | 2 |
| 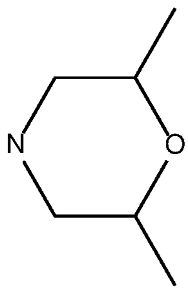 | NHCO-4-Cl | t-Bu | 1.83E-07 | 1.01 | 115% | 2 |
| $NH_2$ | 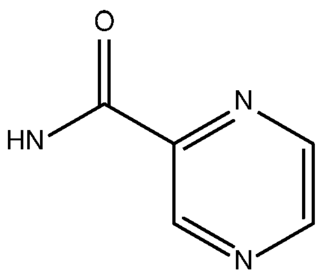 | H | 2.24E-07 | 0.86 | 120% | 2 |

REFERENCES CITED

Amsten, A. F. and A. G. Dudley (2005). "Methylphenidate improves prefrontal cortical cognitive function through alpha2 adrenoceptor and dopamine D1 receptor actions: Relevance to therapeutic effects in Attention Deficit Hyperactivity Disorder." Behav Brain Funct 1(1): 2.

Amsten, A. F. and B. M. Li (2005). "Neurobiology of executive functions: catecholamine influences on prefrontal cortical functions." Biol Psychiatry 57(11): 1377-1384.

Amsten, A. F. T., Vijayragavan S., Wang M., Gamo N.J., Paspalas C.D. (2009). Dopamine's Influence on Prefrontal Cortical Cognition: Actions and Circuits in Behaving Primates. Dopamine Handbook. I. S. D. Iversen L.L., Dunnett S.B., Bjorklund A. New York, NY, Oxford University Press: 230-248.

Eglen, R. M. (2007). "Assessing GPCR activation using protein complementation: a novel technique for HTS." Biochem Soc Trans 35(Pt 4): 746-748.

Gjoni, T. and S. Urwyler (2008). "Receptor activation involving positive allosteric modulation, unlike full agonism,

does not result in GABAB receptor desensitization." Neuropharmacology 55(8): 1293-1299.

Goldman-Rakic, P. S., S. A. Castner, T. H. Svensson, L. J. Siever and G. V. Williams (2004). "Targeting the dopamine D1 receptor in schizophrenia: insights for cognitive dysfunction." Psychopharmacology (Berl) 174(1): 3-16.

May, L. T., K. Leach, P. M. Sexton and A. Christopoulos (2007). "Allosteric modulation of G protein-coupled receptors." Annu Rev Pharmacol Toxicol 47: 1-51.

Sibley, D. R. and F. J. Monsma, Jr. (1992). "Molecular biology of dopamine receptors." Trends Pharmacol Sci 13(2): 61-69.

Tamminga, C. A. (2006). "The neurobiology of cognition in schizophrenia." J Clin Psychiatry 67(9): e11.

Vijayraghavan, S., M. Wang, S. G. Birnbaum, G. V. Williams and A. F. Arnsten (2007). "Inverted-U dopamine D1 receptor actions on prefrontal neurons engaged in working memory." Nat Neurosci 10(3): 376-384.

Conroy J.L., Free R.B. and Sibley D.R. (2015) Identification of G protein-biased agonists that fail to recruit beta-arrestin or promote internalization of the D1 dopamine receptor. ACS chemical neuroscience 6(4): 681-692.

Lewis M.A., Hunihan L., Watson J., Gentles R.G., Hu S., Huang Y., Bronson J., Macor J.E., Beno B.R., Ferrante M., Hendricson A., Knox R.J., Molski T.F., Kong Y., Cvijic M.E., Rockwell K.L., Weed M.R., Cacace A.M., Westphal R.S., Alt A. and Brown J.M. (2015) Discovery of D1 Dopamine Receptor Positive Allosteric Modulators: Characterization of Pharmacology and Identification of Residues that Regulate Species Selectivity. The Journal of pharmacology and experimental therapeutics 354(3): 340-349.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention. Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate.

## Claims

**1.** A compound of formula (I):

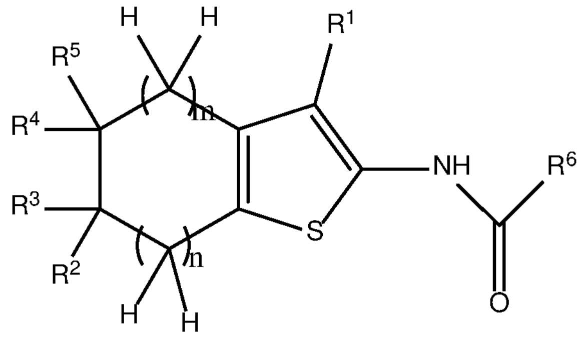

(I)

wherein $R^1$ is $-CONR^7R^8$,
$R^2$ is $C_1$-$C_6$ alkyl or $C_6$-$C_{10}$ aryl and $R^3$-$R^5$ are all H,
m and n are 1,
$R^6$ is 2-pyridyl, 3-pyridyl, or 2-quinolinyl, and

$R^7$ and $R^8$ are independently H or $C_1$-$C_6$ alkyl,
wherein $R^6$ is optionally substituted with one or more groups selected from the group consisting of halo, $C_1$-$C_6$ alkyl, and $C_1$-$C_6$ alkoxy,
or a pharmaceutically acceptable salt thereof.

**2.** The compound or salt of claim 1, wherein $R^6$ is 2-pyridyl or 3-pyridyl, each of which is optionally substituted with one or more groups selected from the group consisting of halo, $C_1$-$C_6$ alkyl, and $C_1$-$C_6$ alkoxy.

preferably wherein the compound is selected from the group consisting of:

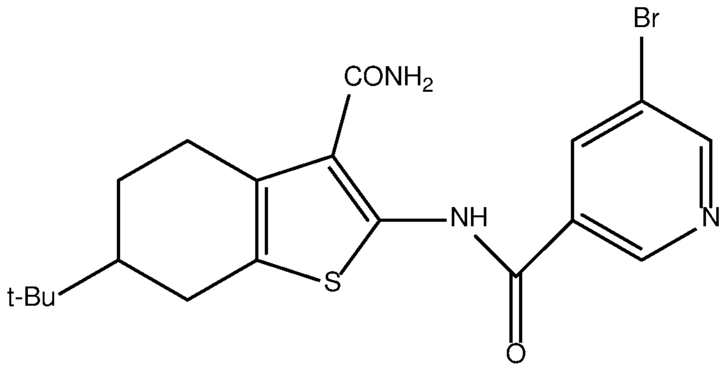

,

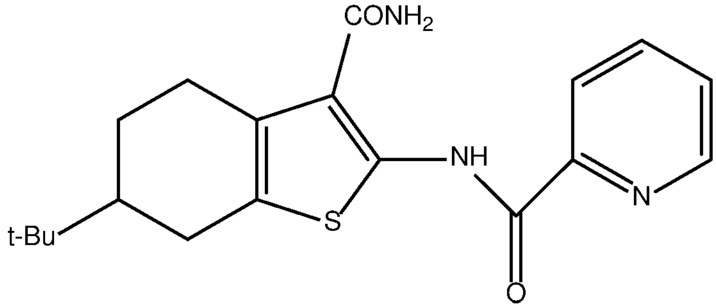

,

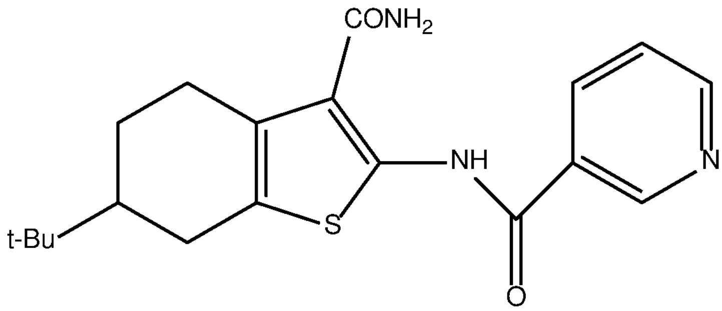

,

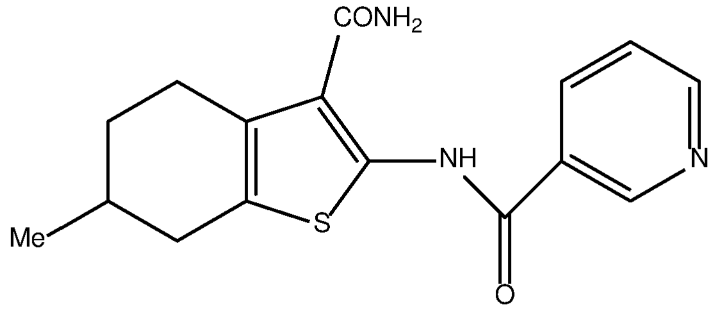

,

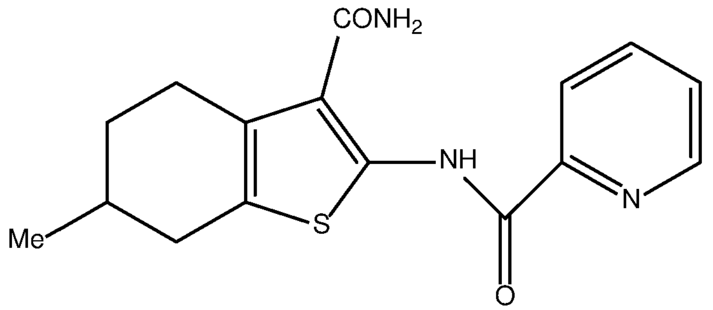

,

or wherein $R^6$ is 2-quinolinyl, preferably the compound is:

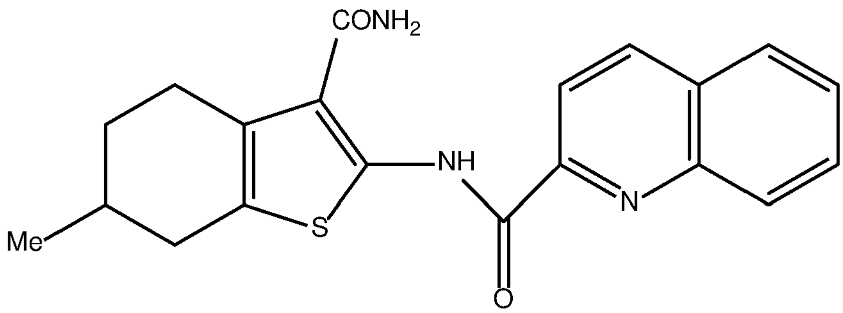

or

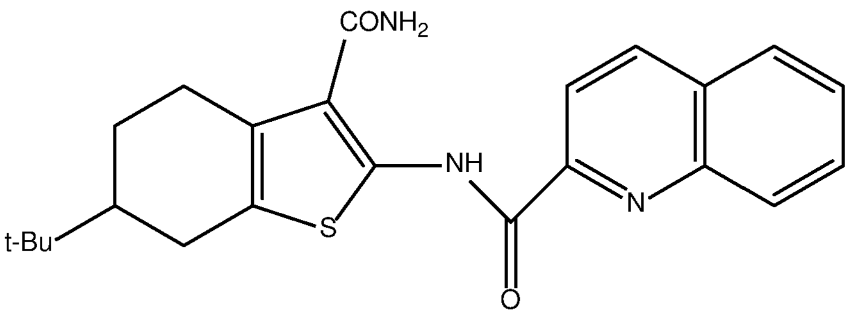

.

**3.** A pharmaceutical composition comprising a compound or salt of claim 1 or claim 2 and a pharmaceutically acceptable carrier.

**4.** A compound of formula (I):

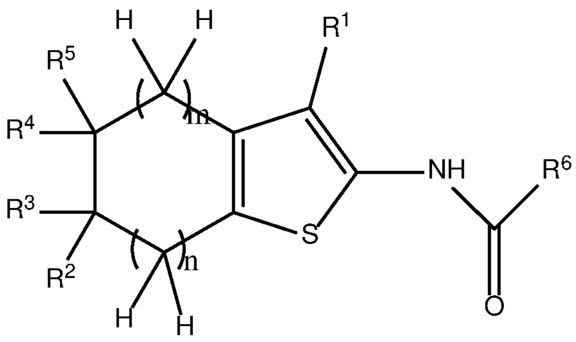

(I)

wherein $R^1$ is -CN or -CONR$^7$R$^8$,
$R^2$ is $C_1$-$C_6$ alkyl or $C_6$-$C_{10}$ aryl and $R^3$-$R^5$ are all H or wherein $R^4$ is $C_1$-$C_6$ alkyl or $C_6$-$C_{10}$ aryl and $R^2$, $R^3$, and $R^5$ are all H,
m and n are independently 1 or 2,
$R^6$ is 2-pyridyl, 3-pyridyl, 4-pyridyl, or 2-quinolinyl, and
$R^7$ and $R^8$ are independently H or $C_1$-$C_6$ alkyl,
wherein $R^6$ is optionally substituted with one or more groups selected from the group consisting of halo, $C_1$-$C_6$ alkyl, and $C_1$-$C_6$ alkoxy,
or a pharmaceutically acceptable salt thereof, and/or
a compound of formula (II):

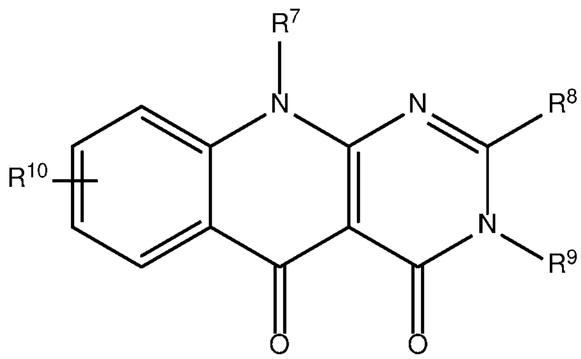

(II)

wherein $R^7$ is $C_1$-$C_6$ alkyl,
$R^8$ is $C_1$-$C_6$ alkyl, $C_3$-$C_8$-cycloalkyl, or $C_3$-$C_8$-cycloalkyl-$C_1$-$C_6$-alkylene,
$R^9$ is phenyl, optionally substituted with one or more substituents selected from the group consisting of halo, $C_1$-$C_6$ alkyl, or $C_1$-$C_6$ alkoxy, and
$R^{10}$ is H, halo, or $C_1$-$C_6$ alkoxy,
or a pharmaceutically acceptable salt thereof,
for use in treating or preventing a disease or disorder responsive to activation of a D1 dopamine receptor agonist in a mammal in need thereof.

**5.** The compound for use according to claim 4, wherein the compound is of formula (II), preferably wherein $R^7$ is methyl and/or
wherein $R^{10}$ is H, and more preferably wherein $R^8$ is $C_1$-$C_6$ alkyl, $C_3$-$C_8$ cycloalkyl, or $C_3$-$C_8$ cycloalkyl-$C_1$-$C_6$ alkylenyl.

**6.** The compound for use according to claim 5, wherein the compound is selected from the group consisting of:

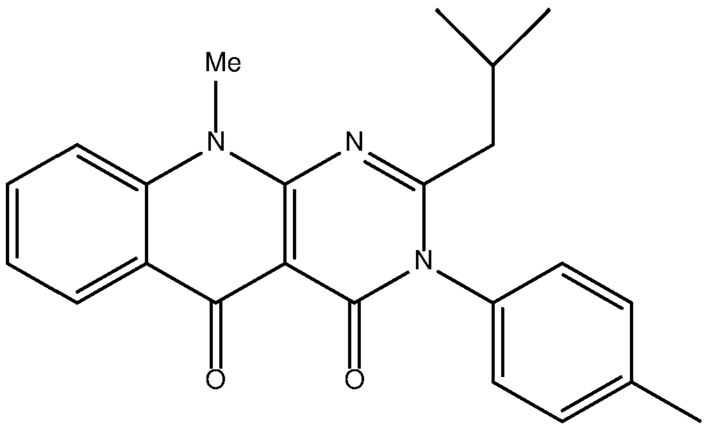

,

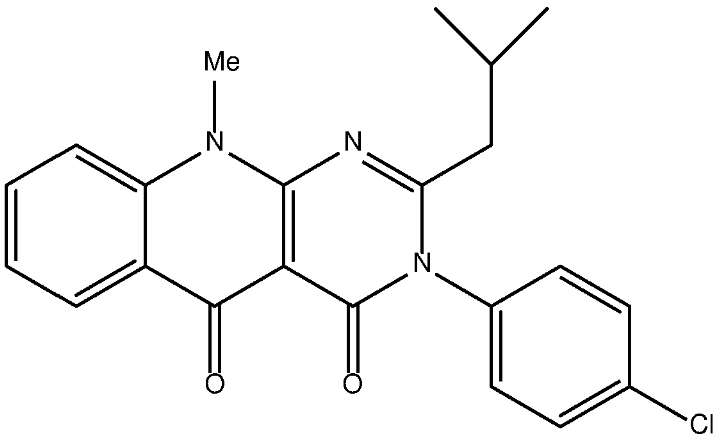

,

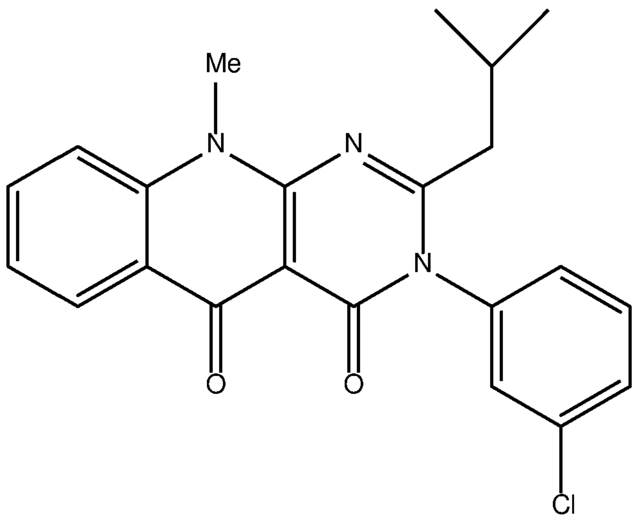

,

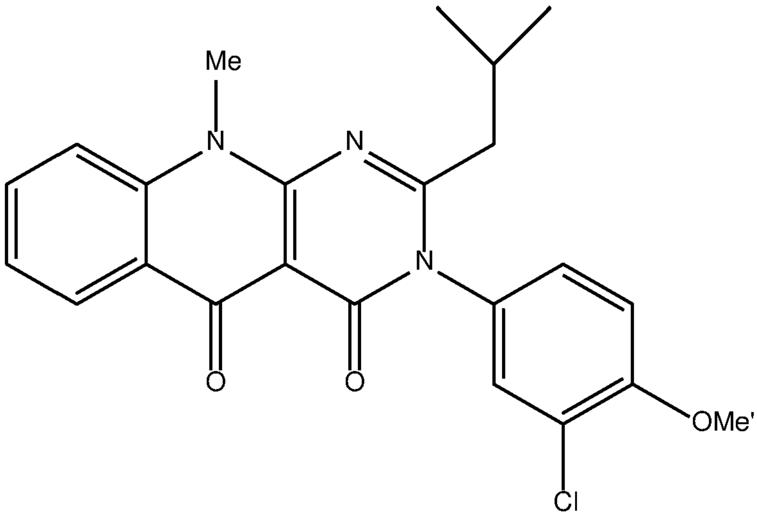

,

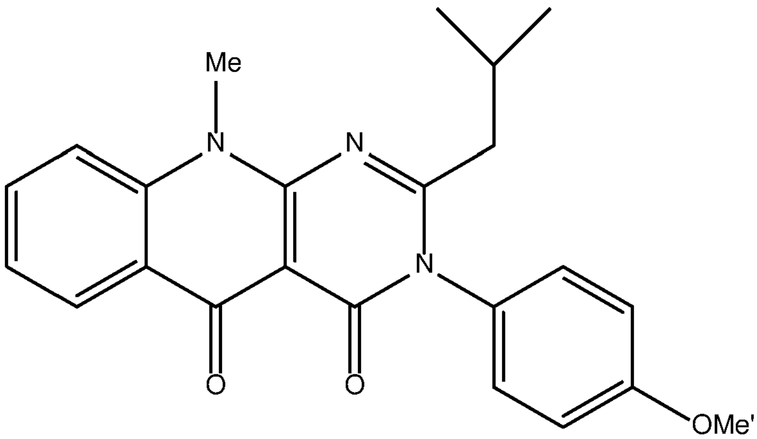

, ,

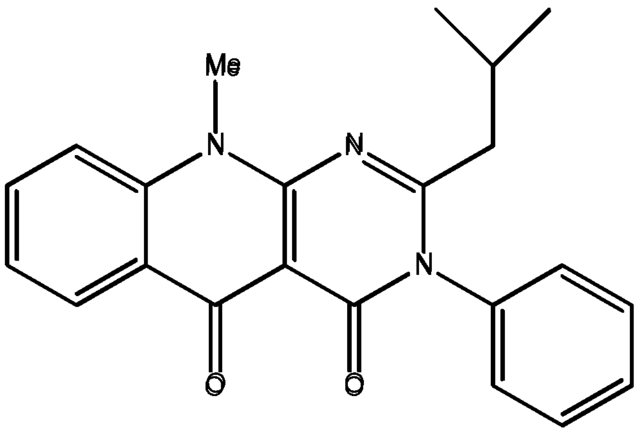

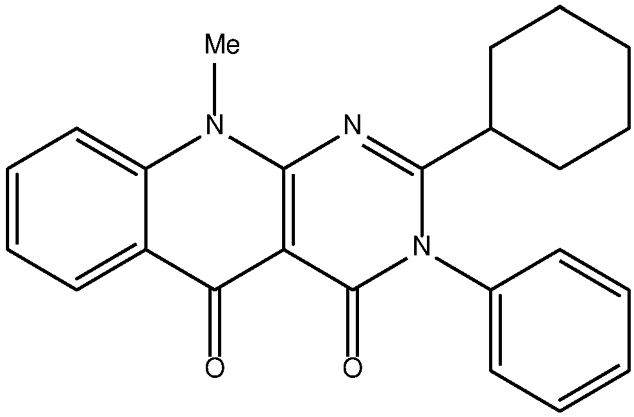,

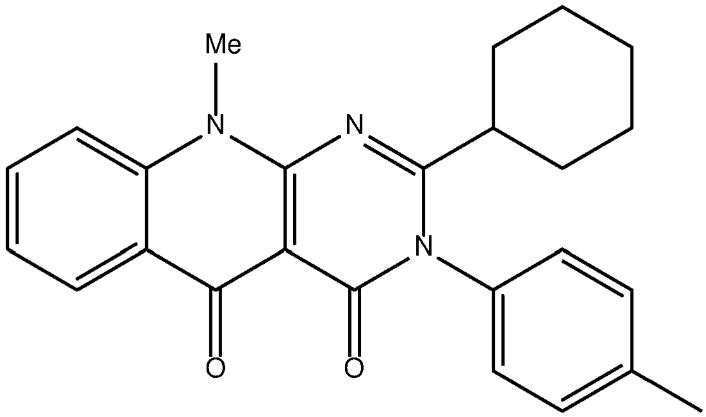,

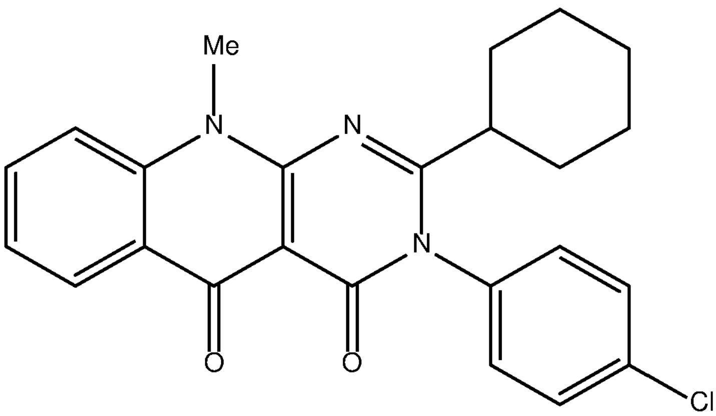,

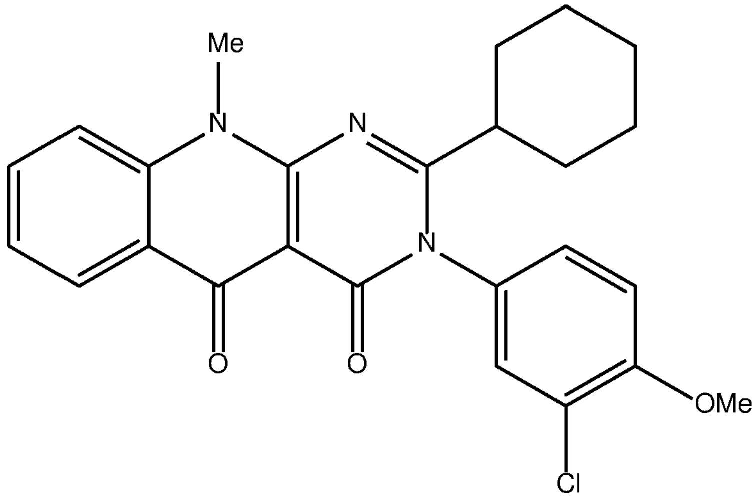

,

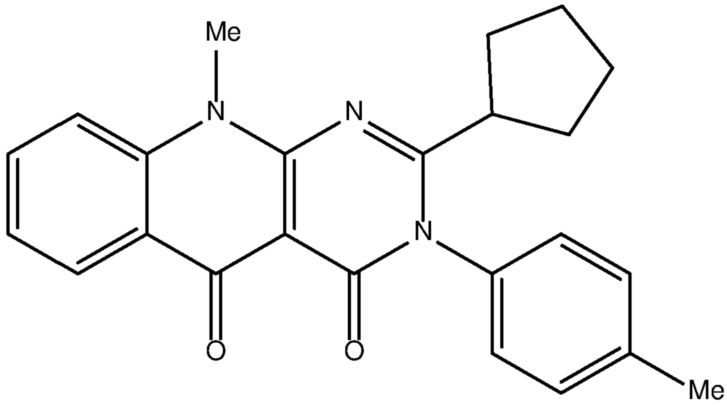

,

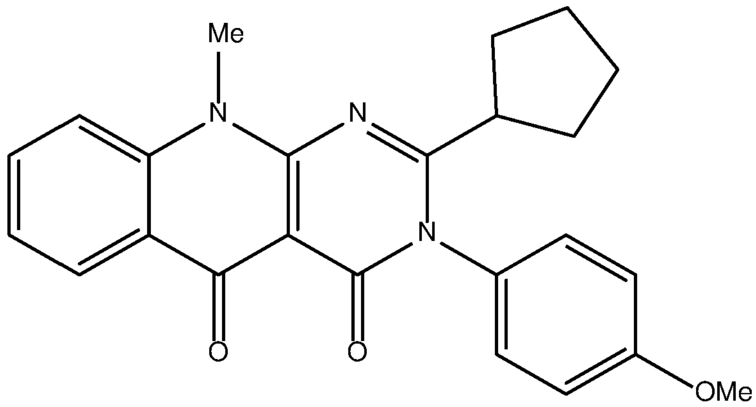

,

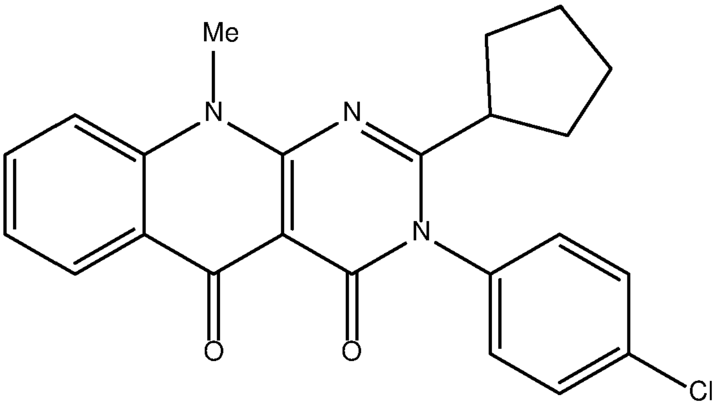
,

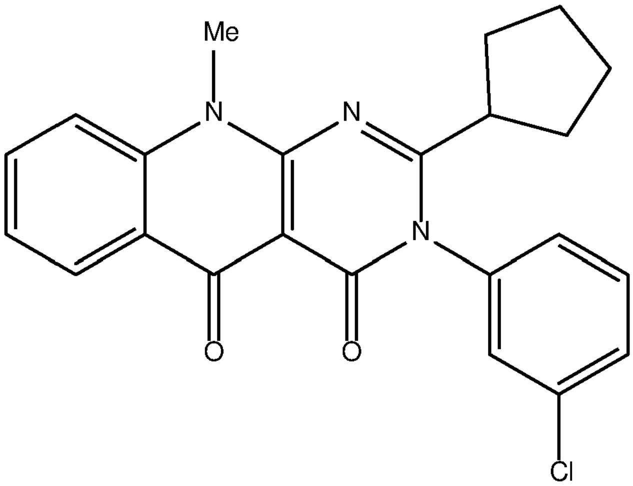
,

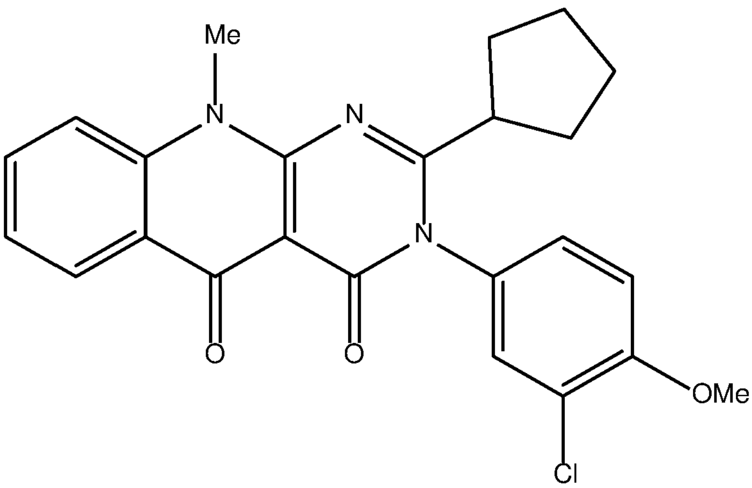
,

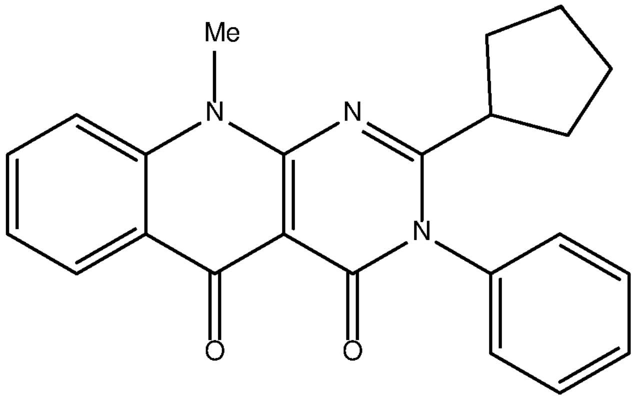

,

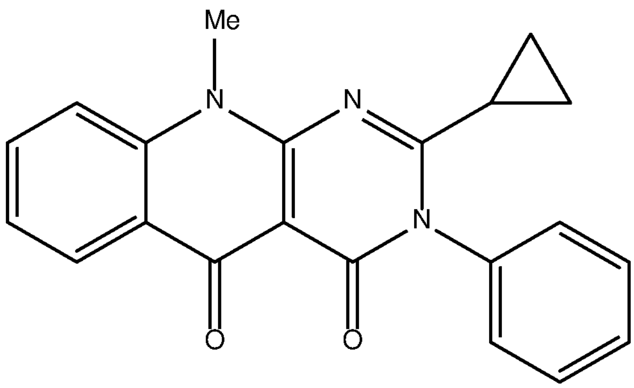

,

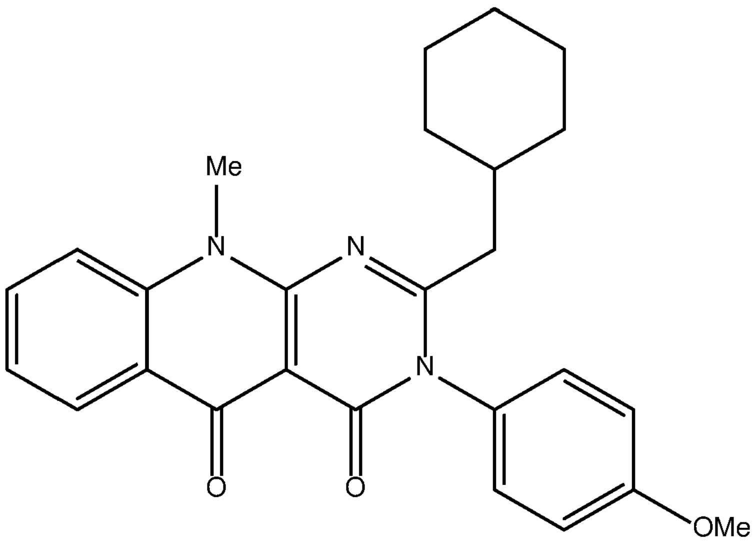

,

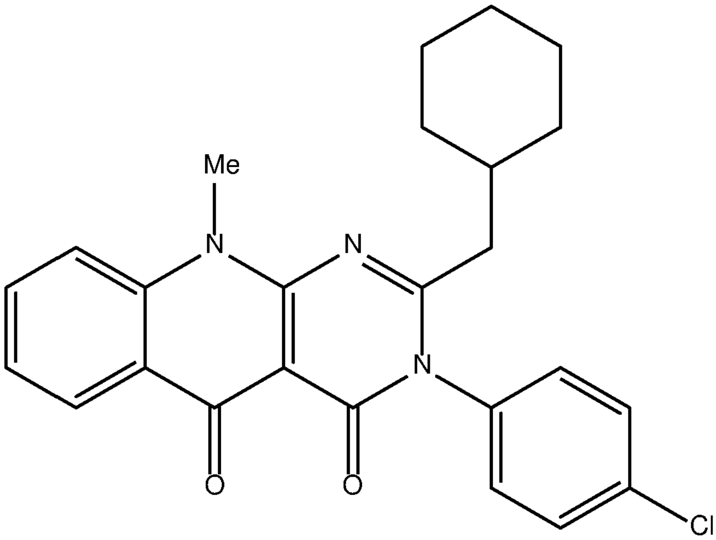

,

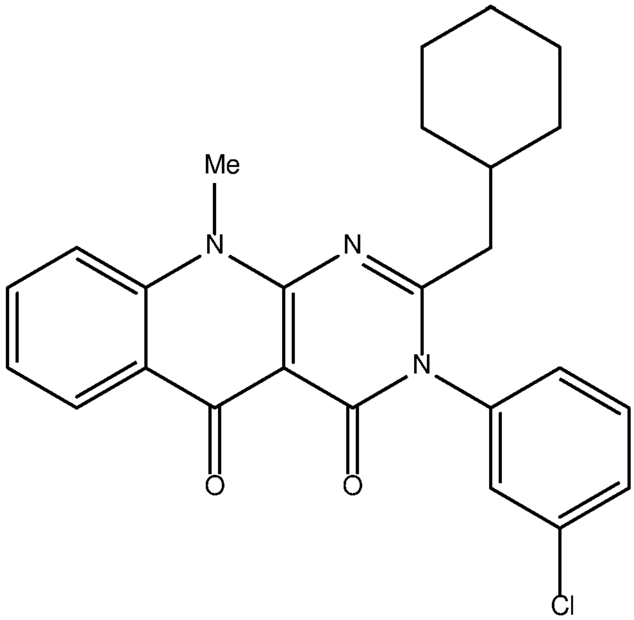

,

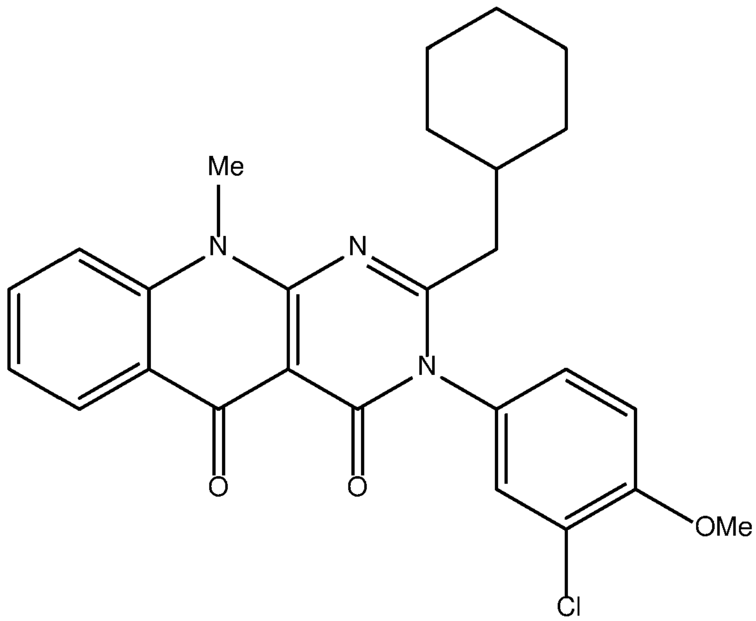

,

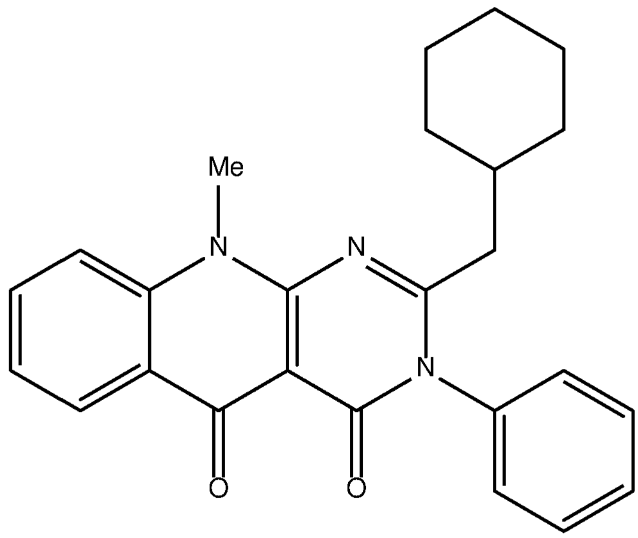

,

and

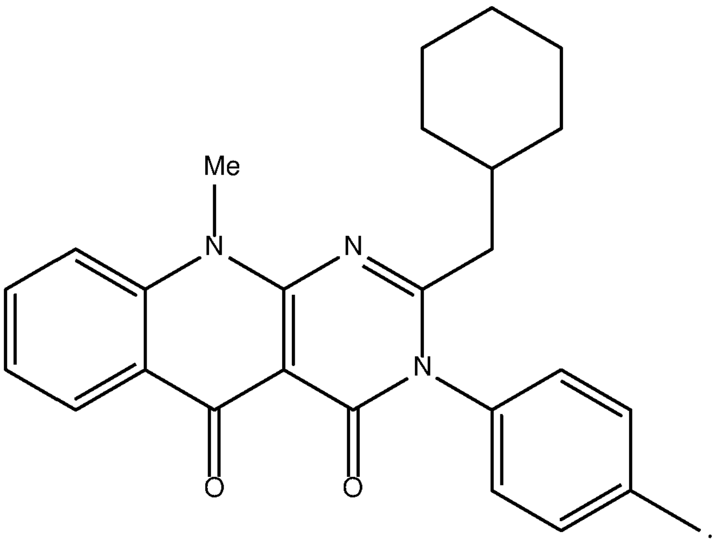

7. The compound for use according to any one of claims 4-6, wherein the dopamine D1 receptor agonist is dopamine, and/or wherein the disease or disorder involves an impairment of mood, cognition, memory, movement or motor skills, or a combination thereof.

8. The compound for use according to any one of claims 4-7, wherein the disease or disorder is Alzheimer's Disease, schizophrenia, Parkinson's disease, a dyskinesia, or Huntington's disease.

9. The compound for use according to any one of claims 4-8, wherein the use further comprises dopamine D1 receptor agonist other than dopamine, preferably wherein the dopamine D1 receptor agonist is apomorphine, fenoldopam, SKF38393, SKF77434, or L-DOPA.

10. A compound of formula (III):

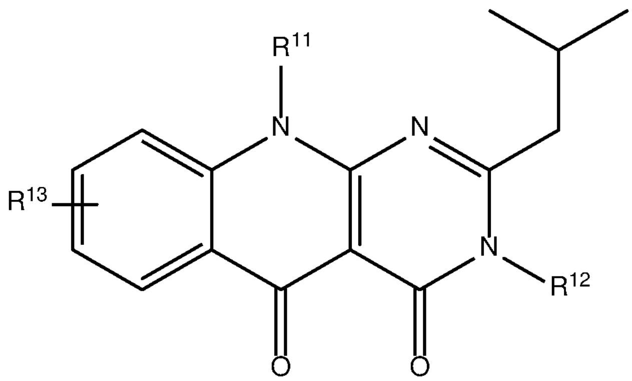

(III)

wherein $R^{11}$ is $C_1$-$C_6$ alkyl,
$R^{12}$ is phenyl, optionally substituted with one or more substituents selected from the group consisting of halo, $C_1$-$C_6$ alkyl, or $C_1$-$C_6$ alkoxy, and
$R^{13}$ is H, halo, or $C_1$-$C_6$ alkoxy,
or a pharmaceutically acceptable salt thereof.

11. A pharmaceutical composition comprising a compound of claim 10 and a pharmaceutically acceptable carrier.

12. The compound or salt of claim 1, wherein $R^6$ is 2-pyridyl or 3-pyridyl, each of which is substituted with a methyl group or a methoxy group.

**13.** A pharmaceutical composition comprising a compound of claim 12 and a pharmaceutically acceptable carrier.

**14.** The compound or salt of claim 1, for use in treating or preventing a disease or disorder responsive to activation of a D1 dopamine receptor agonist in a mammal in need thereof.

**Patentansprüche**

**1.** Verbindung gemäß Formel (I):

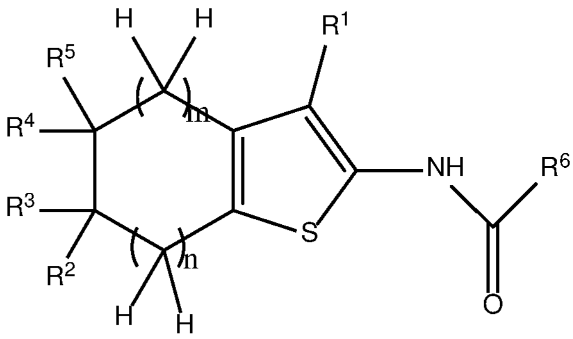

(I)

worin $R^1$ -$CONR^7R^8$ ist,
$R^2$ $C_1$-$C_6$-Alkyl oder $C_6$-$C_{10}$-Aryl ist und $R^3$-$R^5$ jeweils H sind,
m und n 1 sind,
$R^6$ 2-Pyridyl, 3-Pyridyl oder 2-Chinolinyl ist und
$R^7$ und $R^8$ unabhängig jeweils H oder $C_1$-$C_6$-Alkyl sind,
wobei $R^6$ optional mit einer oder mehreren Gruppen substituiert ist, die ausgewählt sind aus der Gruppe bestehend aus Halogen, $C_1$-$C_6$-Alkyl und $C_1$-$C_6$-Alkoxy,
oder ein pharmazeutisch unbedenkliches Salz davon.

**2.** Verbindung oder Salz nach Anspruch 1, wobei $R^6$ 2-Pyridyl oder 3-Pyridyl ist, das jeweils optional mit einer oder mehreren Gruppen substituiert ist, die ausgewählt sind aus der Gruppe bestehend aus Halogen, $C_1$-$C_6$-Alkyl und $C_1$-$C_6$-Alkoxy.

wobei die Verbindung vorzugsweise ausgewählt ist aus der Gruppe bestehend aus:

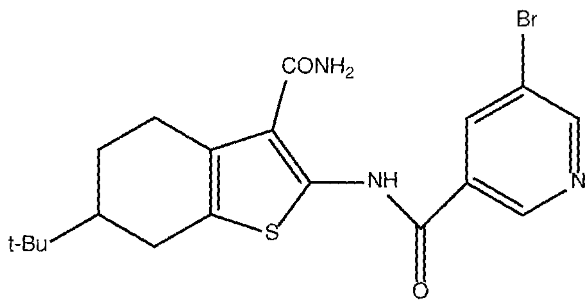

,

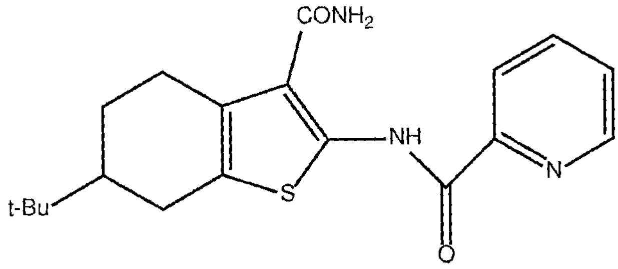

,

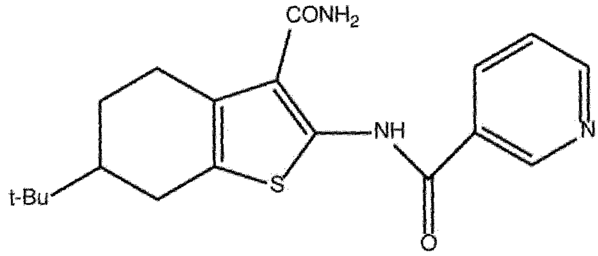

,

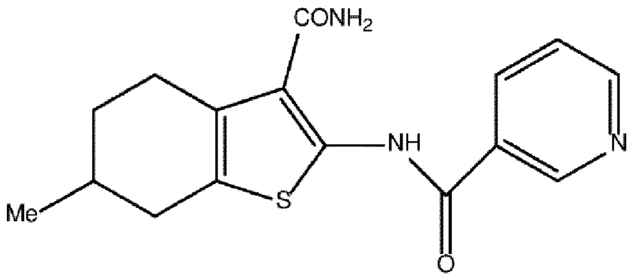

,

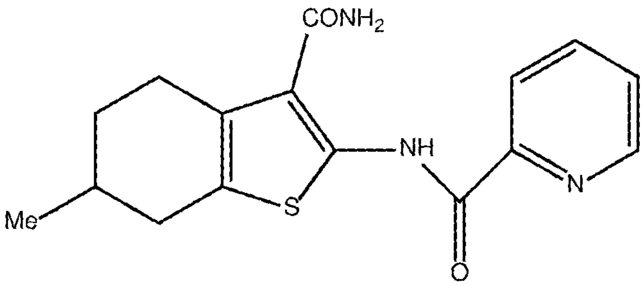

,

oder wobei $R^6$ 2-Chinolinyl ist, wobei die Verbindung vorzugsweise folgendes ist:

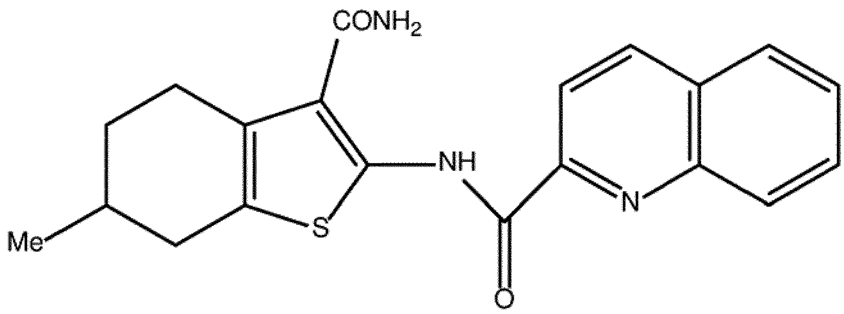

oder

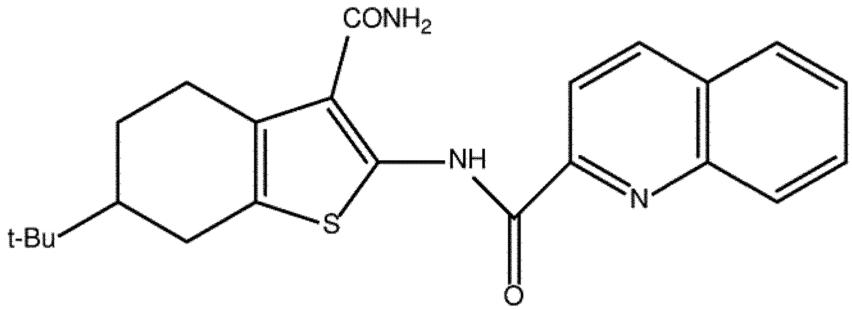

3. Pharmazeutische Zusammensetzung, die eine Verbindung oder ein Salz nach Anspruch 1 oder Anspruch 2 und einen pharmazeutisch akzeptablen Träger umfasst.

4. Verbindung gemäß Formel (I):

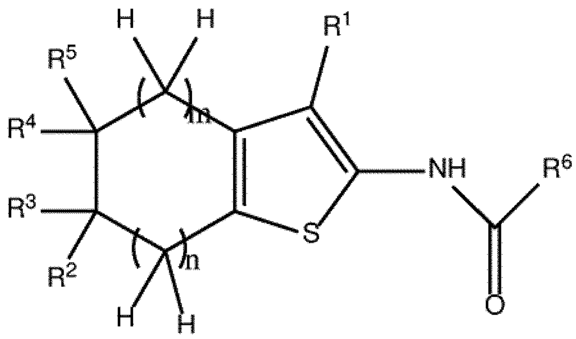

(I)

worin $R^1$ -CN oder -CONR$^7$R$^8$ ist,
$R^2$ $C_1$-$C_6$-Alkyl oder $C_6$-$C_{10}$-Aryl ist und $R^3$-$R^5$ jeweils H sind, oder worin R4 $C_1$-$C_6$-Alkyl oder $C_6$-$C_{10}$-Aryl ist und $R^2$, $R^3$ und $R^5$ jeweils H sind,
m und n unabhängig 1 oder 2 sind,
$R^6$ 2-Pyridyl, 3-Pyridyl, 4-Pyridyl oder 2-Chinolinyl ist und
$R^7$ und $R^8$ unabhängig jeweils H oder $C_1$-$C_6$-Alkyl sind,
wobei $R^6$ optional mit einer oder mehreren Gruppen substituiert ist, die ausgewählt sind aus der Gruppe bestehend aus Halogen, $C_1$-$C_6$-Alkyl und $C_1$-$C_6$-Alkoxy,
oder ein pharmazeutisch akzeptables Salz davon, und/oder
eine Verbindung gemäß Formel (II):

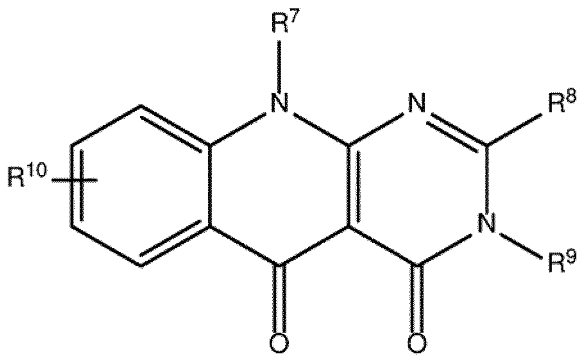

(II)

worin $R^7$ $C_1$-$C_6$-Alkyl ist,

$R^8$ $C_1$-$C_6$-Alkyl, $C_3$-$C_8$-Cycloalkyl oder $C_3$-$C_8$-Cycloalkyl-$C_1$-$C_6$-alkylen ist,
$R^9$ Phenyl ist, optional substituiert mit einem oder mehreren Substituenten, die ausgewählt sind aus der Gruppe bestehend aus Halogen, $C_1$-$C_6$-Alkyl oder $C_1$-$C_6$-Alkoxy, und
$R^{10}$ H, Halogen oder $C_1$-$C_6$-Alkoxy ist,
oder ein pharmazeutisch akzeptables Salz davon,
zur Verwendung bei der Behandlung oder Vorbeugung einer Krankheit oder Störung, die auf eine Aktivierung eines D1-Dopaminre-zeptor-Agonisten in einem Säugetier, das dessen bedarf, anspricht.

**5.** Verbindung zur Verwendung gemäß Anspruch 4, wobei die Verbindung die Formel (II) hat, worin vorzugsweise $R^7$ Methyl ist, und/oder worin $R^{10}$ H ist und worin $R^8$ mehr bevorzugt $C_1$-$C_6$-Alkyl, $C_3$-$C_8$-Cycloalkyl oder $C_3$-$C_8$-Cycloalkyl-$C_1$-$C_6$-alkylenyl ist.

**6.** Verbindung zur Verwendung gemäß Anspruch 5, wobei die Verbindung ausgewählt ist aus der Gruppe bestehend aus:

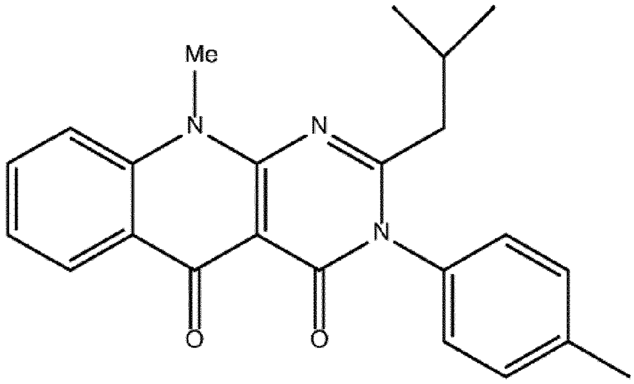

,

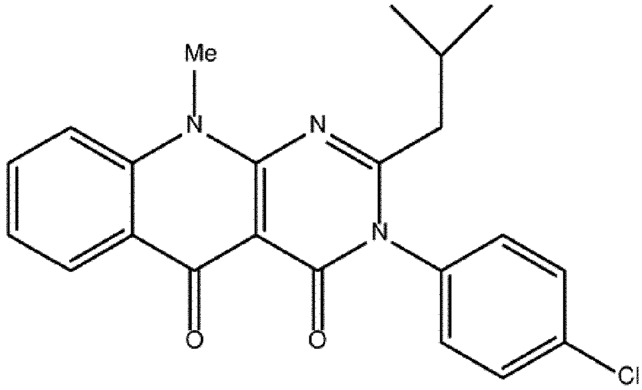

,

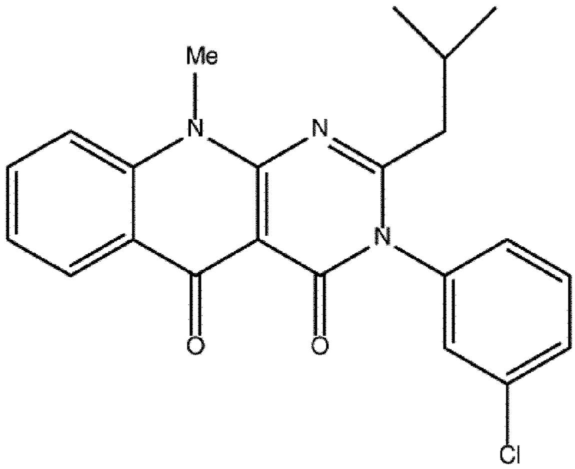

,

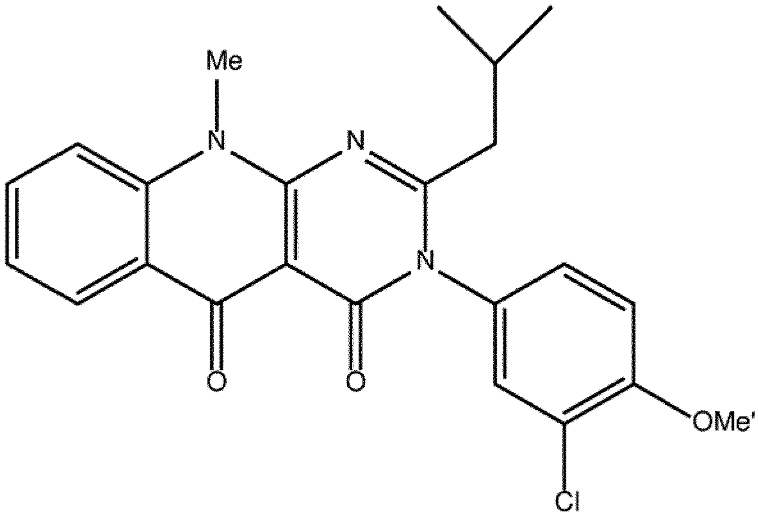

,

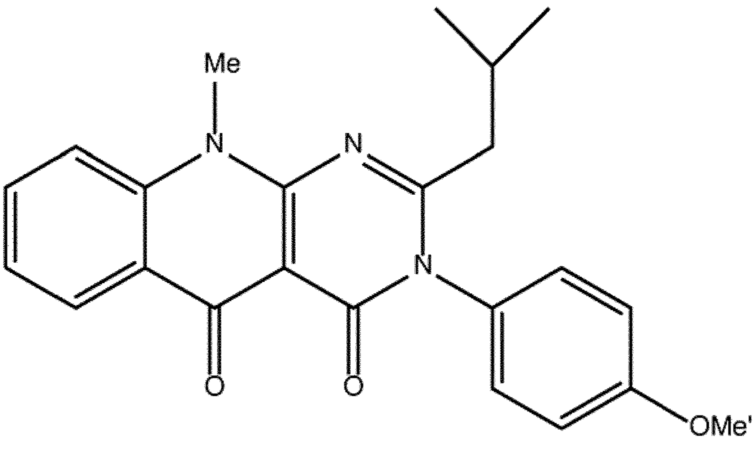

, ,

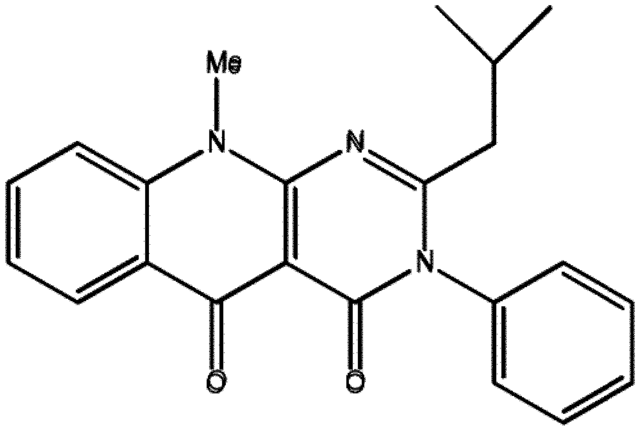

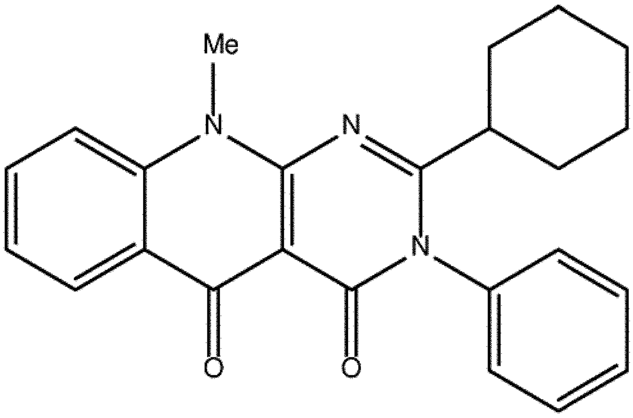

,

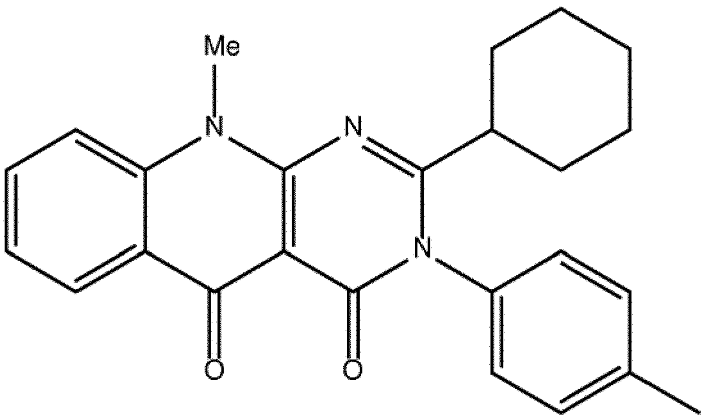

,

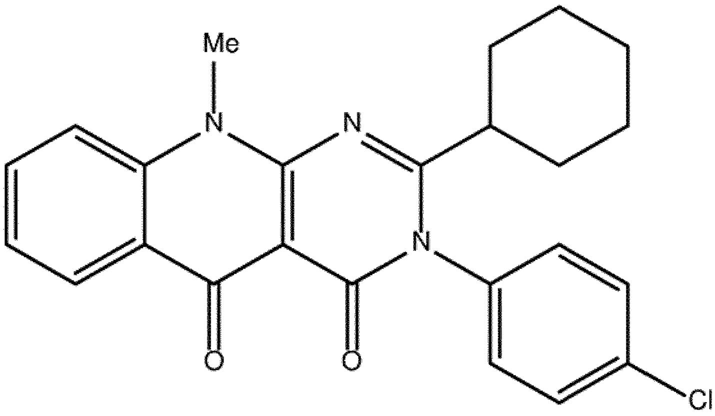

,

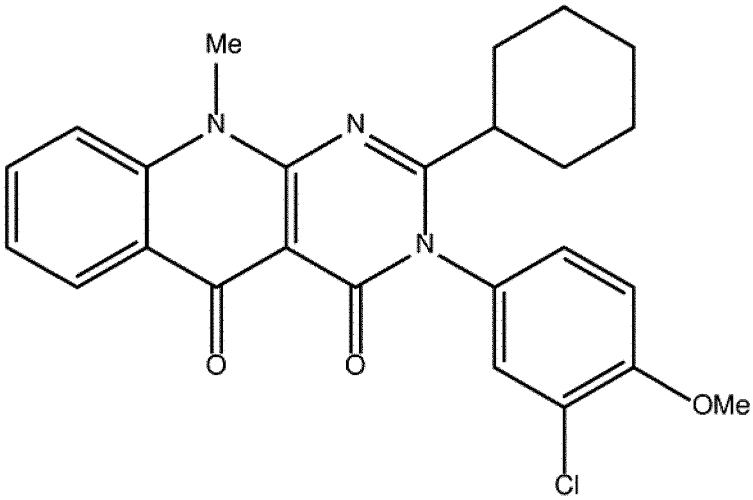

,

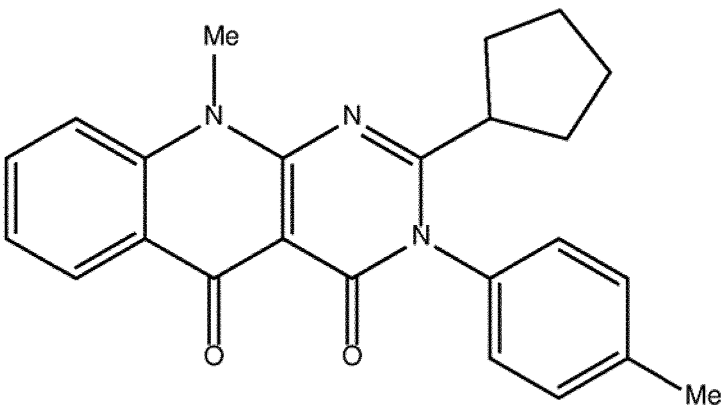

,

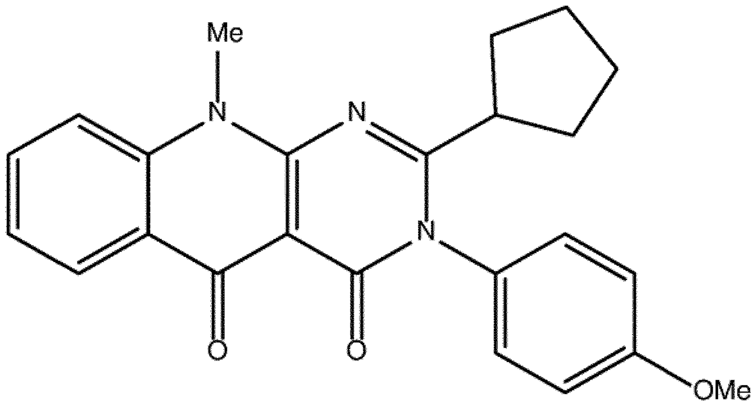
,

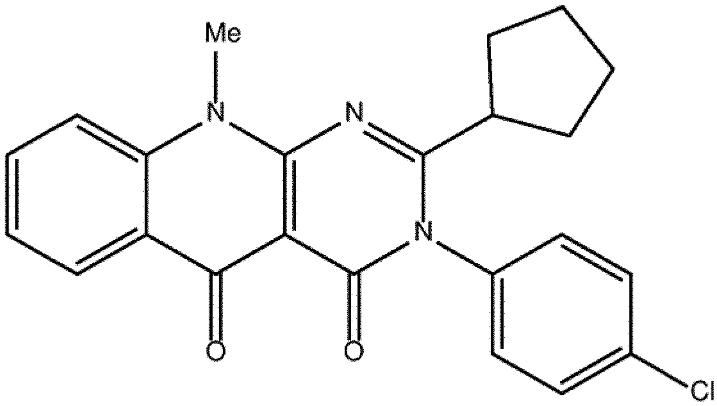
,

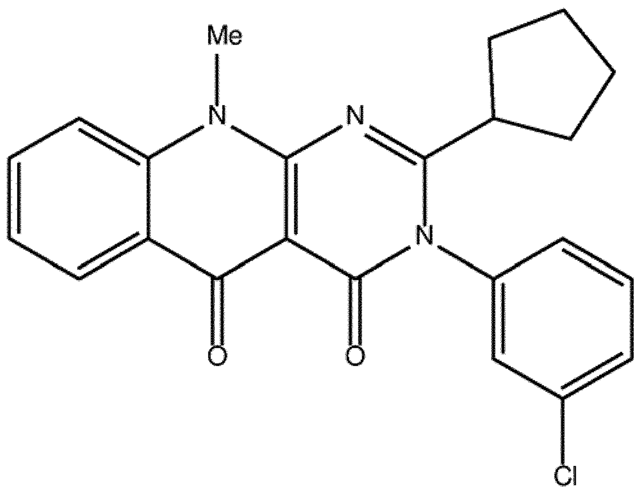
,

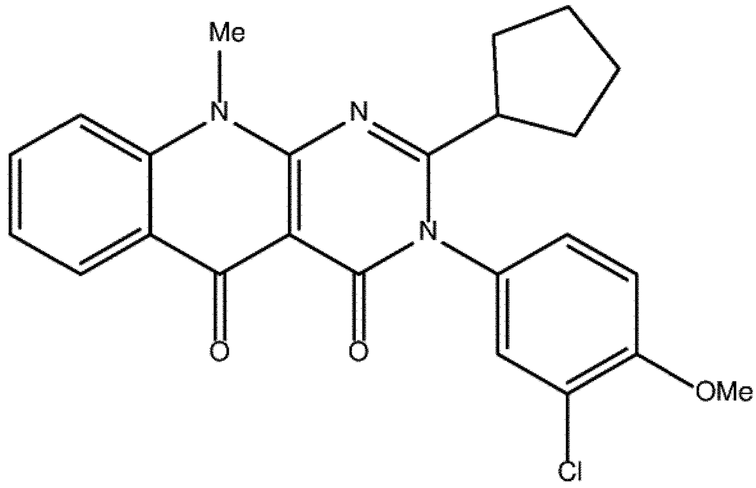

,

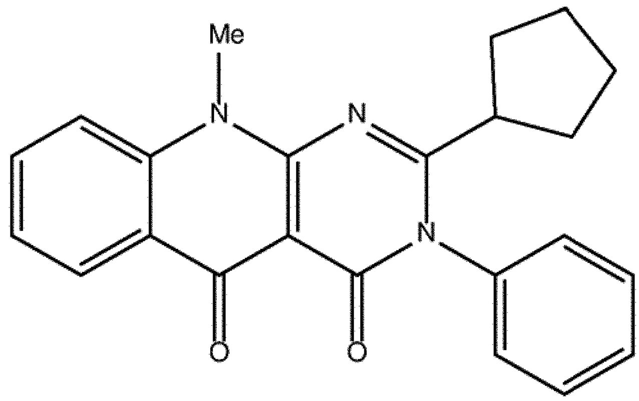

,

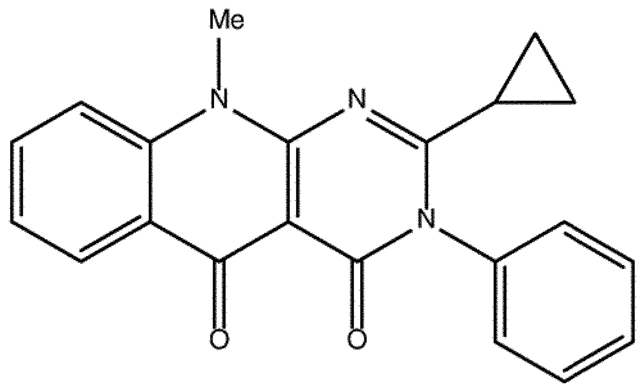

,

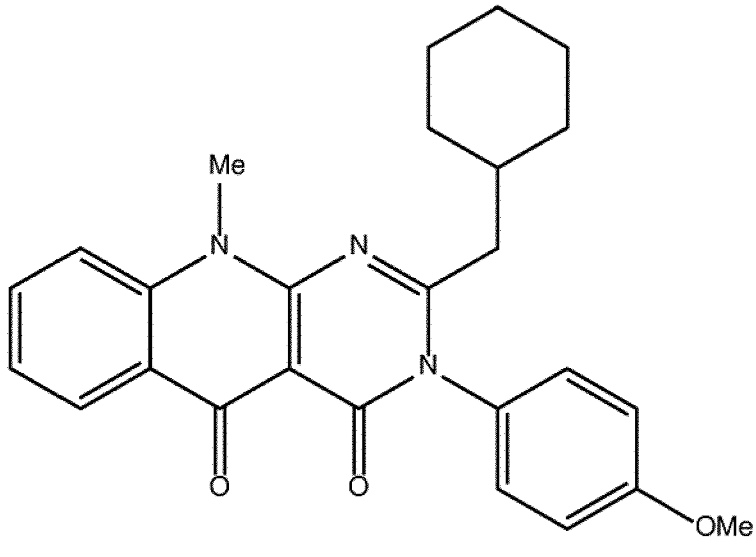

,

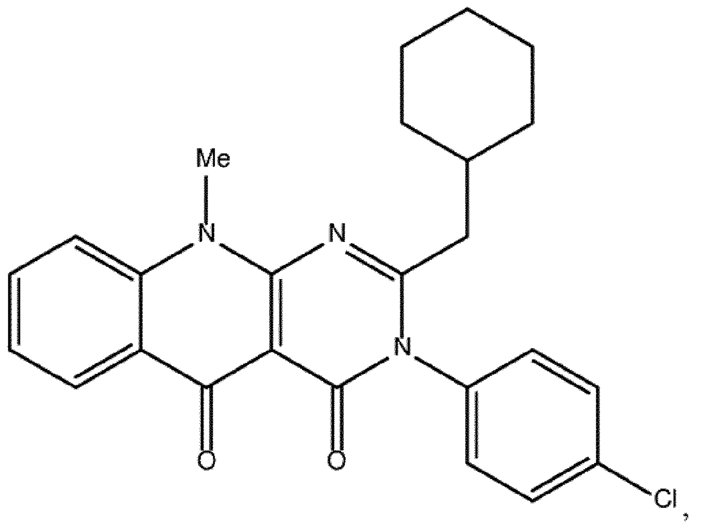

,

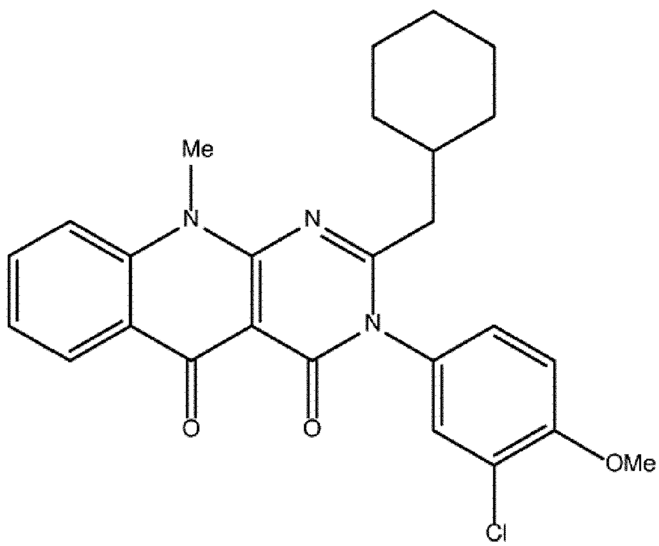

,

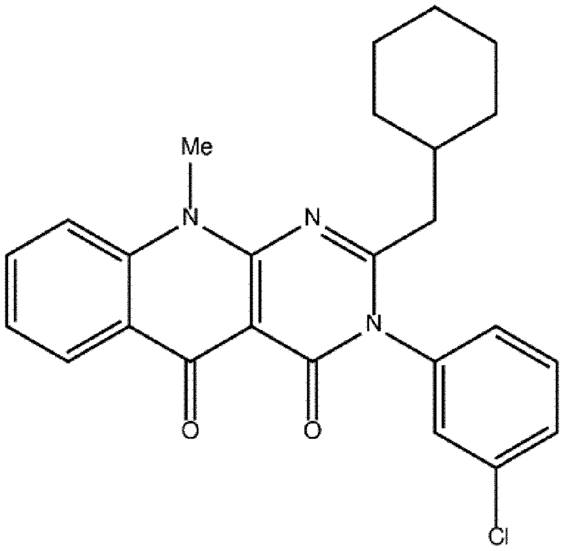

,

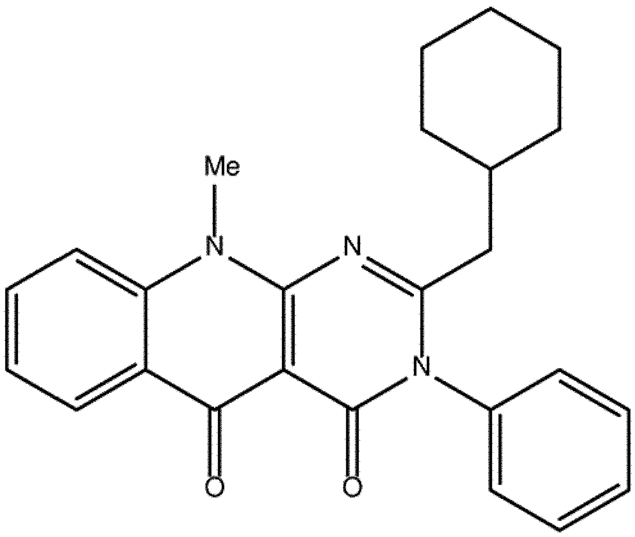

und

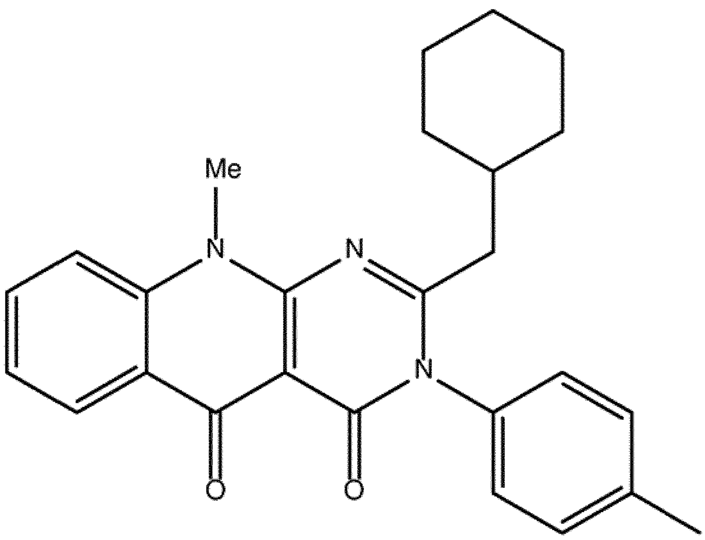

.

7. Verbindung zur Verwendung gemäß einem der Ansprüche 4-6, wobei der D1-Dopaminrezeptor-Agonist Dopamin ist, und/oder wobei die Krankheit oder Störung eine Verschlechterung von Stimmungslage, Wahrnehmungsvermögen, Erinnerungsvermögen, Bewegungsfähigkeit oder motorischen Fähigkeiten oder eine Kombination davon einschließt.

8. Verbindung zur Verwendung gemäß einem der Ansprüche 4-7, wobei die Krankheit oder Störung Alzheimer-Krankheit, Schizophrenie, Parkinson-Krankheit, Dyskinesie oder Huntington-Krankheit ist.

9. Verbindung zur Verwendung gemäß einem der Ansprüche 4-8, wobei die Verwendung ferner einen D1-Dopaminrezeptor-Agonisten umfasst, bei dem es sich nicht um Dopamin handelt, wobei der D1-Dopaminre-zeptor-Agonist vorzugsweise Apomorphin, Fenoldopam, SKF38393, SKF77434 oder L-DOPA ist.

10. Verbindung der Formel (III):

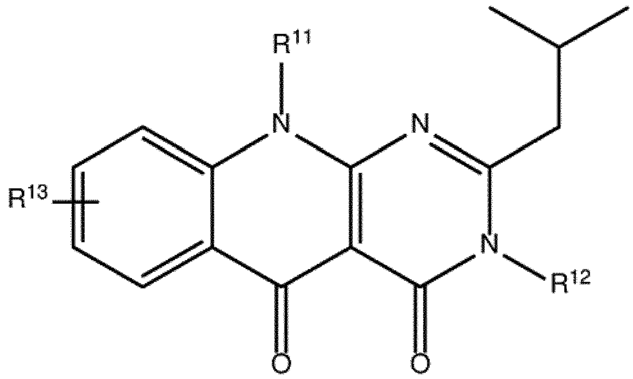

(III)

wobei $R^{11}$ $C_1$-$C_6$-Alkyl ist,
$R^{12}$ Phenyl ist, optional substituiert mit einem oder mehreren Substituenten, die ausgewählt sind aus der Gruppe bestehend aus Halogen, $C_1$-$C_6$-Alkyl oder $C_1$-$C_6$-Alkoxy, und
$R^{13}$ H, Halogen oder $C_1$-$C_6$-Alkoxy ist,
oder ein pharmazeutisch akzeptables Salz davon.

11. Pharmazeutische Zusammensetzung, eine Verbindung nach Anspruch 10 und einen pharmazeutisch akzeptablen Träger umfassend.

12. Verbindung oder Salz nach Anspruch 1, wobei $R^6$ 2-Pyridyl oder 3-Pyridyl ist, das jeweils mit einer Methylgruppe oder einer Methoxygruppe substituiert ist.

13. Pharmazeutische Zusammensetzung, eine Verbindung nach Anspruch 12 und einen pharmazeutisch akzeptablen Träger umfassend.

14. Verbindung oder Salz nach Anspruch 1 zur Verwendung bei der Behandlung oder Vorbeugung einer Krankheit oder Störung, die auf eine Aktivierung eines D1-Dopaminrezeptor-Agonisten in einem Säugetier, das dessen bedarf, anspricht.

## Revendications

1. Composé de formule (I):

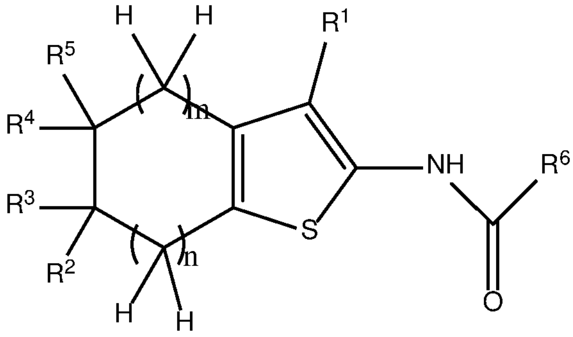

(I)

dans lequel $R^1$ est $-CONR^7R^8$,
$R^2$ est alkyle en $C_1$-$C_6$ ou aryle en $C_6$-$C_{10}$ et $R^3$ à $R^5$ sont tous H,
m et n sont 1,
$R^6$ est 2-pyridyle, 3-pyridyle ou 2-quinolinyle, et
$R^7$ et $R^8$ sont indépendamment H ou alkyle en $C_1$-$C_6$,
dans lequel $R^6$ est facultativement substitué par un ou plusieurs groupes choisis dans le groupe constitué d'halogéno, alkyle en $C_1$-$C_6$, et alcoxy en $C_1$-$C_6$,
ou sel pharmaceutiquement acceptable de celui-ci.

**2.** Composé ou sel selon la revendication 1, dans lequel $R^6$ est 2-pyridyle ou 3-pyridyle, dont chacun est facultativement substitué par un ou plusieurs groupes choisis dans le groupe constitué d'halogéno, alkyle en $C_1$-$C_6$ et alcoxy en $C_1$-$C_6$,

le composé étant de préférence choisi dans le groupe constitué de :

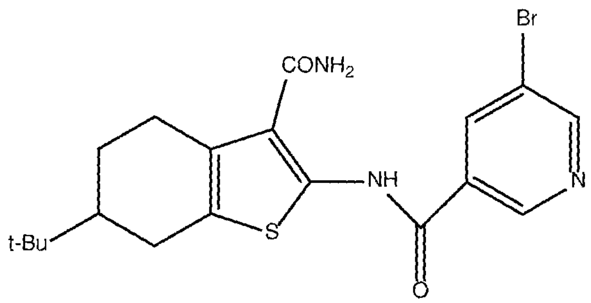

,

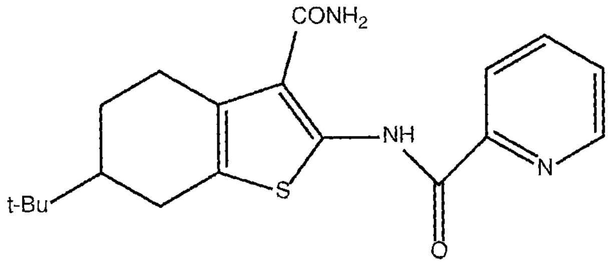

,

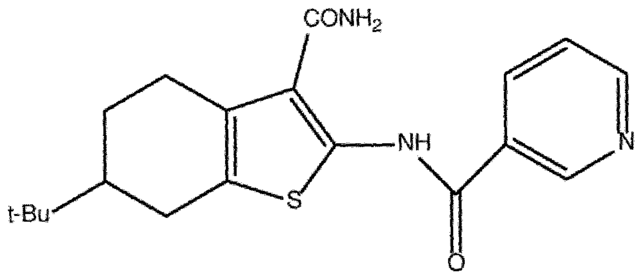

,

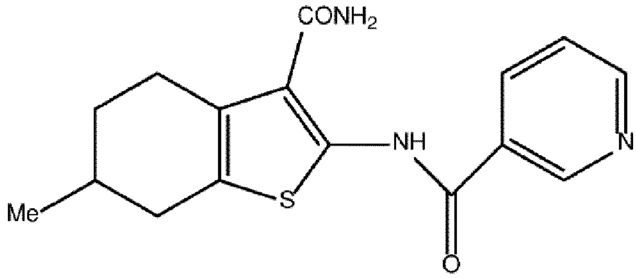

,

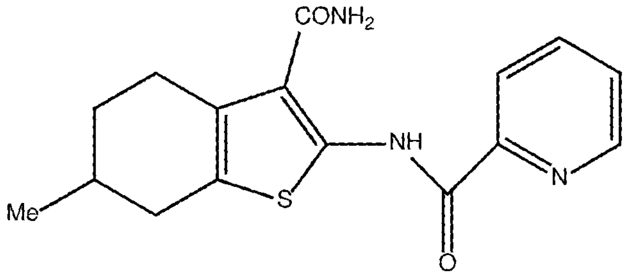

,

ou dans lequel $R^6$ est 2-quinolinyle, le composé étant de préférence :

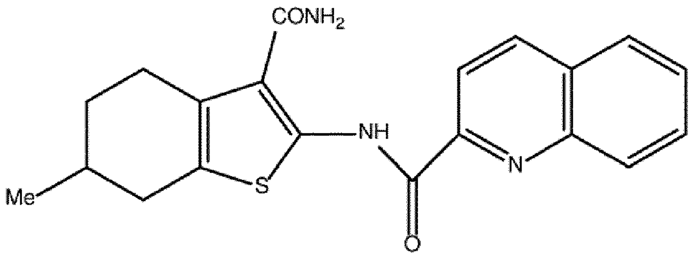

ou

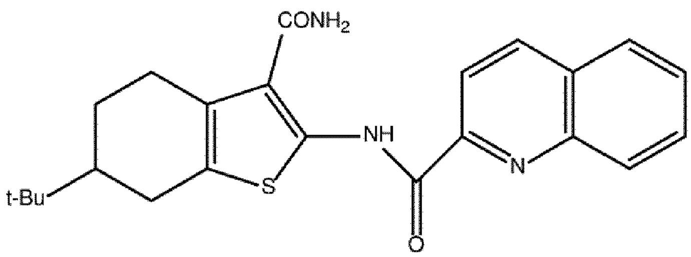

.

**3.** Composé pharmaceutique comprenant un composé ou un sel selon la revendication 1 ou la revendication 2 et un

véhicule pharmaceutiquement acceptable.

**4.** Composé de formule (I) :

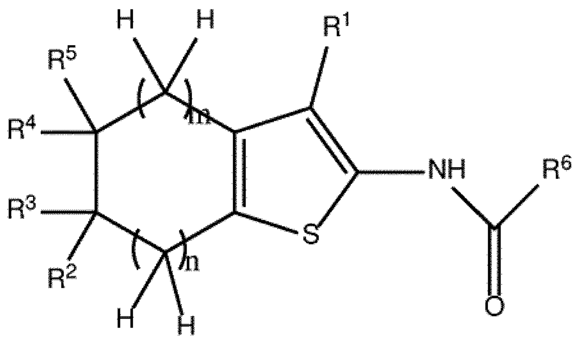

(I)

dans lequel $R^1$ est -CN ou -CONR$^7$R$^8$,
$R^2$ est alkyle en $C_1$-$C_6$ ou aryle en $C_6$-$C_{10}$ et $R^3$ à $R^5$ sont tous H ou dans lequel $R^4$ est alkyle en $C_1$-$C_6$ ou aryle en $C_6$-$C_{10}$ et $R^2$, $R^3$ et $R^5$ sont tous H,
m et n sont indépendamment 1 ou 2,
$R^6$ est 2-pyridyle, 3-pyridyle, 4-pyridyle ou 2-quinolinyle, et
$R^7$ et $R^8$ sont indépendamment H ou alkyle en $C_1$-$C_6$,
dans lequel $R^6$ est facultativement substitué par un ou plusieurs groupes choisis dans le groupe constitué d'halogéno, alkyle en $C_1$-$C_6$, et alcoxy en $C_1$-$C_6$,
ou sel pharmaceutiquement acceptable de celui-ci, et/ou
un composé de formule (II):

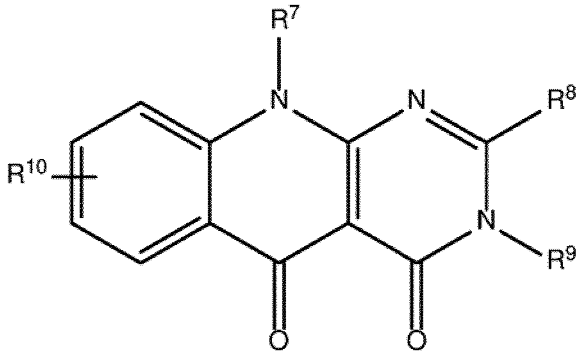

(II)

dans lequel $R^7$ est alkyle en $C_1$-$C_6$,
$R^8$ est alkyle en $C_1$-$C_6$, cycloalkyle en $C_3$-$C_8$ ou (cycloalkyle en $C_3$-$C_8$)-(alkylène en $C_1$-$C_6$),
$R^9$ est phényle, facultativement substitué par un ou plusieurs substituants choisis dans le groupe constitué d'halogéno, alkyle en $C_1$-$C_6$, ou alcoxy en $C_1$-$C_6$, et
$R^{10}$ est H, halogéno ou alcoxy en $C_1$-$C_6$,
ou sel pharmaceutiquement acceptable de celui-ci,
pour utilisation dans le traitement ou la prévention d'une maladie ou d'un trouble sensible à l'activation d'un agoniste des récepteurs dopaminergiques D1 chez un mammifère en ayant besoin.

**5.** Composé pour utilisation selon la revendication 4, le composé étant de formule (II), de préférence dans lequel $R^7$ est méthyle et/ou
dans lequel $R^{10}$ est H, et plus préférablement dans lequel $R^8$ est alkyle en $C_1$-$C_6$, cycloalkyle en $C_3$-$C_8$, ou (cycloalkyle en $C_3$-$C_8$)-(alky-lényle en $C_1$-$C_6$).

**6.** Composé pour utilisation selon la revendication 5, le composé étant choisi dans le groupe constitué de :

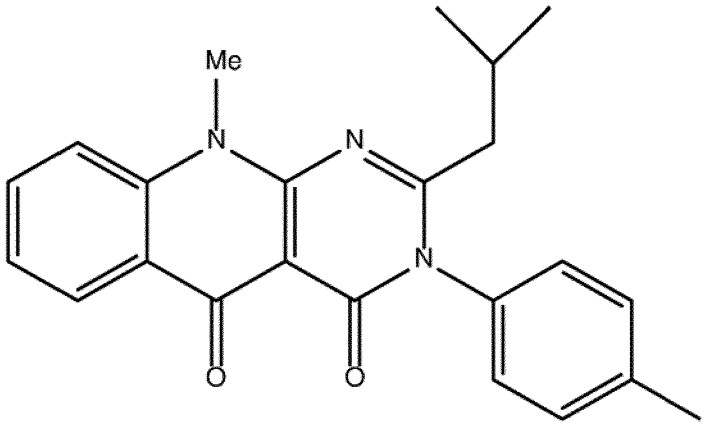

,

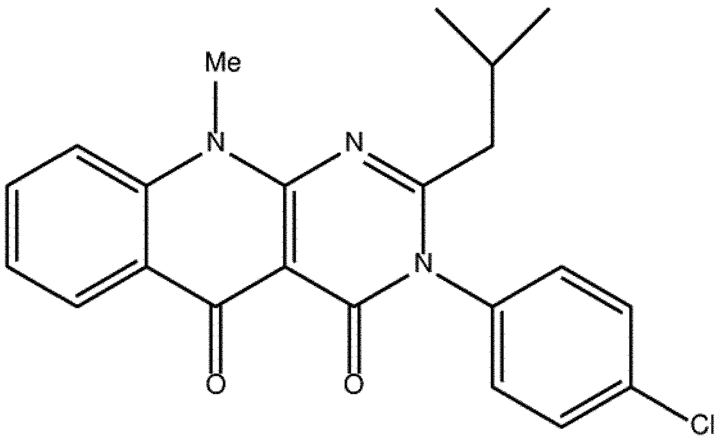

,

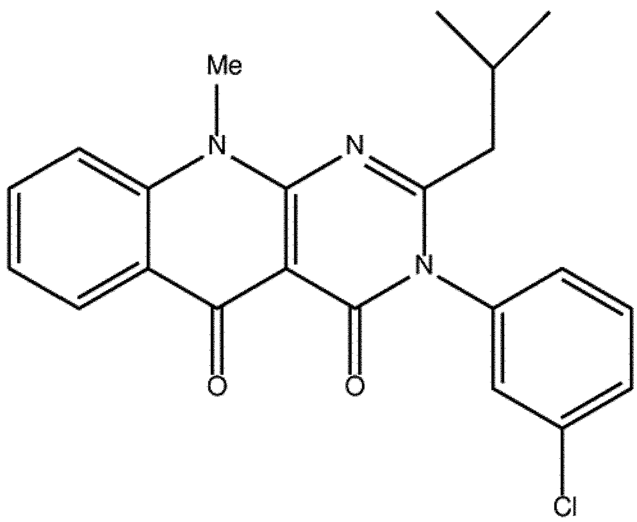

,

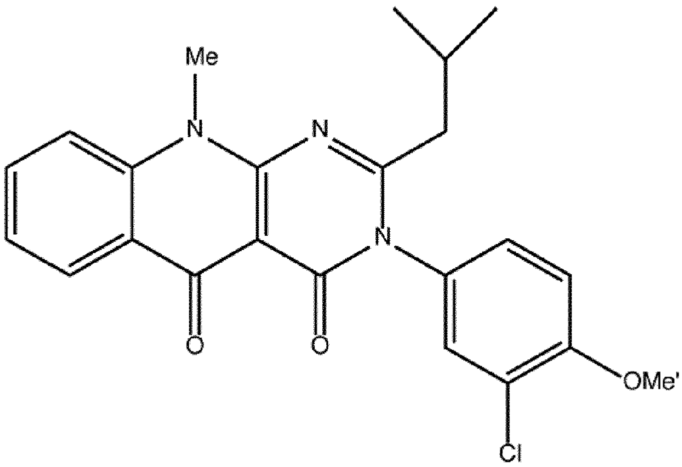

,

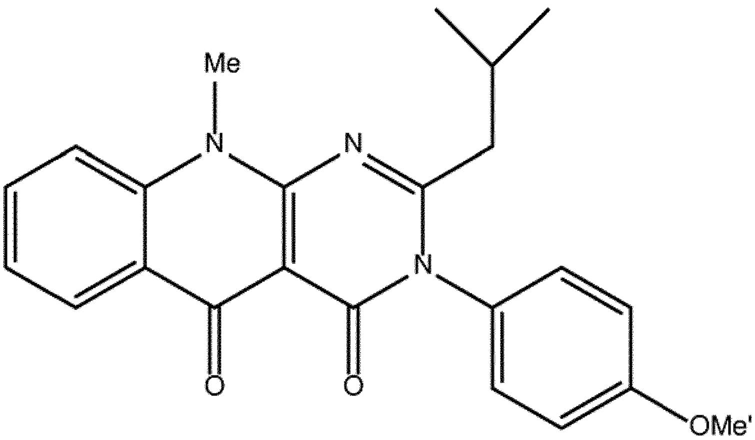

, ,

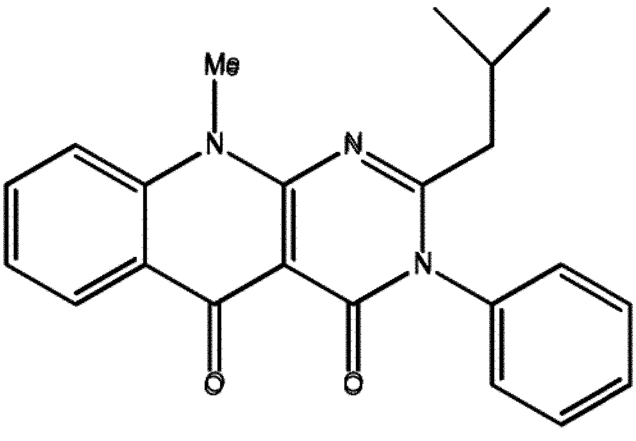

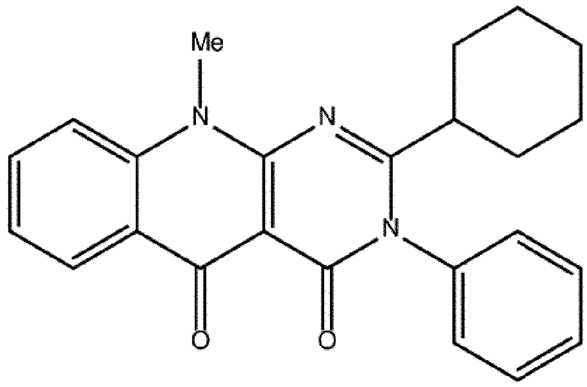

,

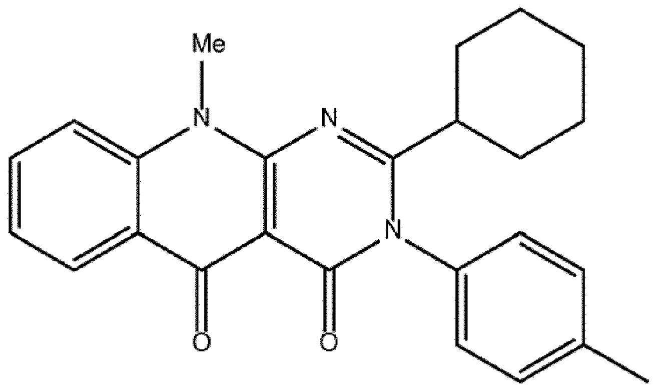

,

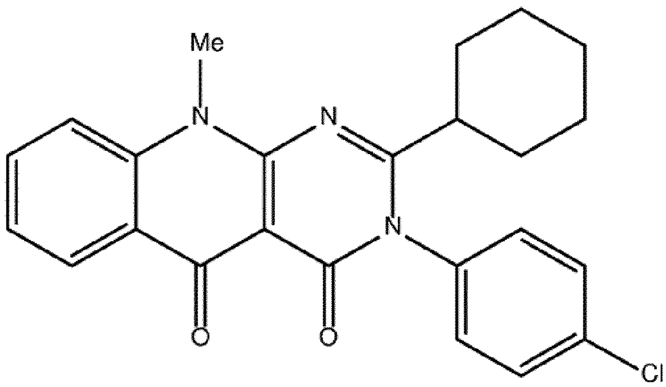

,

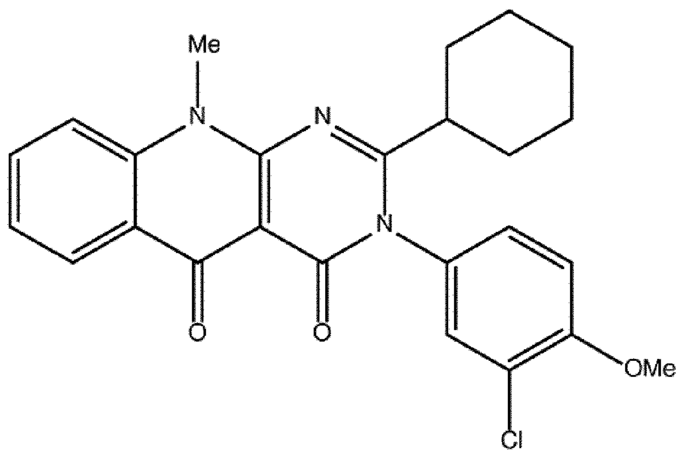

,

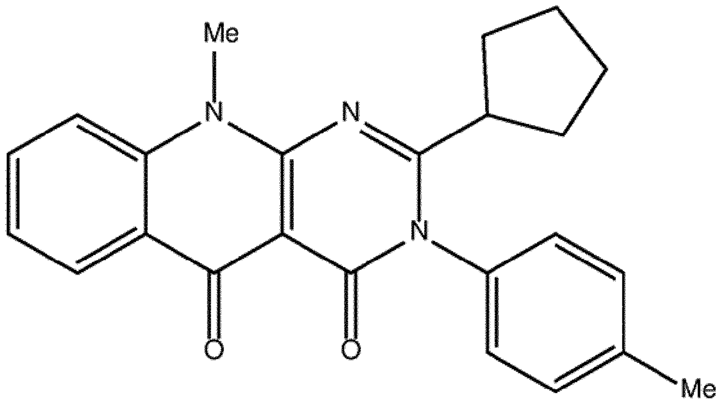

,

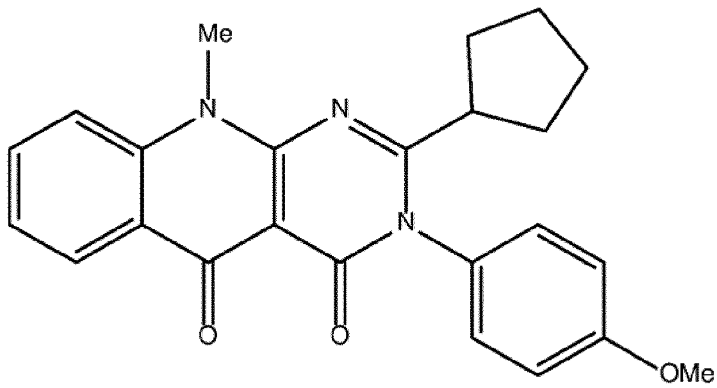

,

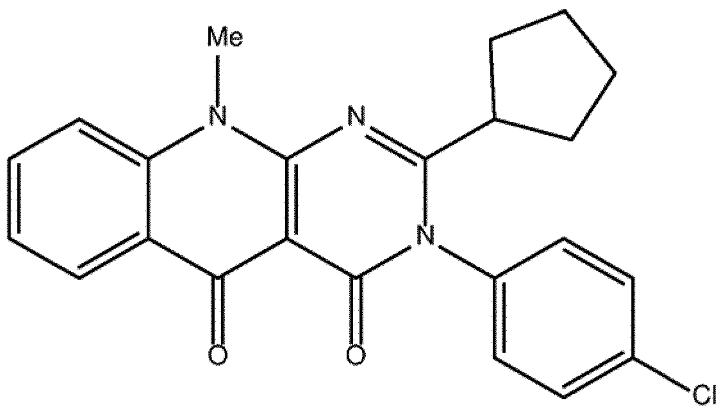

,

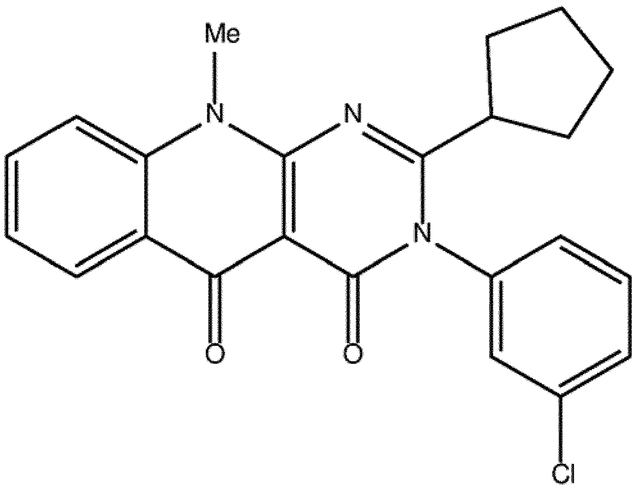

,

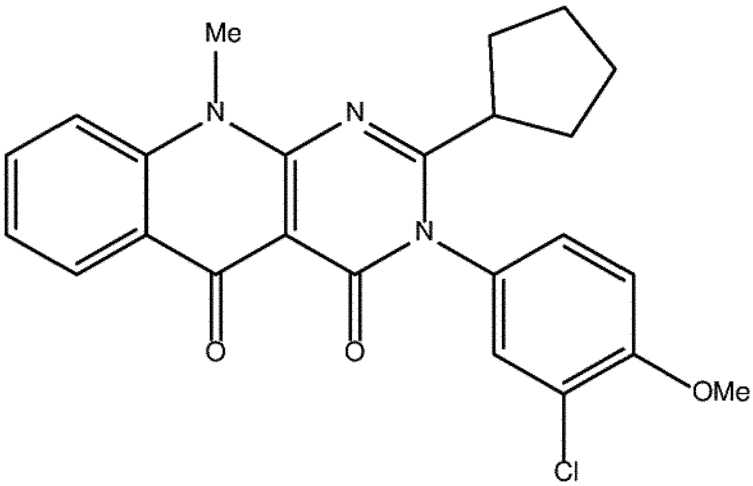

,

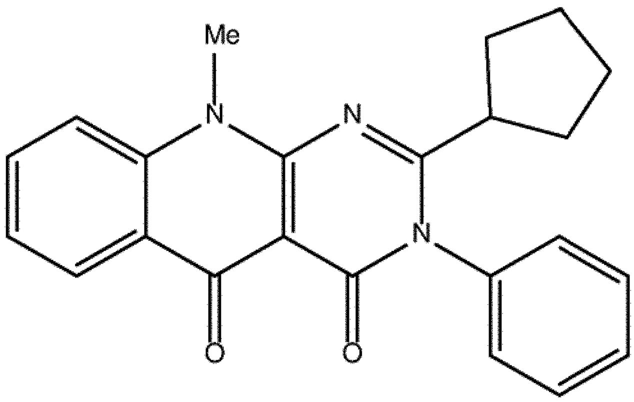

,

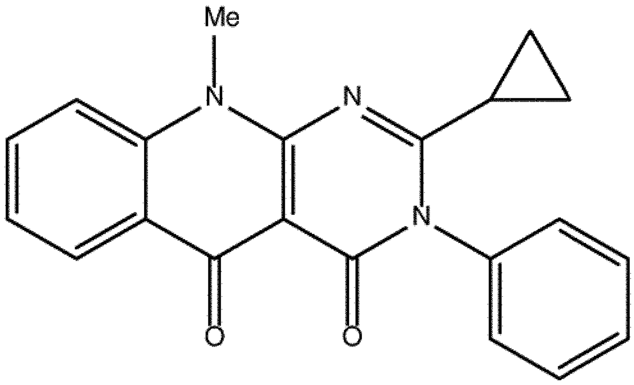

,

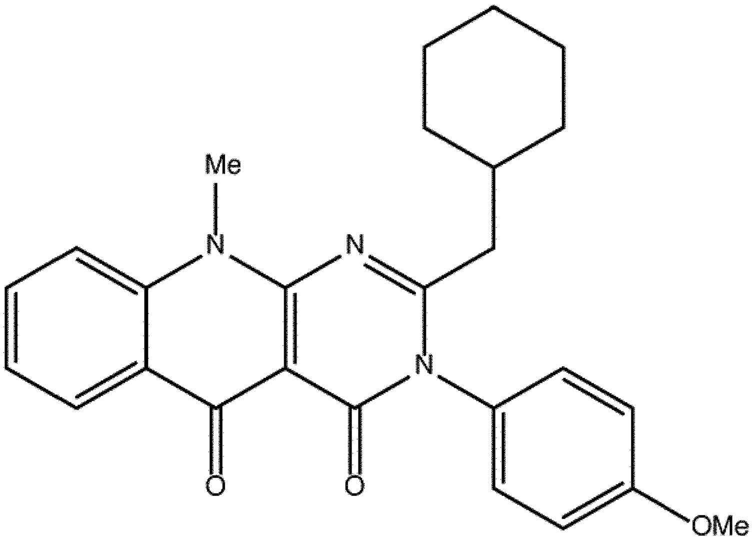

,

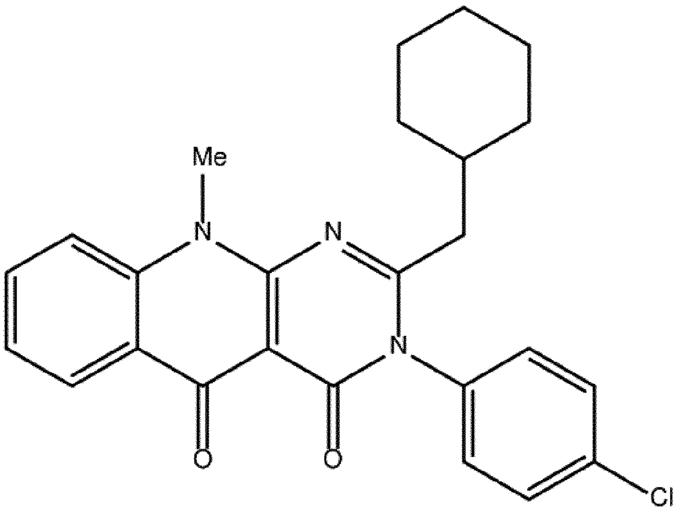

,

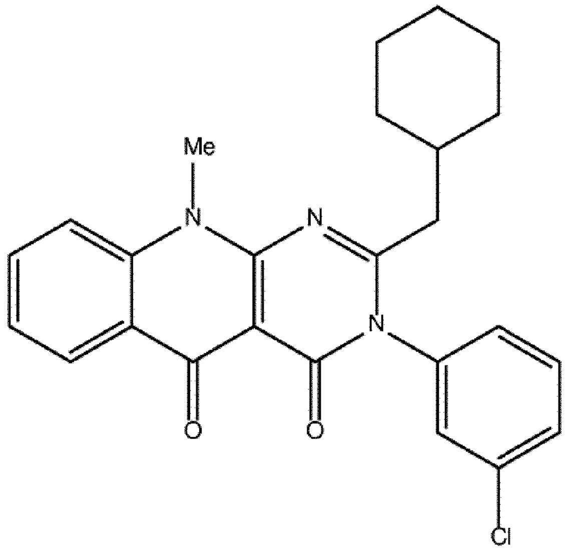

.

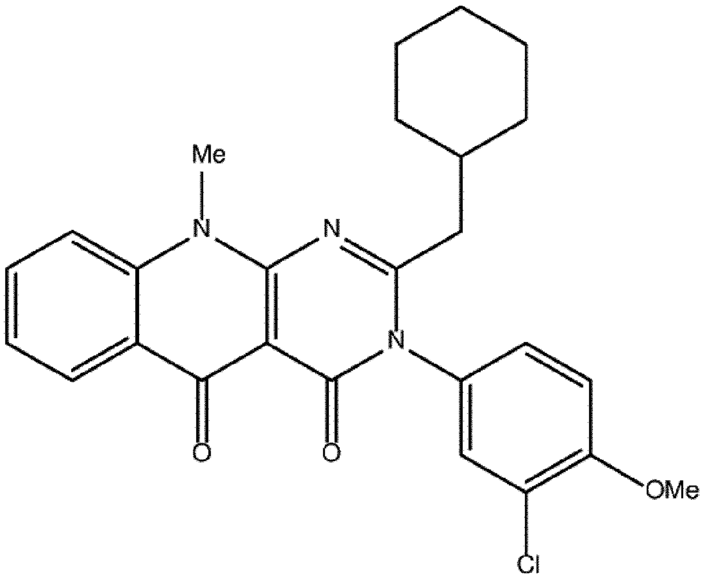

,

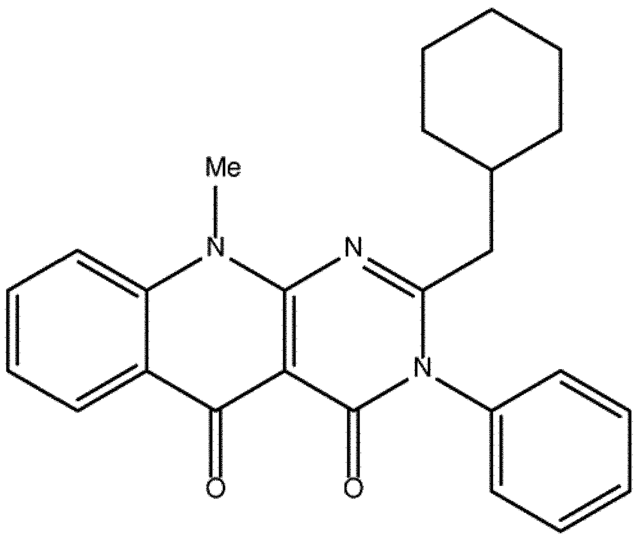

et

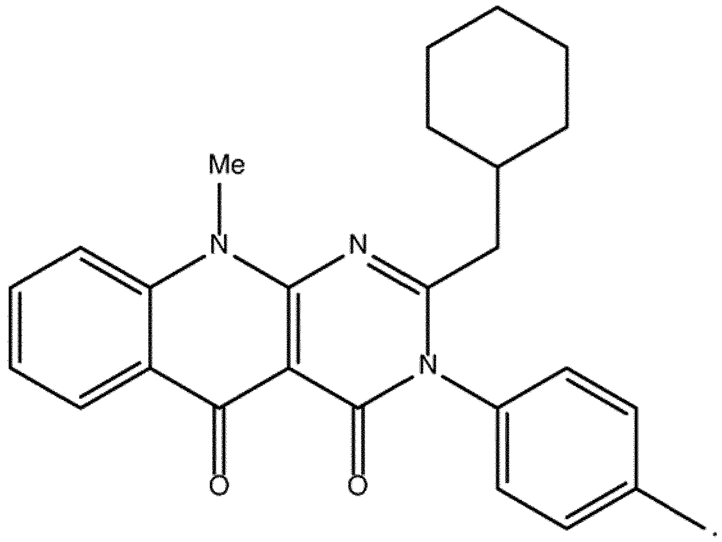

7. Composé pour utilisation selon l'une quelconque des revendications 4 à 6, dans lequel l'agoniste des récepteurs de dopamine D1 est la dopamine, et/ou dans lequel la maladie ou le trouble met en oeuvre une altération de l'humeur, de la cognition, de la mémoire, du mouvement ou des aptitudes motrices, ou une combinaison de ceux-ci.

8. Composé pour utilisation selon l'une quelconque des revendications 4 à 7, la maladie ou le trouble étant la maladie d'Alzheimer, la schizophrénie, la maladie de Parkinson, une dyskinésie ou la maladie de Huntington.

9. Composé pour utilisation selon l'une quelconque des revendications 4 à 8, l'utilisation comprenant en outre un agoniste des récepteurs de dopamine D1 autre que la dopamine, l'agoniste des récepteurs de dopamine D1 étant de préférence l'apomorphine, le fénoldopam, SKF38393, SKF77434 ou L-DOPA

10. Composé de formule (III):

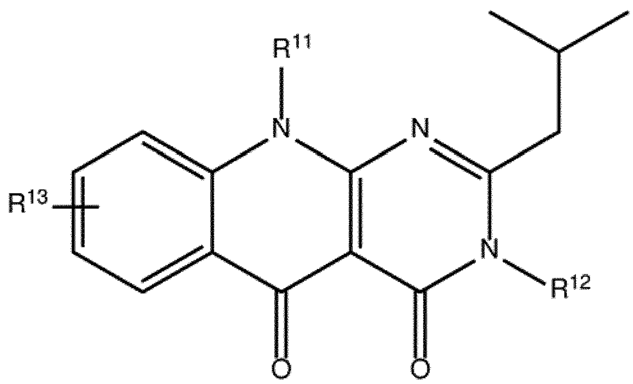

(III)

dans lequel $R^{11}$ est alkyle en $C_1$-$C_6$,
$R^{12}$ est phényle, facultativement substitué par un ou plusieurs substituants choisis dans le groupe constitué d'halogéno, alkyle en $C_1$-$C_6$ ou alcoxy en $C_1$-$C_6$, et
$R^{13}$ est H, halogéno ou alcoxy en $C_1$-$C_6$,
ou sel pharmaceutiquement acceptable de celui-ci.

11. Composition pharmaceutique comprenant un composé selon la revendication 10 et un véhicule pharmaceutiquement acceptable.

**12.** Composé ou sel selon la revendication 1, dans lequel $R^6$ est 2-pyridyle ou 3-pyridyle, dont chacun est substitué par un groupe méthyle ou un groupe méthoxy.

**13.** Composition pharmaceutique comprenant un composé selon la revendication 12 et un véhicule pharmaceutiquement acceptable.

**14.** Composé ou sel selon la revendication 1, pour utilisation dans le traitement ou la prévention d'une maladie ou d'un trouble sensible à l'activation d'un agoniste des récepteurs de dopamine D1 chez un mammifère en ayant besoin.

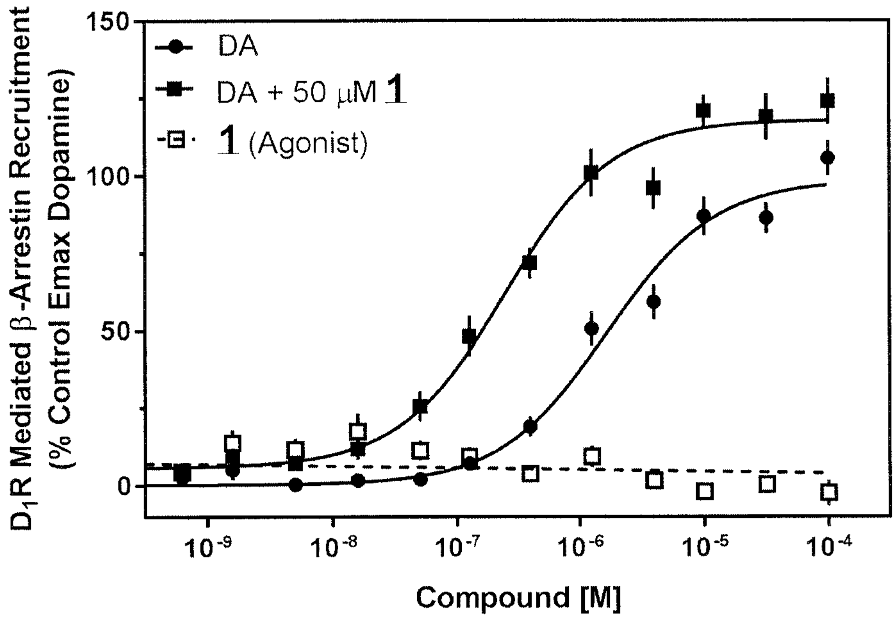

DA
DA + 50 μM 1
1 (Agonist)
$D_1R$ Mediated β-Arrestin Recruitment (% Control Emax Dopamine)
150
100
50
0
$10^{-9}$
$10^{-8}$
$10^{-7}$
$10^{-6}$
$10^{-5}$
$10^{-4}$
Compound [M]


FIG. 1A

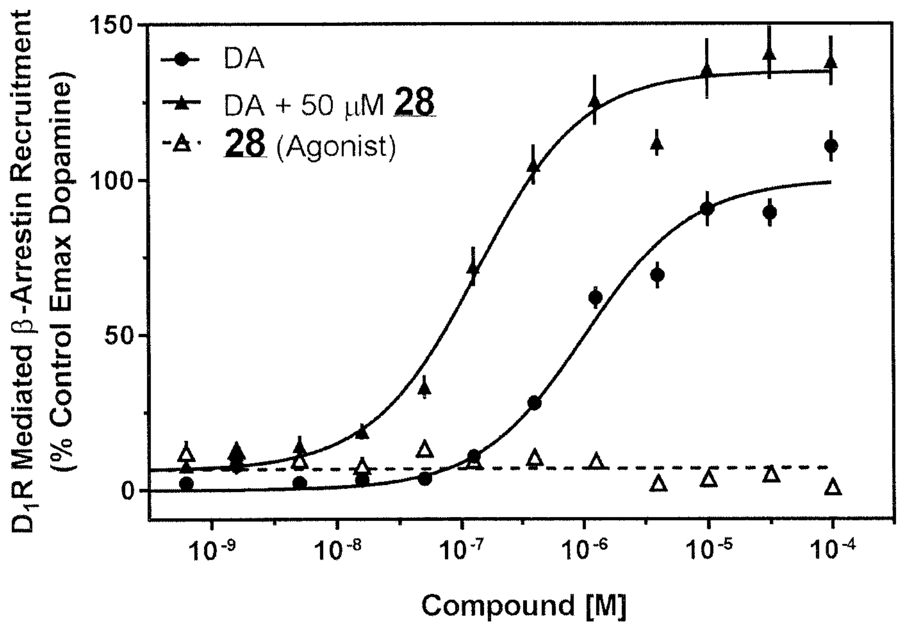

DA
DA + 50 μM 28
28 (Agonist)
$D_1R$ Mediated β-Arrestin Recruitment (% Control Emax Dopamine)
150
100
50
0
$10^{-9}$
$10^{-8}$
$10^{-7}$
$10^{-6}$
$10^{-5}$
$10^{-4}$
Compound [M]


FIG. 1B

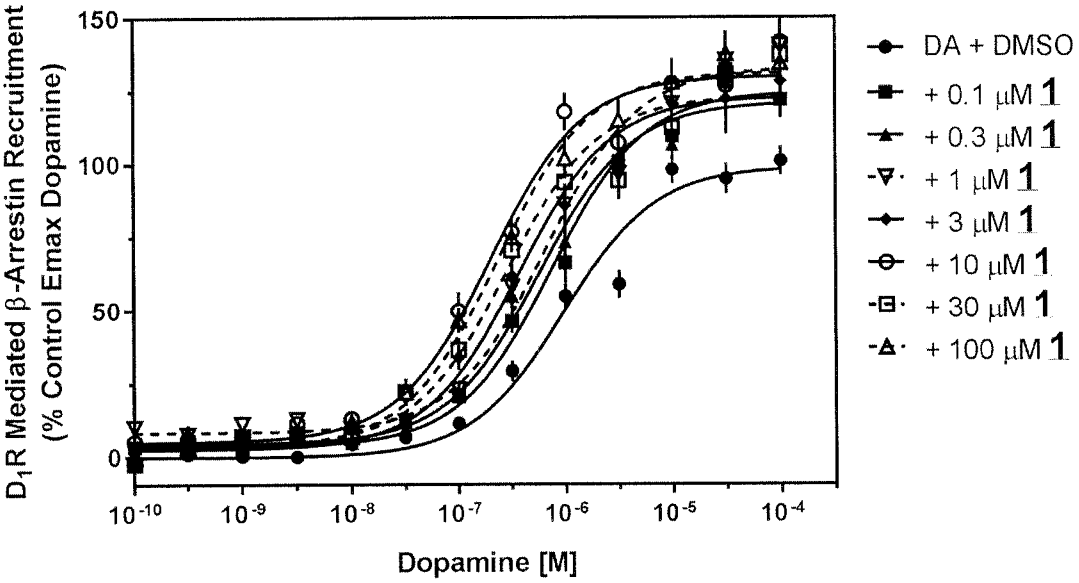

D$_1$R Mediated β-Arrestin Recruitment (% Control Emax Dopamine)
150
100
50
0
$10^{-10}$
$10^{-9}$
$10^{-8}$
$10^{-7}$
$10^{-6}$
$10^{-5}$
$10^{-4}$
Dopamine [M]
DA + DMSO
+ 0.1 μM 1
+ 0.3 μM 1
+ 1 μM 1
+ 3 μM 1
+ 10 μM 1
+ 30 μM 1
+ 100 μM 1


FIG. 2A

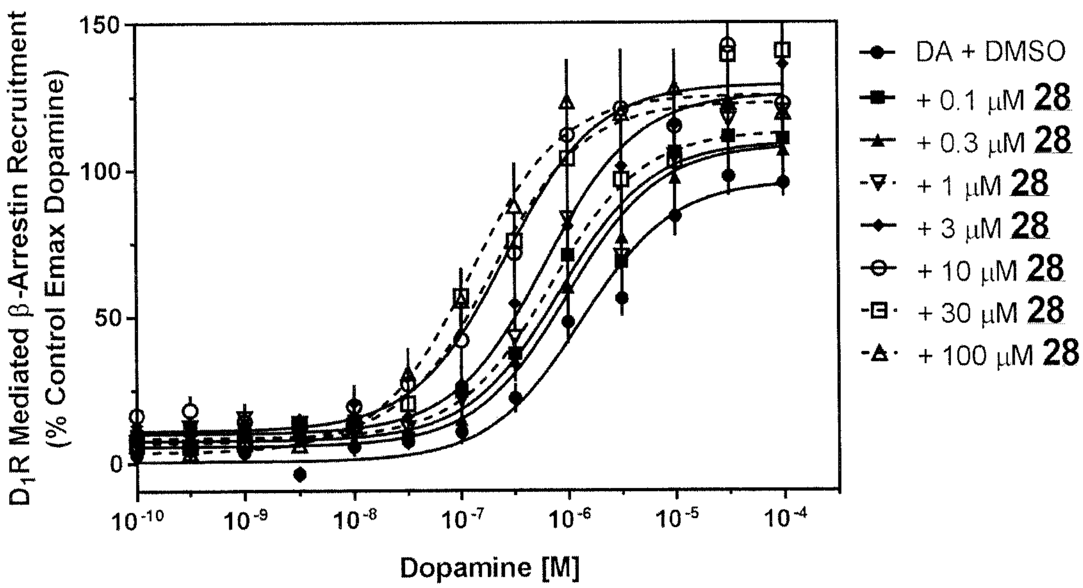

D$_1$R Mediated β-Arrestin Recruitment (% Control Emax Dopamine)
150
100
50
0
$10^{-10}$
$10^{-9}$
$10^{-8}$
$10^{-7}$
$10^{-6}$
$10^{-5}$
$10^{-4}$
Dopamine [M]
DA + DMSO
+ 0.1 μM 28
+ 0.3 μM 28
+ 1 μM 28
+ 3 μM 28
+ 10 μM 28
+ 30 μM 28
+ 100 μM 28


FIG. 2B

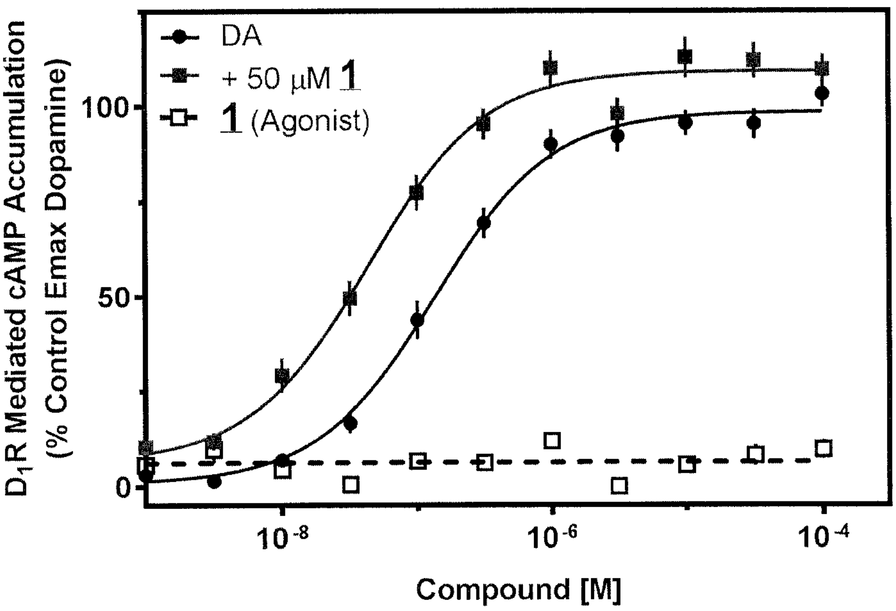
D1R Mediated cAMP Accumulation
(% Control Emax Dopamine)
DA
+ 50 μM 1
1 (Agonist)
100
50
0
10-8
10-6
10-4
Compound [M]

FIG. 3A

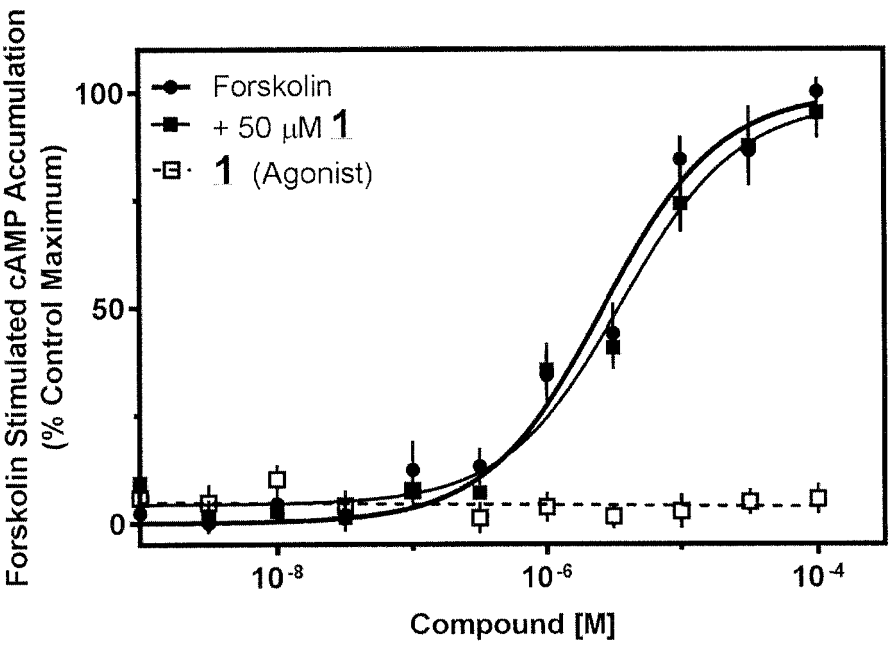
Forskolin Stimulated cAMP Accumulation
(% Control Maximum)
Forskolin
+ 50 μM 1
1 (Agonist)
100
50
0
10-8
10-6
10-4
Compound [M]

FIG. 3B

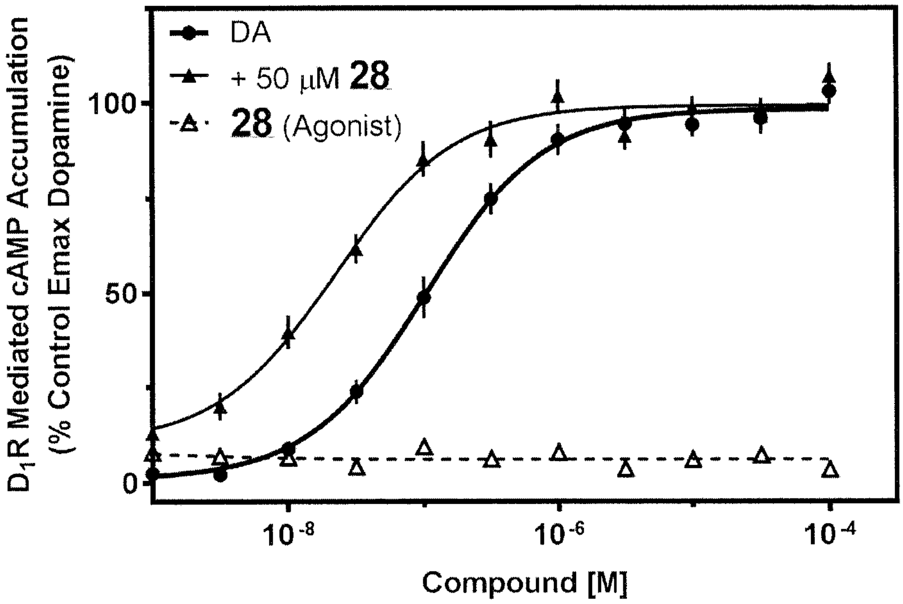
DA
+ 50 μM 28
28 (Agonist)
D1R Mediated cAMP Accumulation
(% Control Emax Dopamine)
100
50
0
10-8
10-6
10-4
Compound [M]

FIG. 3C

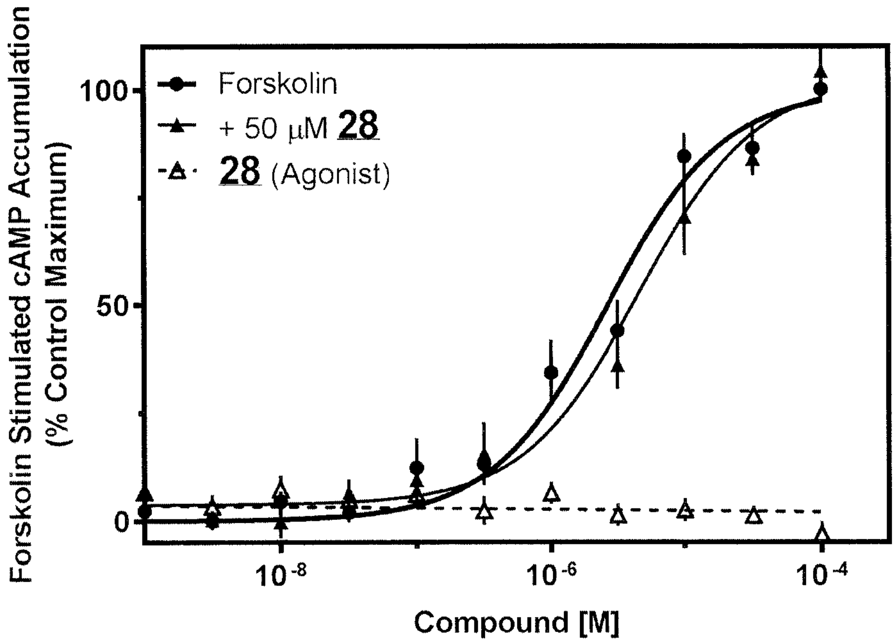
Forskolin
+ 50 μM 28
28 (Agonist)
Forskolin Stimulated cAMP Accumulation
(% Control Maximum)
100
50
0
10-8
10-6
10-4
Compound [M]

FIG. 3D

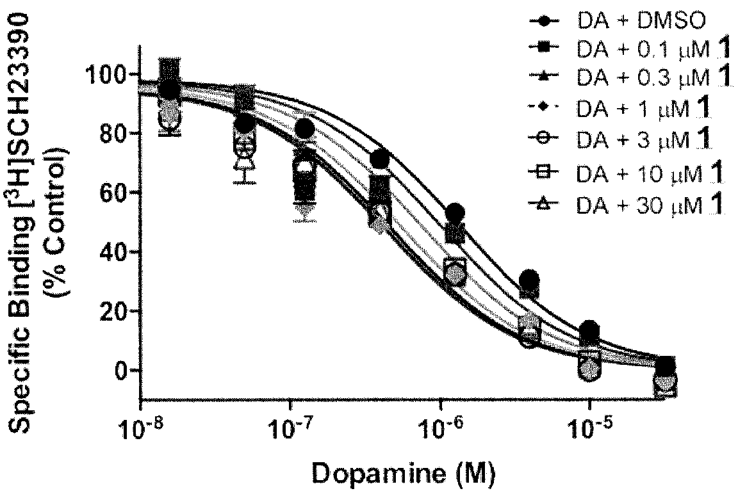


FIG. 4A

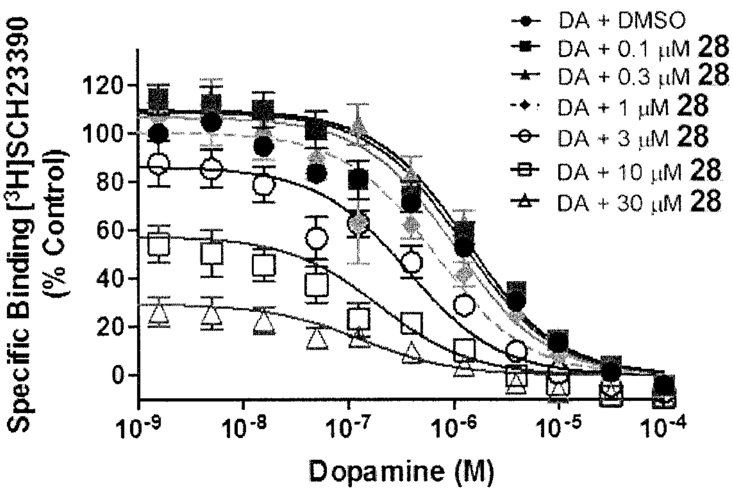


FIG. 4B

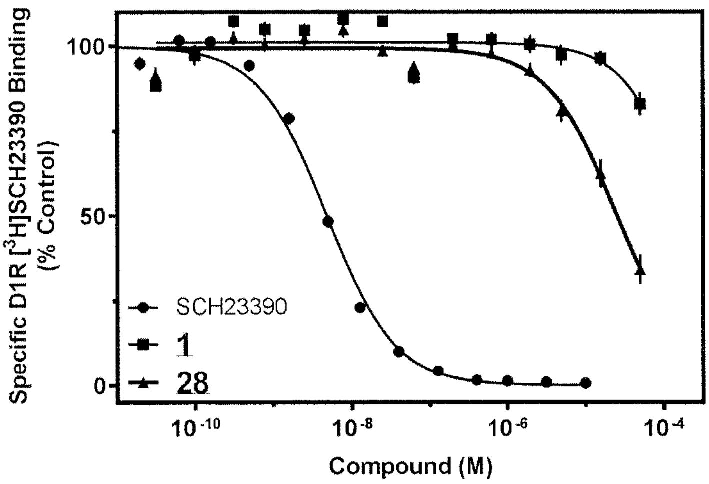


FIG. 4C

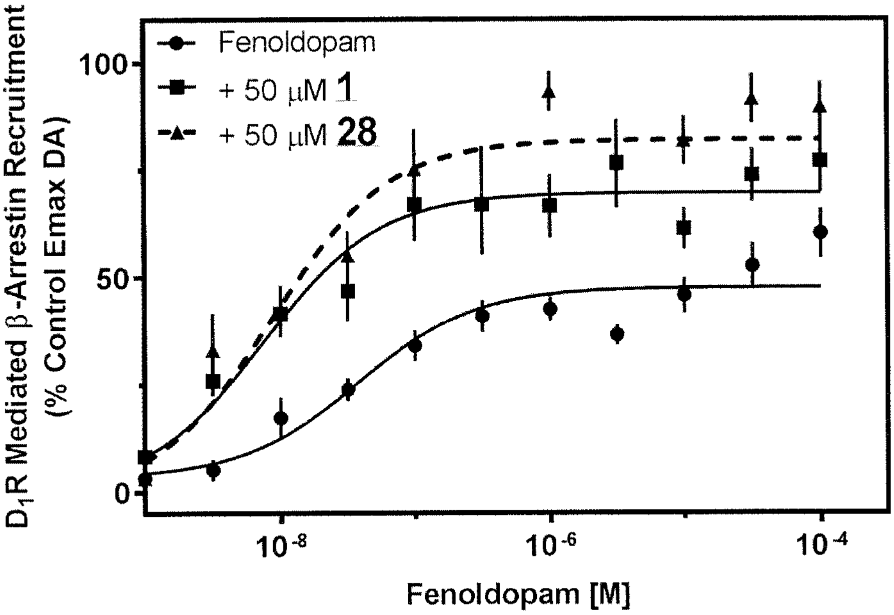

Fenoldopam
+ 50 μM 1
+ 50 μM 28
100
50
0
10-8
10-6
10-4
Fenoldopam [M]
D1R Mediated β-Arrestin Recruitment
(% Control Emax DA)


FIG. 5A

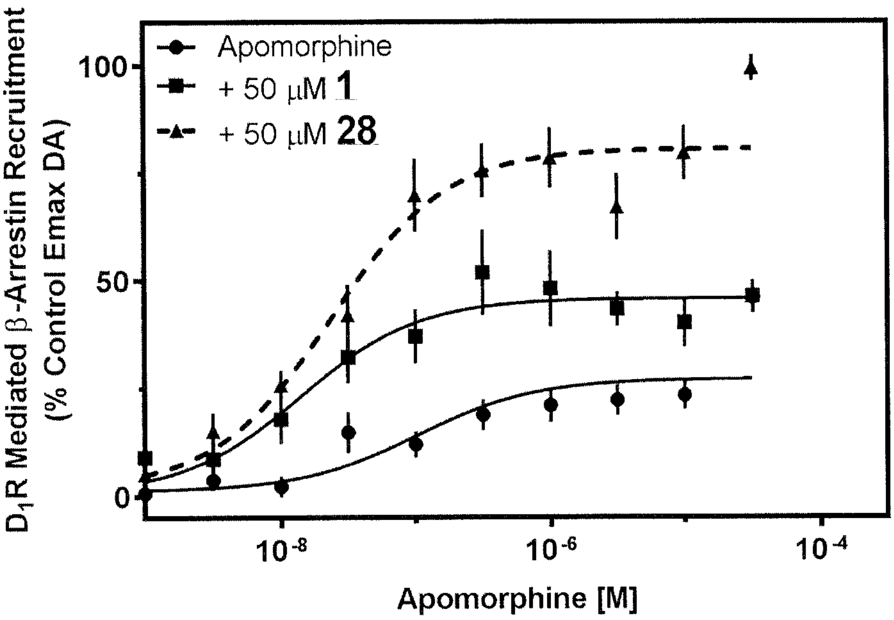

Apomorphine
+ 50 μM 1
+ 50 μM 28
100
50
0
10-8
10-6
10-4
Apomorphine [M]
D1R Mediated β-Arrestin Recruitment
(% Control Emax DA)


FIG. 5B

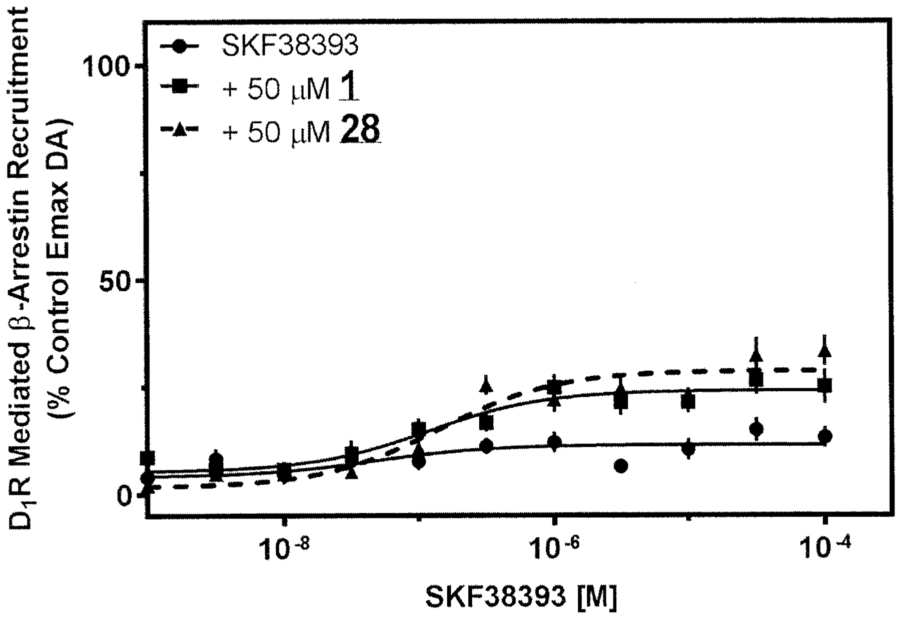
D1R Mediated β-Arrestin Recruitment (% Control Emax DA)
SKF38393
+ 50 μM 1
+ 50 μM 28
100
50
0
10-8
10-6
10-4
SKF38393 [M]

FIG. 5C

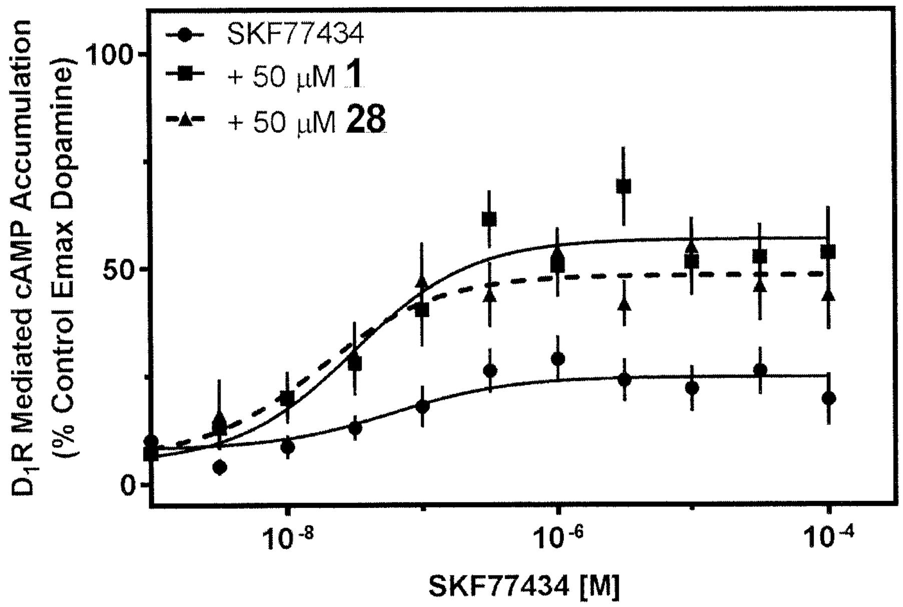
D1R Mediated cAMP Accumulation (% Control Emax Dopamine)
SKF77434
+ 50 μM 1
+ 50 μM 28
100
50
0
10-8
10-6
10-4
SKF77434 [M]

FIG. 5D

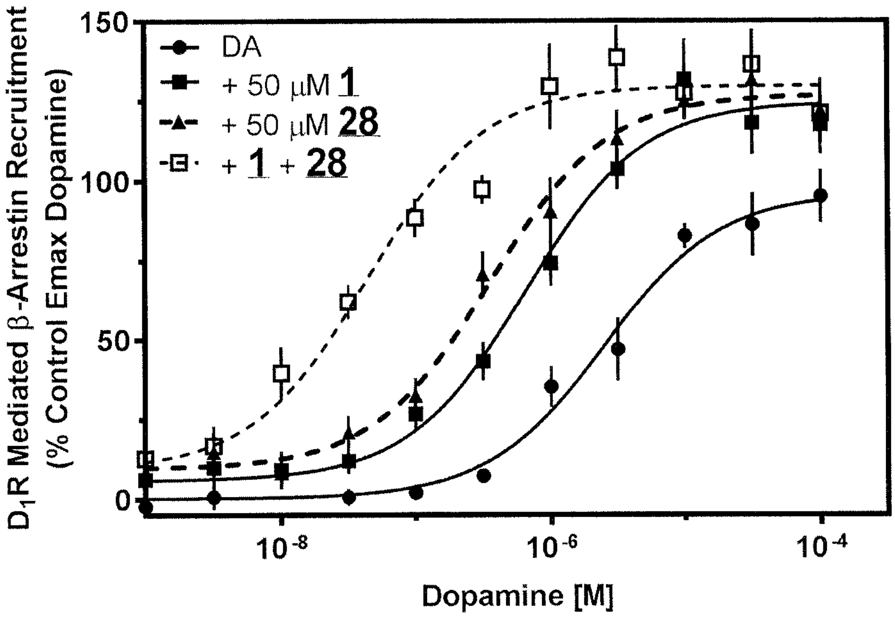

$D_1R$ Mediated β-Arrestin Recruitment
(% Control Emax Dopamine)
150
100
50
0
DA
+ 50 μM 1
+ 50 μM 28
+ 1 + 28
$10^{-8}$
$10^{-6}$
$10^{-4}$
Dopamine [M]


**FIG. 6A**

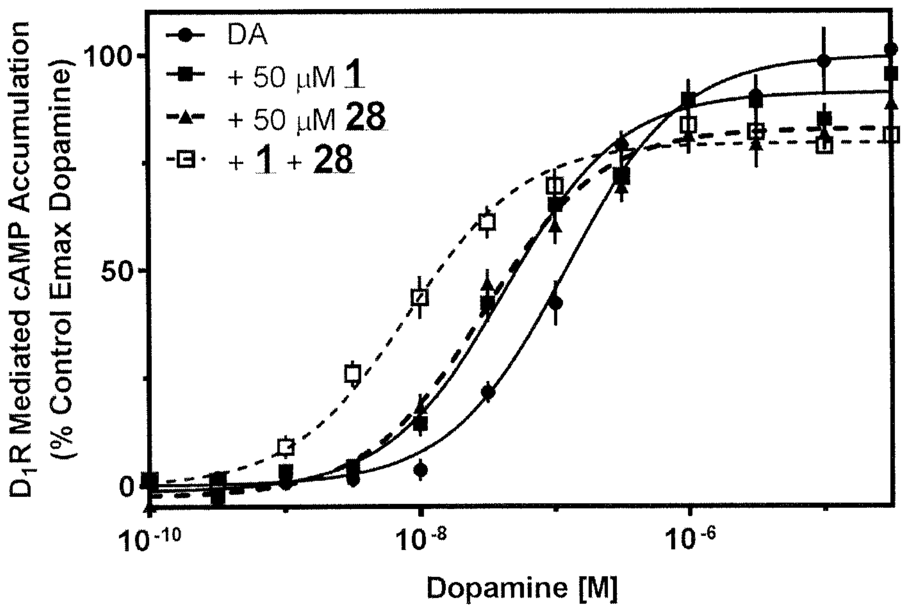

$D_1R$ Mediated cAMP Accumulation
(% Control Emax Dopamine)
100
50
0
DA
+ 50 μM 1
+ 50 μM 28
+ 1 + 28
$10^{-10}$
$10^{-8}$
$10^{-6}$
Dopamine [M]


**FIG. 6B**

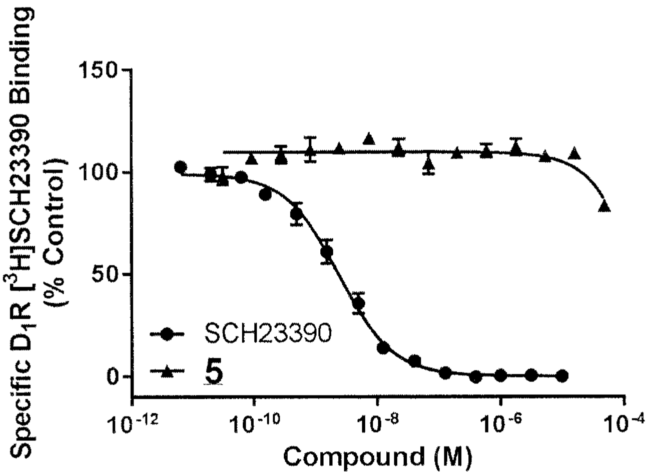


FIG. 7A

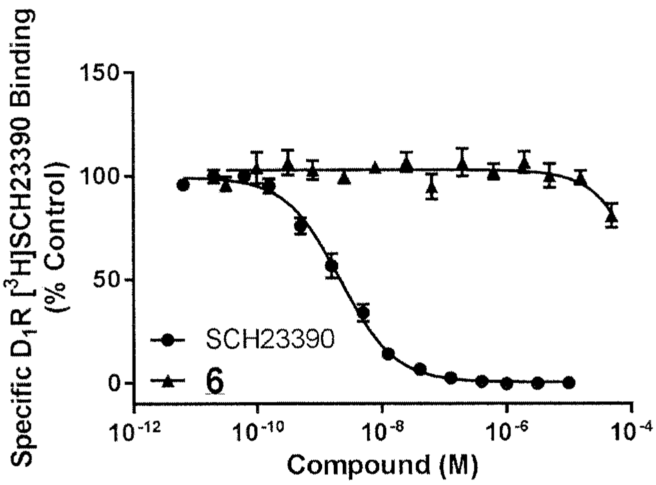


FIG. 7B

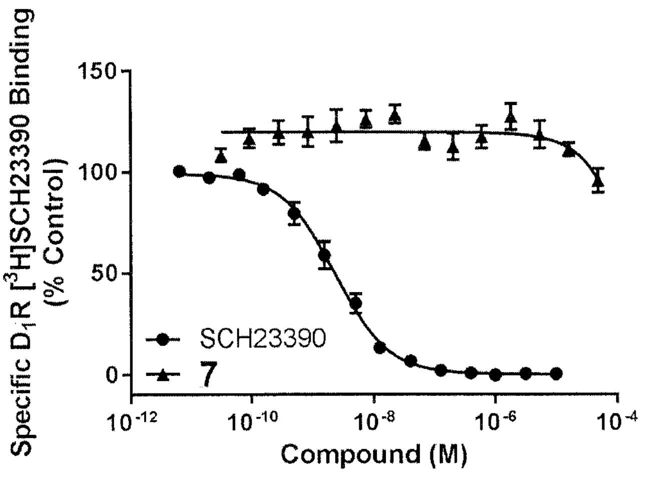


FIG. 7C

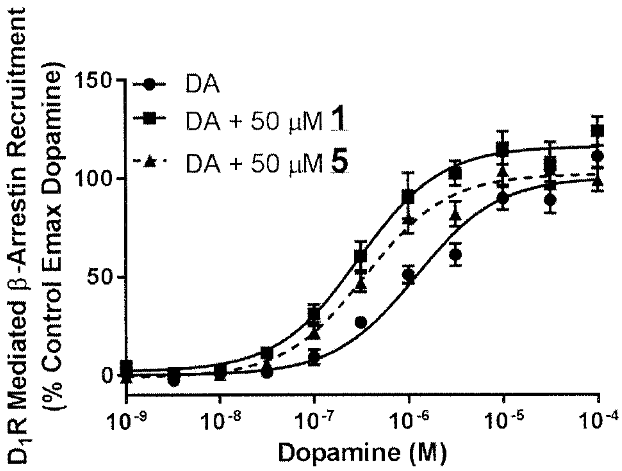


**FIG. 8A**

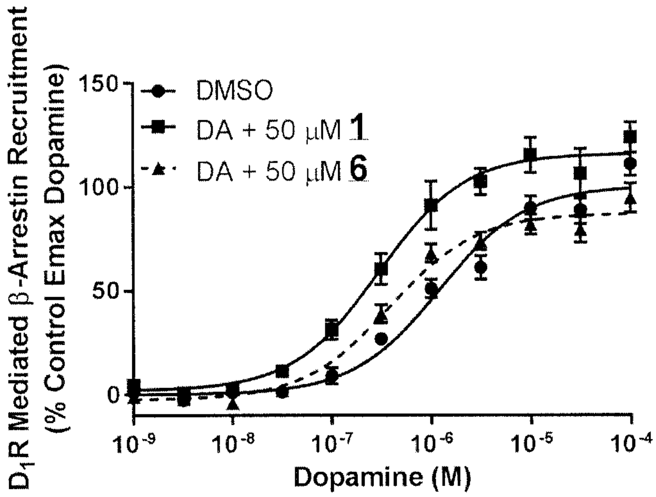


**FIG. 8B**

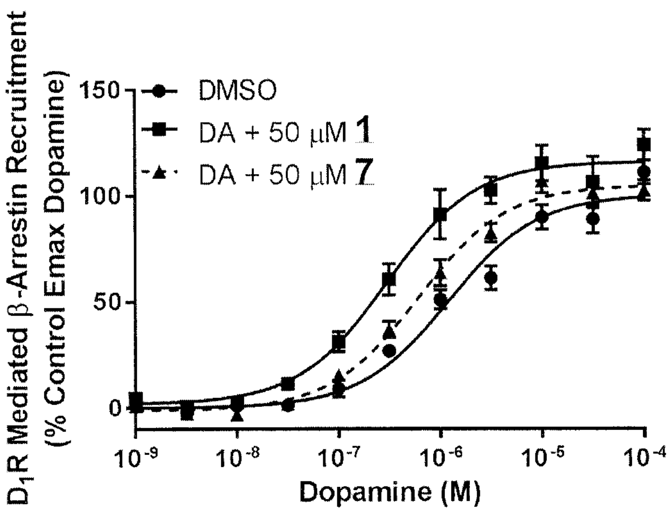


**FIG. 8C**

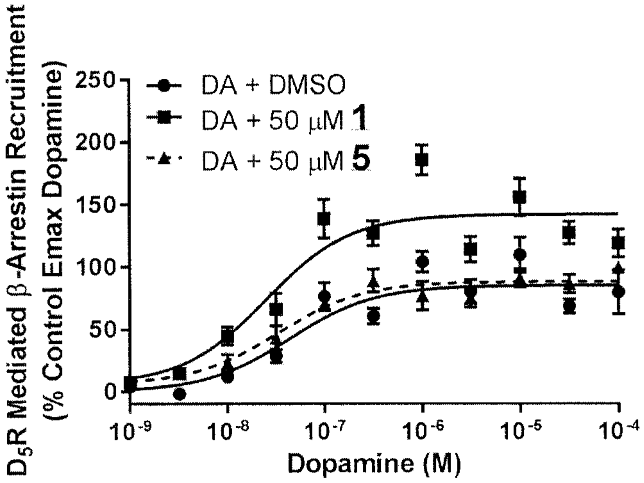


FIG. 9A

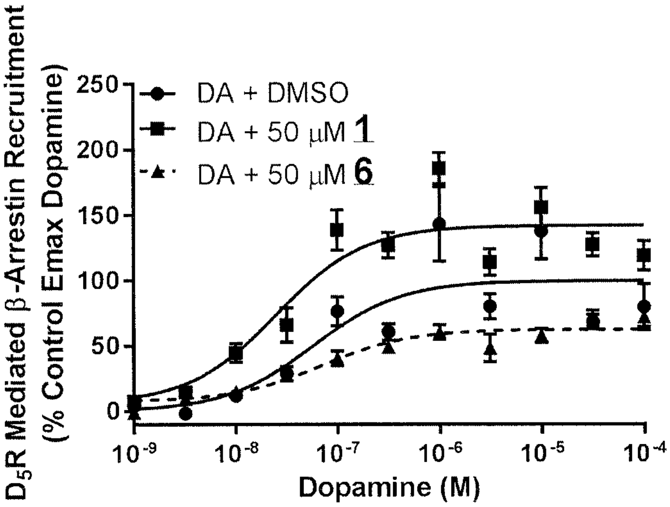


FIG. 9B

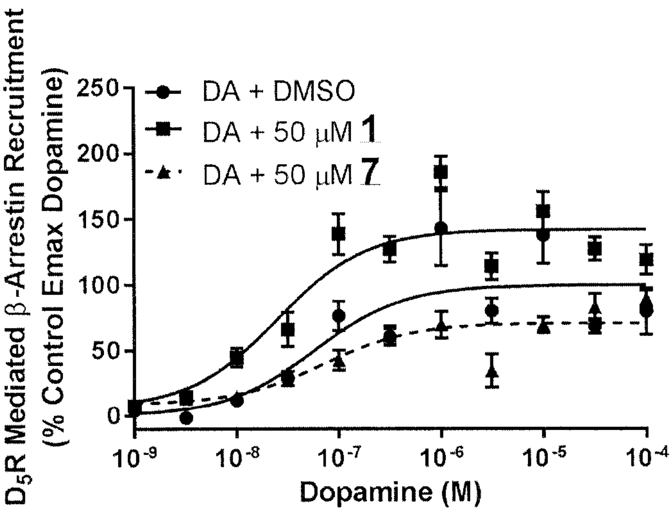


FIG. 9C

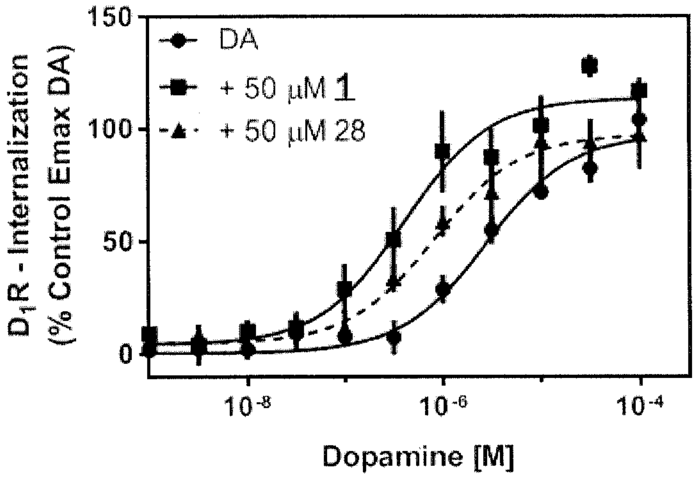
150
100
50
0
D1R - Internalization
(% Control Emax DA)
DA
+ 50 μM 1
+ 50 μM 28
10-8
10-6
10-4
Dopamine [M]

FIG. 10

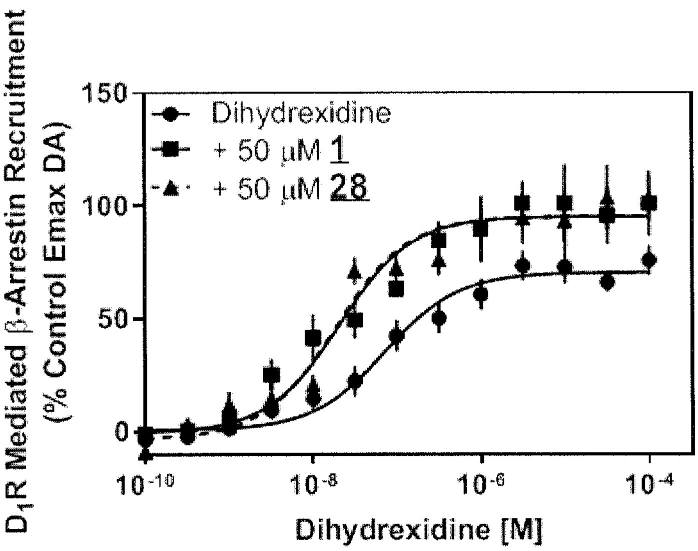
150
100
50
0
D1R Mediated β-Arrestin Recruitment
(% Control Emax DA)
Dihydrexidine
+ 50 μM 1
+ 50 μM 28
10-10
10-8
10-6
10-4
Dihydrexidine [M]

FIG. 11

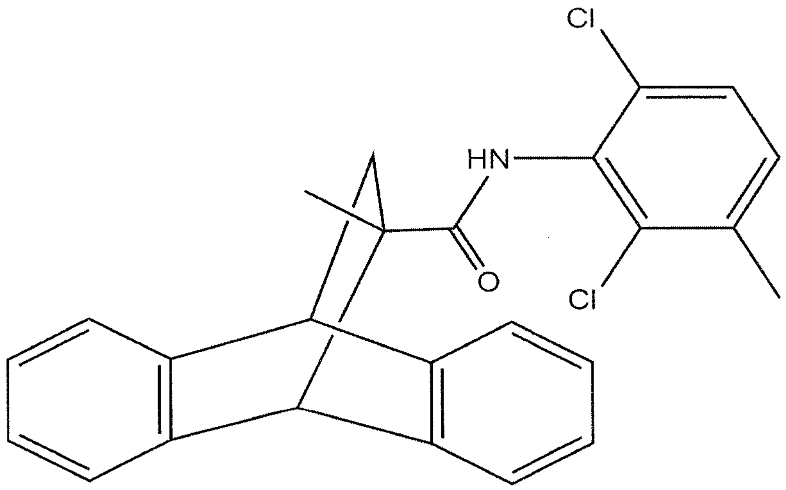

FIG. 12A

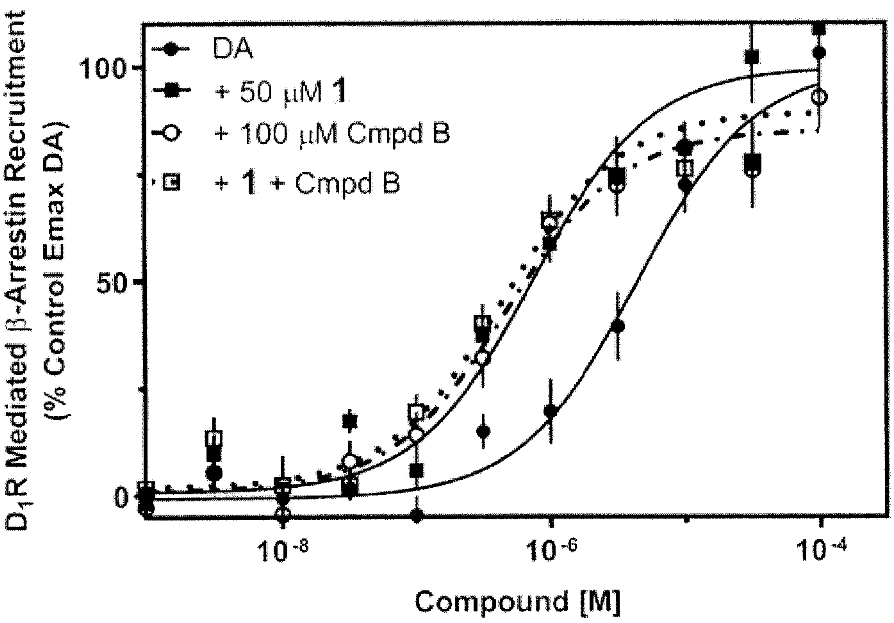


FIG. 12B

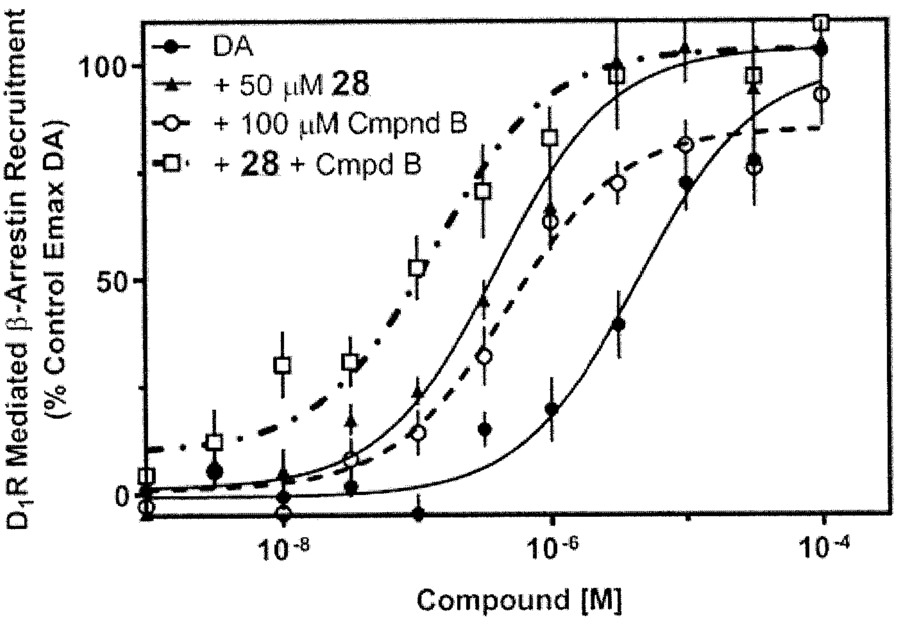


FIG. 12C

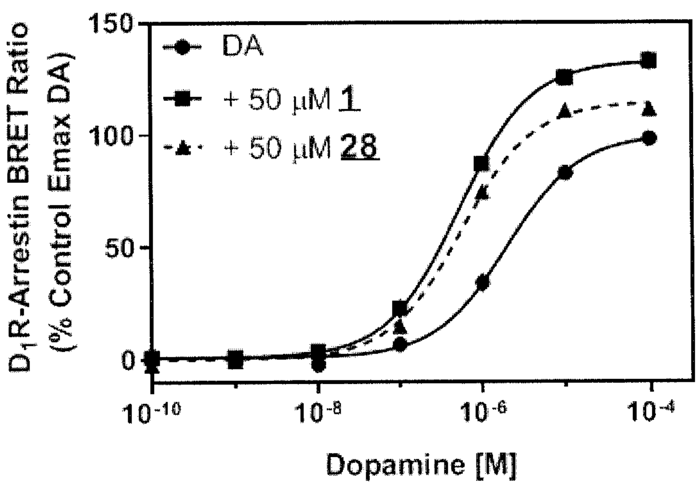


FIG. 13A

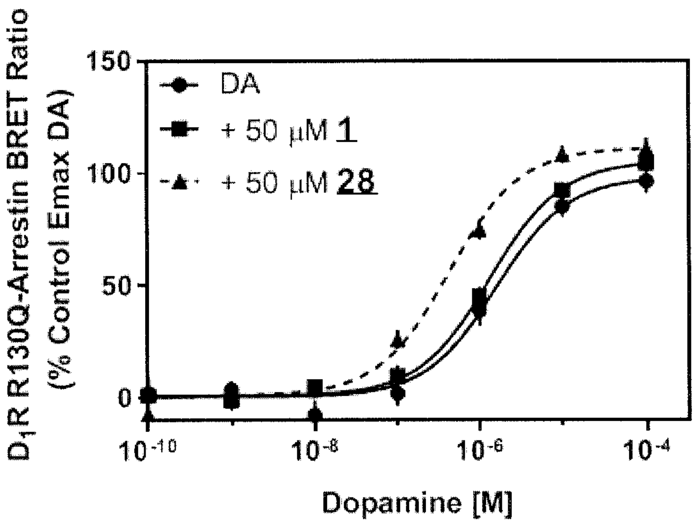


FIG. 13B

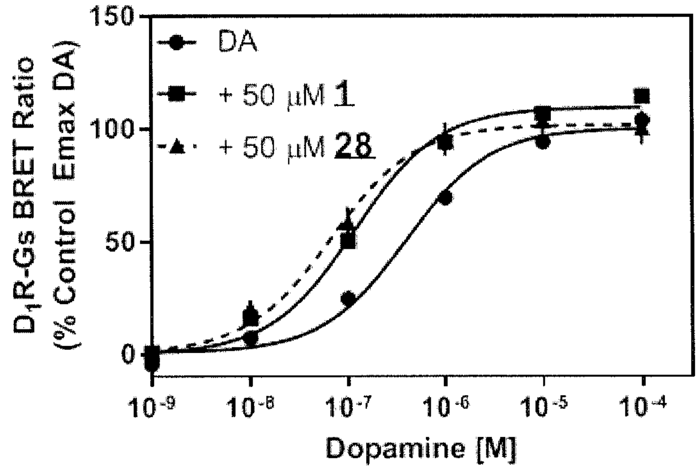


FIG. 13C

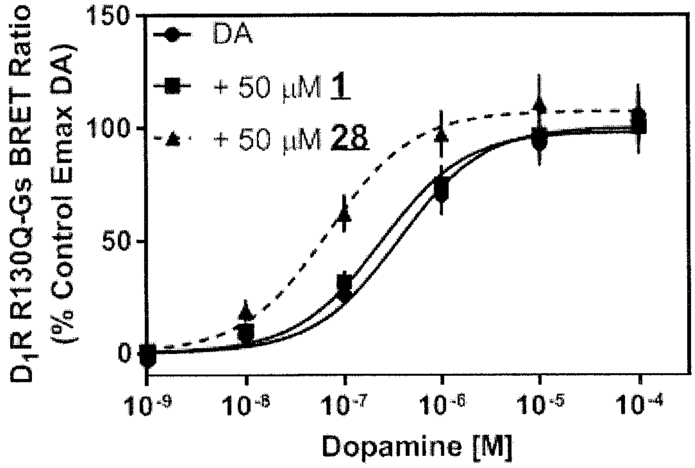


FIG. 13D

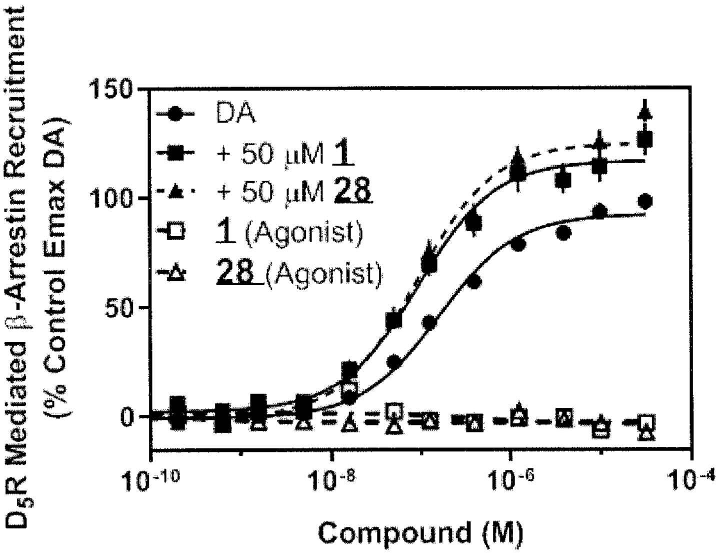


FIG. 14

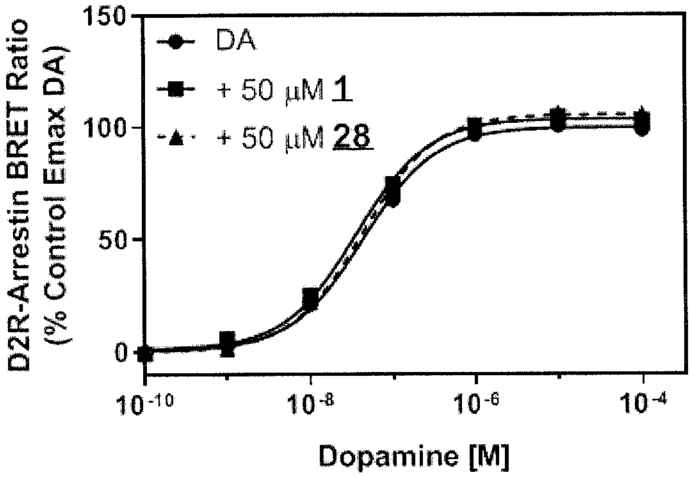


FIG. 15A

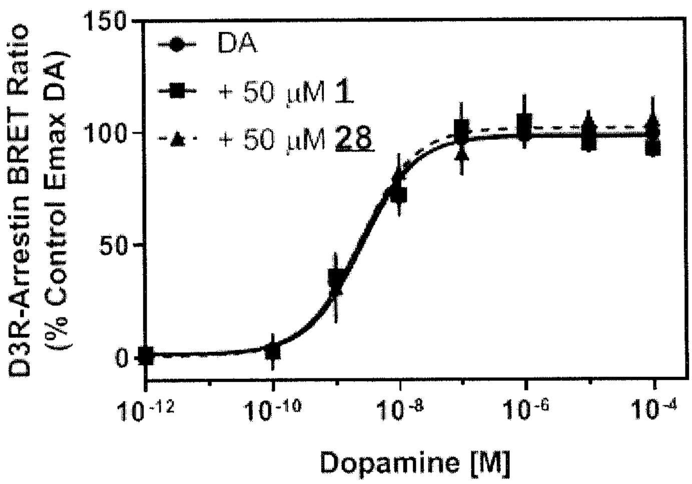


FIG. 15B

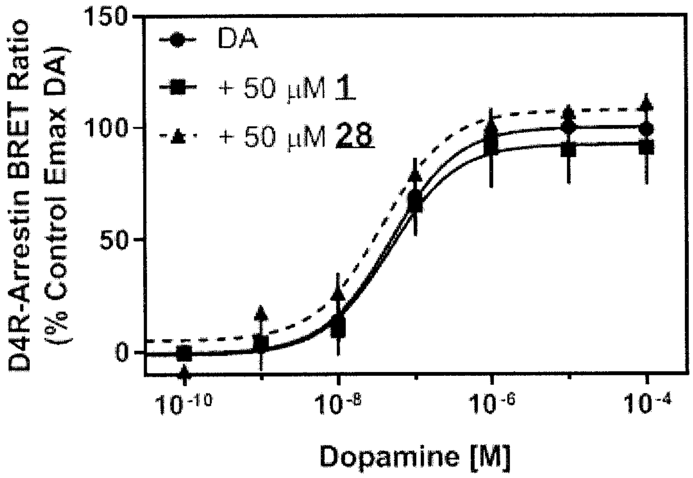


FIG. 15C

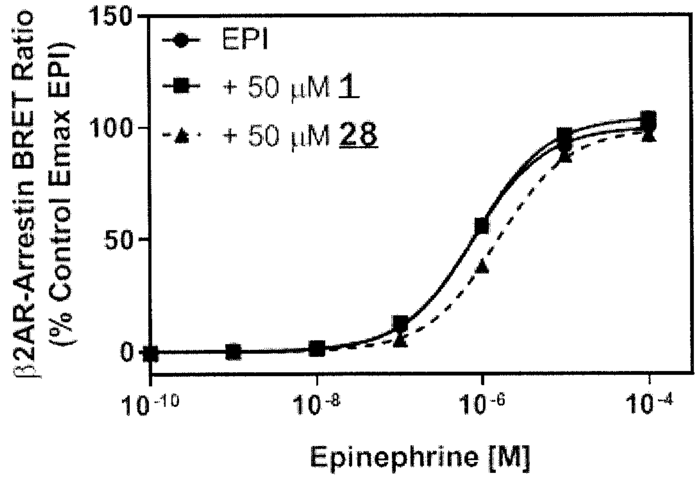


FIG. 15D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62639271


### Non-patent literature cited in the description


- **A. SOR!ANO et al.** A Hybrid Indoloquinolizidine Peptide as Allosteric Modulator of Dopamine D1 Receptors. *JOURNAL OF PHARMACOLOGY AND EXPERIMENTAL THERAPEUTICS, US,* 01 March 2010, vol. 332 (3), ISSN 0022-3565, 876-885
- **M. A. LEWIS et al.** Discovery of D1 Dopamine Receptor Positive Allosteric Modulators: Characterization of Pharmacology and Identification of Residues that Regulate Species Selectivity. *PHARMACOLOGICAL REVIEWS, US,* 24 July 2015, vol. 354 (3), ISSN 0031-6997, 340-349
- Remington's Pharmaceutical Sciences. Mack Publishing Company, 1990, 1445
- *Journal of Pharmaceutical Science,* 1977, vol. 66, 2-19
- Pharmaceutics and Pharmacy Practice. J. B. Lippincott Co, 1982, 238-250
- ASHP Handbook on Injectable Drugs. Toissel, 1986, 622-630
- *Org. Biomol. Chem.,* 2014, vol. 12, 1942-1956
- *Chem. Ber.,* 1966, vol. 99, 94-100
- **AMSTEN, A. F. ; A. G. DUDLEY.** Methylphenidate improves prefrontal cortical cognitive function through alpha2 adrenoceptor and dopamine D1 receptor actions: Relevance to therapeutic effects in Attention Deficit Hyperactivity Disorder. *Behav Brain Funct,* 2005, vol. 1 (1), 2
- **AMSTEN, A. F. ; B. M. LI.** Neurobiology of executive functions: catecholamine influences on prefrontal cortical functions. *Biol Psychiatry,* 2005, vol. 57 (11), 1377-1384
- Dopamine's Influence on Prefrontal Cortical Cognition: Actions and Circuits in Behaving Primates. **AMSTEN, A. F. T. ; VIJAYRAGAVAN S. ; WANG M. ; GAMO N.J. ; PASPALAS C.D. ; I. S. D. IVERSEN L.L. ; DUNNETT S.B. ; BJORKLUND A.** Dopamine Handbook. Oxford University Press, 2009, 230-248
- **EGLEN, R. M.** Assessing GPCR activation using protein complementation: a novel technique for HTS. *Biochem Soc Trans,* 2007, vol. 35, 746-748
- **GJONI, T. ; S. URWYLER.** Receptor activation involving positive allosteric modulation, unlike full agonism, does not result in GABAB receptor desensitization. *Neuropharmacology,* 2008, vol. 55 (8), 1293-1299
- **GOLDMAN-RAKIC, P. S. ; S. A. CASTNER, T. H. SVENSSON ; L. J. SIEVER ; G. V. WILLIAMS.** Targeting the dopamine D1 receptor in schizophrenia: insights for cognitive dysfunction. *Psychopharmacology (Berl),* 2004, vol. 174 (1), 3-16
- **MAY, L. T ; K. LEACH, P. M. SEXTON ; A. CHRISTOPOULOS.** Allosteric modulation of G protein-coupled receptors. *Annu Rev Pharmacol Toxicol,* 2007, vol. 47, 1-51
- **SIBLEY, D. R. ; F. J. MONSMA, JR.** Molecular biology of dopamine receptors. *Trends Pharmacol Sci,* 1992, vol. 13 (2), 61-69
- **TAMMINGA, C. A.** The neurobiology of cognition in schizophrenia. *J Clin Psychiatry,* 2006, vol. 67 (9), e11
- **VIJAYRAGHAVAN, S. ; M. WANG ; S. G. BIRNBAUM ; G. V. WILLIAMS ; A. F. ARNSTEN.** Inverted-U dopamine D1 receptor actions on prefrontal neurons engaged in working memory. *Nat Neurosci,* 2007, vol. 10 (3), 376-384
- **CONROY J.L. ; FREE R.B. ; SIBLEY D.R.** Identification of G protein-biased agonists that fail to recruit beta-arrestin or promote internalization of the D1 dopamine receptor. *ACS chemical neuroscience,* 2015, vol. 6 (4), 681-692
- **LEWIS M.A. ; HUNIHAN L. ; WATSON J. ; GENTLES R.G. ; HU S. ; HUANG Y. ; BRONSON J. ; MACOR J.E. ; BENO B.R. ; FERRANTE M.** Discovery of D1 Dopamine Receptor Positive Allosteric Modulators: Characterization of Pharmacology and Identification of Residues that Regulate Species Selectivity. *The Journal of pharmacology and experimental therapeutics,* 2015, vol. 354 (3), 340-349